(19) Europäisches Patentamt
European Patent Office
Office européen des brevets

(11) **EP 4 292 532 A1**

(12) **EUROPEAN PATENT APPLICATION**
published in accordance with Art. 153(4) EPC

(43) Date of publication:
**20.12.2023 Bulletin 2023/51**

(21) Application number: **21925532.0**

(22) Date of filing: **31.12.2021**

(51) International Patent Classification (IPC):
**A61B 5/1455** $^{(2006.01)}$    **A61B 5/00** $^{(2006.01)}$

(86) International application number:
**PCT/CN2021/143795**

(87) International publication number:
**WO 2022/170887 (18.08.2022 Gazette 2022/33)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.02.2021 CN 202110185768**

(71) Applicant: **Sunrise Technologies Co., Ltd.**
**Fengtai District Beijing 100071 (CN)**

(72) Inventors:
• **XU, Kexin**
**Beijing 100071 (CN)**
• **HAN, Tongshuai**
**Beijing 100071 (CN)**
• **YAO, Mingfei**
**Beijing 100071 (CN)**
• **LIU, Xueyu**
**Beijing 100071 (CN)**

(74) Representative: **Isarpatent**
**Patent- und Rechtsanwälte**
**Barth Hassa Peckmann & Partner mbB**
**Friedrichstraße 31**
**80801 München (DE)**

(54) **TISSUE ELEMENT MEASUREMENT METHOD AND APPARATUS, AND WEARABLE DEVICE**

(57) A method and a device of measuring a tissue element, and a wearable apparatus. The method includes: irradiating (S310) a measurement region with incident light having a single predetermined wavelength, where each beam of the incident light passes through the measurement region to form at least one beam of exit light exited from at least one exit position, and a number of incident positions of the incident light is at least one; obtaining (S320) a light intensity value corresponding to each beam of the exit light acquired by M photosensitive surfaces so as to obtain T output light intensities, where each output light intensity is obtained by processing the light intensity value of the exit light acquired by one or more photosensitive surfaces, and each photosensitive surface is used to acquire the light intensity value of the exit light exited from the exit position within a predetermined anti-jitter range corresponding to the photosensitive surface, $1 \leq T \leq M$; and determining (S330) a concentration of a detected tissue element according to at least one output light intensity corresponding to the predetermined wavelength.

FIG. 3

EP 4 292 532 A1

**Description**

TECHNICAL FIELD

Embodiments of the present disclosure relate to a field of spectrum measurement technology, and in particular, to a method of measuring a tissue element, a device of measuring a tissue element, and a wearable apparatus.

BACKGROUND

A variety of tissue elements, such as blood glucose, fat, and white blood cells, etc. are contained in a body fluid of a human body. A concentration of each tissue element is required to be within a corresponding concentration range to ensure a healthy operation of the human body. However, for some individuals, the tissue element is prone to imbalance, that is, the concentration of the tissue element is not within a numerical range, which may lead to diseases, and endanger health and even life. For such objects, there is a need to perform a real-time measurement on the tissue element.

An optical method has characteristics of rapidness, non-invasiveness, and multidimensional information, etc., and is generally adopted to measure a tissue element in a related art. According to a measurement principle, the optical method mainly includes Raman spectrometry, polarization method, optical coherence tomography method, photo-acoustic spectrometry, mid-infrared spectrometry and near-infrared spectrometry.

In a process of implementing concepts of the present disclosure, the inventors found that the related art has at least a problem that it is difficult to obtain a reliable measurement result by using the related art.

SUMMARY

In view of this, embodiments of the present disclosure provide a method of measuring a tissue element, a device of measuring a tissue element, and a wearable apparatus.

In an aspect of embodiments of the present disclosure, a method of measuring a tissue element is provided, including: irradiating a measurement region with incident light having a single predetermined wavelength, where each beam of the incident light passes through the measurement region to form at least one beam of exit light exited from at least one exit position, and a number of incident positions of the incident light is at least one; obtaining a light intensity value corresponding to each beam of the exit light acquired by M photosensitive surfaces , so as to obtain T output light intensities, where each of the T output light intensities is obtained by processing the light intensity value of the exit light acquired by one or more of the M photosensitive surfaces, and each of the M photosensitive surfaces is configured to acquire the light intensity value of the exit light exited from the exit position within a predetermined anti-jitter range corresponding to the photosensitive surface, and where $1 \leq T \leq M$; and determining a concentration of a detected tissue element according to at least one output light intensity corresponding to the predetermined wavelength.

In another aspect of embodiments of the present disclosure, a device of measuring a tissue element is provided, including: a light source module configured to irradiate a measurement region with incident light having a single predetermined wavelength, where each beam of the incident light passes through the measurement region to form at least one beam of exit light exited from at least one exit position, and a number of incident positions of the incident light is at least one; an acquisition module including M photosensitive surfaces, where each of the M photosensitive surfaces is configured to acquire the light intensity value of the exit light exited from the exit position within a predetermined anti-jitter range corresponding to the photosensitive surface, the acquisition module is configured to obtain a light intensity value corresponding to each beam of the exit light acquired by the M photosensitive surfaces, so as to obtain T output light intensities, and each of the T output light intensities is obtained by processing the light intensity value of the exit light acquired by one or more of the M photosensitive surfaces, and where $1 < T < M$; and a processing module configured to determine a concentration of a detected tissue element according to at least one output light intensity corresponding to the predetermined wavelength.

In another aspect of embodiments of the present disclosure, a wearable apparatus is provided, and the apparatus includes the device of measuring the tissue element as described above.

BRIEF DESCRIPTION OF THE DRAWINGS

The above and other objectives, features and advantages of the present disclosure will be clearer with following descriptions of embodiments of the present disclosure with reference to the accompanying drawings, in the drawings:

FIG. 1 schematically shows a schematic diagram of receiving exit light using a photosensitive surface with a small area when a jitter occurs according to embodiments of the present disclosure;

FIG. 2 schematically shows a schematic diagram of receiving exit light using a photosensitive surface with a large area when a jitter occurs according to embodiments of the present disclosure;

FIG. 3 schematically shows a flowchart of a method of measuring a tissue element according to embodiments of the present disclosure;

FIG. 4 schematically shows a schematic diagram of a measurement result obtained based on a Monte Carlo simulation method according to embodiments of the present disclosure;

FIG. 5 schematically shows a schematic diagram of positioning a measurement region based on an optical method according to embodiments of the present disclosure;

FIG. 6 schematically shows another schematic diagram of positioning a measurement region based on an optical method according to embodiments of the present disclosure;

FIG. 7 schematically shows a schematic diagram of positioning a measurement region based on an image matching method according to embodiments of the present disclosure;

FIG. 8 schematically shows another schematic diagram of positioning a measurement region based on an image matching method according to embodiments of the present disclosure;

FIG. 9 schematically shows a schematic diagram of positioning a measurement region based on an imaging method according to embodiments of the present disclosure;

FIG. 10 schematically shows another schematic diagram of positioning a measurement region based on an imaging method according to embodiments of the present disclosure;

FIG. 11 schematically shows a schematic diagram of positioning a measurement posture based on an optical method according to embodiments of the present disclosure;

FIG. 12 schematically shows a schematic diagram of positioning a measurement posture based on an image matching method according to embodiments of the present disclosure;

FIG. 13 schematically shows a schematic diagram of positioning a measurement posture based on an imaging method according to embodiments of the present disclosure;

FIG. 14 schematically shows a schematic diagram of a differential measurement according to embodiments of the present disclosure;

FIG. 15 schematically shows a schematic diagram of an annular photosensitive surface according to embodiments of the present disclosure;

FIG. 16 schematically shows a schematic diagram of a sector-annular photosensitive surface according to embodiments of the present disclosure;

FIG. 17 schematically shows a schematic diagram of a circular photosensitive surface according to embodiments of the present disclosure;

FIG. 18 schematically shows a schematic diagram of a square photosensitive surface according to embodiments of the present disclosure;

FIG. 19 schematically shows a schematic diagram of providing a mask on an initial photosensitive surface to obtain a photosensitive surface according to embodiments of the present disclosure;

FIG. 20 schematically shows a block diagram of a device of measuring a tissue element according to embodiments of the present disclosure;

FIG. 21 schematically shows a block diagram of another device of measuring a tissue element according to embodiments of the present disclosure;

FIG. 22 schematically shows a schematic diagram of a positional relationship between a fixing portion and a measurement probe according to embodiments of the present disclosure;

FIG. 23 schematically shows a schematic structural diagram of a fixing portion according to embodiments of the present disclosure;

FIG. 24 schematically shows a schematic diagram of a first fitting part according to embodiments of the present disclosure;

FIG. 25 schematically shows a schematic diagram of another first fitting part according to embodiments of the present disclosure;

FIG. 26 schematically shows a schematic diagram of a region positioning portion according to embodiments of the present disclosure;

FIG. 27 schematically shows a schematic diagram of another region positioning portion according to embodiments of the present disclosure;

FIG. 28 schematically shows a schematic diagram of a first image acquisition portion according to embodiments of the present disclosure;

FIG. 29 schematically shows a schematic diagram of a first posture positioning portion according to embodiments of the present disclosure;

FIG. 30 schematically shows a schematic diagram of another first posture positioning portion according to embodiments of the present disclosure;

FIG. 31 schematically shows a schematic diagram of a third image acquisition portion according to embodiments of the present disclosure;

FIG. 32 schematically shows a schematic diagram of positioning a measurement posture and a measurement region according to embodiments of the present disclosure;

FIG. 33 schematically shows another schematic diagram of positioning a measurement posture and a measurement region according to embodiments of the present disclosure;

FIG. 34 schematically shows a schematic diagram of an electrical connection of anodes of different photosensitive surfaces according to embodiments of the present disclosure;

FIG. 35 schematically shows a schematic diagram of a three-dimensional photosensitive surface in a form of a glove according to embodiments of the present disclosure;

FIG. 36 schematically shows a schematic diagram of another three-dimensional photosensitive surface in a form of a glove according to embodiments of the present disclosure;

FIG. 37 schematically shows a schematic diagram of a three-dimensional photosensitive surface in a form of a finger ring according to embodiments of the present disclosure;

FIG. 38 schematically shows a schematic diagram of another three-dimensional photosensitive surface in a form of a finger ring according to embodiments of the present disclosure;

FIG. 39 schematically shows a schematic diagram of a three-dimensional photosensitive surface for an on-arm measurement according to embodiments of the present disclosure;

FIG. 40 schematically shows a schematic diagram of providing a first sleeve on a measurement probe according to embodiments of the present disclosure;

FIG. 41 schematically shows a schematic diagram of providing a second sleeve outside a target region of the first sleeve according to embodiments of the present disclosure;

FIG. 42 schematically shows a schematic diagram of a photosensitive surface receiving exit light without a refractive index matcher being filled according to embodiments of the present disclosure;

FIG. 43 schematically shows a schematic diagram of a photosensitive surface receiving exit light in a case of a refractive index matcher being filled according to embodiments of the present disclosure;

FIG. 44 schematically shows another schematic diagram of a photosensitive surface receiving exit light in a case of a refractive index matcher being filled according to embodiments of the present disclosure;

FIG. 45 schematically shows a schematic diagram of a diffusion measurement according to embodiments of the present disclosure;

FIG. 46 schematically shows a schematic diagram of a wearable apparatus according to embodiments of the present disclosure;

FIG. 47 schematically shows a schematic diagram of an assembly process of a wearable apparatus according to embodiments of the present disclosure;

FIG. 48 schematically shows a schematic diagram of maintaining an average optical path of exit light received by a measurement probe within a predetermined optical path range during a skin jitter process in a case that a mode of a wearable apparatus is identical to a skin jitter mode according to embodiments of the present disclosure;

FIG. 49 schematically shows a schematic diagram of maintaining an average optical path of exit light received by a measurement probe within a predetermined optical path range during a skin jitter process in a case that a wearable apparatus causes a movement amplitude of a skin at a measurement region to be less than or equal to a movement amplitude threshold according to embodiments of the present disclosure;

FIG. 50 schematically shows a schematic diagram of synchronously positioning a measurement region and a measurement posture based on an optical method according to embodiments of the present disclosure;

FIG. 51 schematically shows a schematic diagram of a change in a variation of a differential signal obtained by measuring a standard reflective plate over a measurement duration according to embodiments of the present disclosure;

FIG. 52 schematically shows a schematic diagram of a change in a variation of a differential signal obtained by measuring a forearm extensor side of a detected object in a stable state of a concentration of a blood glucose over a measurement duration according to embodiments of the present disclosure;

FIG. 53 schematically shows a schematic diagram of a result of a single glucose loading experiment using an OGTT method according to embodiments of the present disclosure;

FIG. 54 schematically shows a schematic diagram of a relationship between a variation of a differential signal and a true value of a blood glucose according to embodiments of the present disclosure;

FIG. 55 schematically shows a schematic diagram of a result of a double glucose loading experiment using an MTT method according to embodiments of the present disclosure; and

FIG. 56 schematically shows a schematic diagram of a relationship between a predicted value of a blood glucose and a true value of the blood glucose according to embodiments of the present disclosure.

DETAILED DESCRIPTION OF EMBODIMENTS

Embodiments of the present disclosure will be described below with reference to the accompanying drawings. It should be understood, however, that these descriptions are merely exemplary and are not intended to limit the scope of the present disclosure. In the following detailed descriptions, for ease of interpretation, many specific details are set forth to provide a comprehensive understanding of embodiments of the present disclosure. However, it is clear that one or more embodiments may also be implemented without these specific details. In addition, in the following descriptions, descriptions of well-known structures and technologies are omitted to avoid unnecessarily obscuring concepts of the present disclosure.

Terms used herein are for the purpose of describing specific embodiments only and are not intended to limit the present disclosure. The terms "including", "containing", etc. used herein indicate the presence of the feature, step, operation and/or component, but do not exclude the presence or addition of one or more other features, steps, operations or components.

All terms used herein (including technical and scientific terms) have the meanings generally understood by those skilled in the art, unless otherwise defined. It should be noted that the terms used herein shall be interpreted to have meanings consistent with the context of this specification, and shall not be interpreted in an idealized or overly rigid manner.

In a case of using the expression similar to "at least one selected from A, B, and C", it should be explained according to the meaning of the expression generally understood by those skilled in the art (for example, "a system including at least one selected from A, B, and C" should include but not be limited to a system including A alone, a system including B alone, a system including C alone, a system including A and B, a system including A and C, a system including B and C, and/or a system including A, B and C). In a case of using the expression similar to "at least one selected from A, B, or C", it should be explained according to the meaning of the expression generally understood by those skilled in the art (for example, "a system including at least one selected from A, B, or C" should include but not be limited to a system including A alone, a system including B alone, a system including C alone, a system including A and B, a system including A and C, a system including B and C, and/or a system including A, B and C).

A research on a living tissue element measurement based on an optical method has experienced nearly fifty years of development, and a large number of scientific research institutes and companies have invested great research enthusiasm in this field. Due to a weak absorption of a detected tissue element and a small variation range of a concentration of the detected tissue element of the detected object, a signal of the detected tissue element is generally weak, and the weak signal of the detected tissue element may be easily submerged by an interference such as a change in a measurement condition. Up to now, there has not appeared a solution that may achieve a reliable living tissue element measurement. Therefore, the living tissue element measurement is a global problem that needs to be solved. The tissue element may include blood glucose, fat, and white blood cells, etc. The signal of the detected tissue element represents a change in an output light intensity caused by a change in a concentration of the detected tissue element. The measurement condition may be understood as a condition that affects a transmission path of light. The measurement condition may include a controllable measurement condition and an uncontrollable measurement condition. The controllable measurement condition refers to a measurement condition that may be controlled within a predetermined change range (that is, kept unchanged or substantially unchanged) by adopting an effective control method during each process of measuring the tissue element. The uncontrollable measurement condition refers to a measurement condition with unpredictable and uncontrollable characteristics. The controllable measurement condition may include a temperature, a pressure, a measurement region and a measurement posture, etc. The uncontrollable measurement condition may include a change in a physiological background and a drift of a measurement device, etc.

In a process of implementing concepts of the present disclosure, the inventors found that main causes for the difficulty in obtaining a reliable measurement result by using the related art are as follows.

The inventors found that: different measurement results may be obtained if only an intensity distribution of a light spot irradiated to a measurement region by incident light is changed while other conditions remain unchanged; and if a measurement result obtained when arranging a photosensitive surface close to a blood vessel is compared with a measurement result obtained when arranging the same photosensitive surface far away from the blood vessel while keeping other conditions unchanged, the measurement result obtained when arranging the photosensitive surface far away from the blood vessel is better than that obtained when arranging the photosensitive surface close to the blood vessel. The measurement result may be represented by a relative variation of a light intensity value of exit light received by the photosensitive surface or a standard deviation of the light intensity value. When studying the causes for the different measurement results, it was found that changing the intensity distribution of the light spot irradiated by the incident light on the measurement region may reflect a randomness of an illumination of a light source, and a distance to the blood vessel may reflect a strength of a pulse beat. The randomness of the illumination of the light source and the pulse beat are both sources of jitter. Therefore, it has been found that one of the causes for the difficulty in obtaining a reliable measurement result is the jitter.

On the basis of a research on the jitter, it was found that a source of the jitter may be classified into an internal source and an external source. The internal source may further include a change in the physiological background in addition to the pulse beat. The external source may further include an uncertainty of a transmission of the incident light in addition to the randomness of the illumination of the light source. The randomness of the illumination of the light source may be reflected by the intensity distribution of the light spot irradiated by the incident light on the measurement region. It was found that both the jitter caused by the internal source and the jitter caused by the external source may affect a transmission path of light in the tissue, and then affect the intensity distribution of the exit light on the measurement region.

In order to solve the problem of a low reliability of the measurement result caused by the jitter, the inventors found that a solution of acquiring the light intensity value of the exit light by using a photosensitive surface with a large area (that is, a large-area photosensitive surface) may be adopted to effectively reduce an adverse influence of the jitter on the measurement result. That is, the large-area photosensitive surface may effectively reduce the adverse influence caused by the jitter. The so-called "large-area photosensitive surface" may be understood as a photosensitive surface with such area that the photosensitive surface may acquire the light intensity value of the exit light exited from an exit position within a predetermined anti-jitter range. The area of the large-area photosensitive surface is continuous. The large-area photosensitive surface is made of a photosensitive material, and it is different from a single-point optical fiber reception and a joint reception of multiple single optical fibers. A description will be given below to explain in detail why the solution of acquiring the output light intensity of the exit light by using the large-area photosensitive surface may be adopted to effectively reduce the adverse influence of the jitter on the measurement result.

In the large-area photosensitive surface, a ratio of an area of the photosensitive surface that may stably receive the exit light to the area of the photosensitive surface may be increased, so that a stability of receiving the exit light may be improved, an adverse influence of a change in the intensity distribution of the exit light caused by the jitter may be reduced, and then the reliability of the measurement result may be improved. The stability may be represented by a relative variation of the light intensity value of the exit light received by the photosensitive surface or a standard deviation of the light intensity value. The less the relative variation of the light intensity value, the higher the stability, and the less the standard deviation of the light intensity value, the higher the stability.

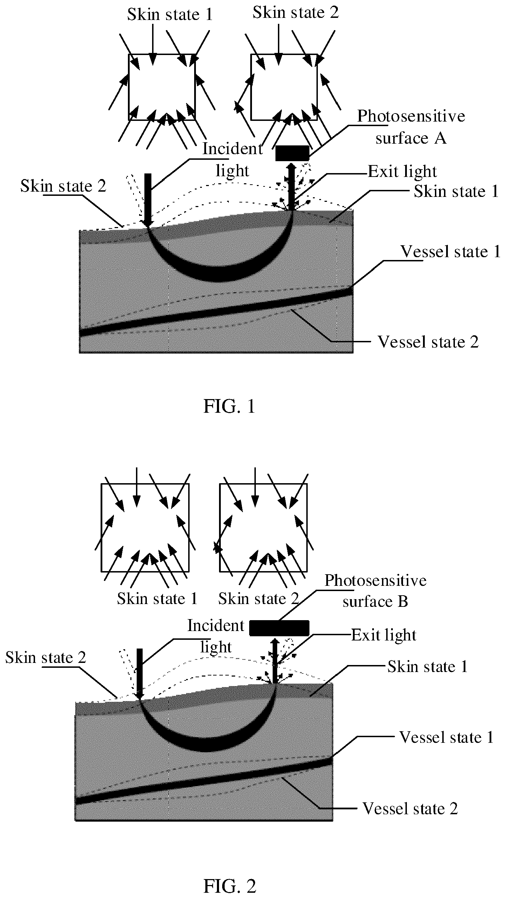

Illustratively, the jitter caused by the pulse beat is taken as an example for description. The pulse beat may be reflected by a blood vessel state. FIG. 1 schematically shows a schematic diagram of receiving the exit light using a photosensitive surface with a small area when a jitter occurs according to embodiments of the present disclosure. FIG. 2 schematically shows a schematic diagram of receiving the exit light using a photosensitive surface with a large area when a jitter occurs according to embodiments of the present disclosure. The jitter occurs in FIG. 1 is the same as that occurs in FIG. 2. An area of a photosensitive surface A in FIG. 1 is less than an area of a photosensitive surface B in FIG. 2. Both the photosensitive surface A and the photosensitive surface B are square photosensitive surfaces. In FIG. 1 and FIG. 2, a blood vessel state 1 represents a vasoconstriction state, a blood vessel state 2 represents a vasodilation state, a skin state 1 represents a skin state corresponding to the blood vessel state 1, and a skin state 2 represents a skin state corresponding to the blood vessel state 2. A change from the skin state 1 to the skin state 2 reflects a jitter.

The measurement results obtained using photosensitive surfaces with different areas when the same jitter occurs are compared. The measurement result is represented by the relative variation of the light intensity value of the exit light received by the photosensitive surface within a predetermined time period or the standard deviation of the light intensity value. The relative variation of the light intensity value may be determined by: calculating a difference between a maximum light intensity value and a minimum light intensity value within the predetermined time period, calculating an average value of the light intensity values within the predetermined time period, calculating a ratio of the difference to the average value, and determining the ratio as the relative variation of the light intensity value. The predetermined time period may be a pulsation cycle.

The measurement results also show that the measurement result obtained by using the photosensitive surface B is better than the measurement result obtained by using the photosensitive surface A whether the measurement result is represented by the relative variation of the light intensity value of the exit light received by the photosensitive surface or the measurement result is represented by the standard deviation of the light intensity value of the exit light received by the photosensitive surface.

As the area of the photosensitive surface B is greater than the area of the photosensitive surface A, it may indicate that the large-area photosensitive surface may improve the stability of receiving the exit light, and then reduce the adverse influence of the change in the intensity distribution of the exit light caused by the jitter, thereby improving a measurement accuracy.

In addition, the output light intensity of the exit light is weak, the change in the output light intensity caused by a change in the concentration of the detected tissue element is also weak, and the method of receiving the exit light adopted in the related art has a low efficiency of receiving the exit light, so that the received output light intensity has a low signal-to-noise ratio, which results in a low reliability of the measurement result. The large-area photosensitive surface of embodiments of the present disclosure may improve the signal-to-noise ratio of the output light intensity, thereby improving the reliability of the measurement result. This is because the large-area photosensitive surface may receive a wide range of exit light and improve the efficiency of receiving the exit light, so that the signal-to-noise ratio of the output light intensity may be increased, and the reliability of the measurement result may be improved.

It should be noted that the large-area photosensitive surface described in embodiments of the present disclosure may achieve a high stability and efficiency of receiving the exit light in a case of a small distance to a surface of the measurement region, that is, in a case that the large-area photosensitive surface is close to the surface of the measurement region. This may not be achieved by using a single-point optical fiber reception and a joint reception of multiple single optical fibers due to a constraint of a numerical aperture of the optical fiber and a limitation of a state change of the optical fiber. The state of the optical fiber is easily affected by an environment, and the state change of the optical fiber has a great influence on the stability of receiving the exit light.

It should also be noted that the large-area photosensitive surface is generally adopted to improve the signal-to-noise ratio of the output light intensity. In other words, a function of the large-area photosensitive surface is generally to increase the signal-to-noise ratio of the output light intensity, which is different from a main function of the large-area photosensitive surface in embodiments of the present disclosure. In embodiments of the present disclosure, the main function of the large-area photosensitive surface is to effectively suppress the jitter.

Furthermore, in the process of achieving concepts of the present disclosure, the inventors found that in the related art, multi-wavelength spectral data is generally processed using a multivariate analysis method, that is, a mathematical model between an optical signal and a true value of the concentration of the detected tissue element is established using the multivariate analysis method, and the concentration of the detected tissue element is predicted using the established mathematical model, so that a signal of the detected tissue element may be obtained indirectly. It is believed that the living tissue element measurement may only be achieved using the multivariate analysis method, because a tissue element and a physical state (such as temperature and pressure, etc.) have a characteristic absorption in a predetermined band and it is generally believed that the multivariate analysis method is a potential tool for an interference correction in the living tissue element measurement. The predetermined band may include a visible-near infrared band. In view of the above-mentioned properties of the multivariate analysis method, some researchers have over trusted a reliability of the multivariate analysis method, and an improvement in the related art is also made in a direction of the multivariate analysis method.

However, the above-mentioned method has the following problems. A signal change caused by the change in the measurement condition is generally much greater than a signal change caused by the change in the concentration of the detected tissue element, and the measurement result obtained using the multivariate analysis method is likely to be accidentally related with a signal change caused by an interference (e.g., interference from the physiological background) other than the detected tissue element, so that such result obtained by indirectly extracting the signal of the detected tissue element may be a pseudo-correlation result.

Since the measurement result obtained by the above-mentioned method may be a pseudo-correlation result, it may not directly prove that the obtained signal of the detected tissue element is a real signal of the detected tissue element, and thus may not directly prove a feasibility of the living tissue element measurement. Accordingly, the measurement result has a low reliability.

Directly obtaining a real signal of the detected tissue element is a prerequisite for achieving the living tissue element measurement. In order to ensure that the real signal of the detected tissue element may be obtained to improve the reliability of the measurement result, embodiments of the present disclosure propose a solution of performing a living tissue element measurement by using a single predetermined wavelength combined with a large-area photosensitive surface.

Moreover, in order to achieve an application of a device of measuring a living tissue element and a wearable apparatus provided with the device of measuring the living tissue element, it is needed to minimize volumes of the device and the apparatus and minimize a power consumption to facilitate a practical use. When a single predetermined wavelength is used, a volume and a structural complexity of a light source module may be reduced, so that the volume and structural complexity of the device and the apparatus may be reduced to facilitate a portable measurement. A capacity requirement for a power module may also be reduced, so that a production cost may be reduced. In addition, an amount of data processing may also be reduced.

A description will be given below in conjunction with specific embodiments.

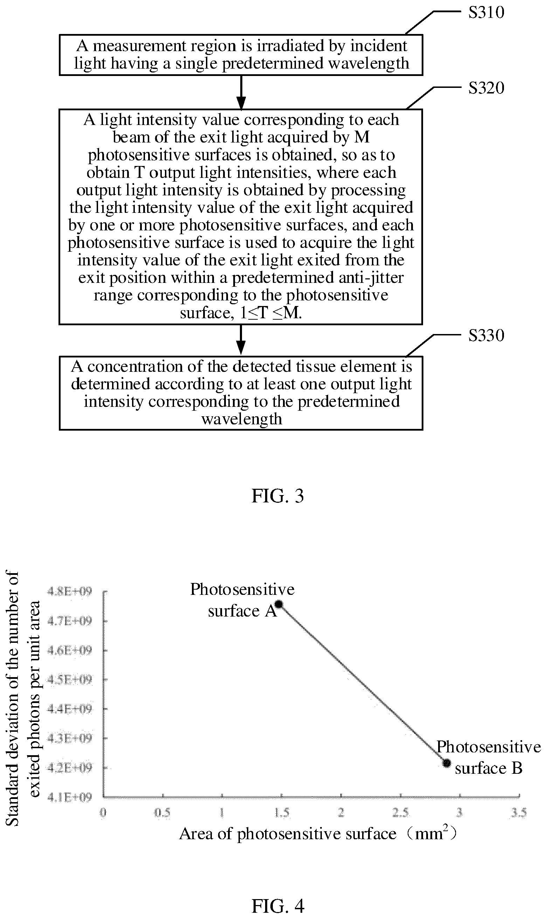

FIG. 3 schematically shows a flowchart of a method of measuring a tissue element according to embodiments of the present disclosure.

As shown in FIG. 3, the method includes operations S310 to S330.

In operation S310, a measurement region is irradiated by incident light having a single predetermined wavelength. Each beam of incident light passes through the measurement region to form at least one beam of exit light exited from at least one exit position, and the number of incident positions of the incident light is at least one.

According to embodiments of the present disclosure, as different measurement sites have different skin characteristics, including smoothness, hair presence or absence, flatness, skin thickness and softness, etc., it is needed to select an appropriate measurement site according to an actual situation, such as a structure of a measurement probe.

The measurement site may include at least one selected from a finger, a palm, an arm, a forehead, or an earlobe. The measurement region may be a region on the measurement site.

According to embodiments of the present disclosure, the single predetermined wavelength may be a wavelength sensitive to the detected tissue element. A band to which the single predetermined wavelength belongs may include an ultraviolet band, a visible light band, a near-infrared band, a mid-infrared band or a far-infrared band. In an example, if the detected tissue element is blood glucose, the single predetermined wavelength may be a wavelength sensitive to blood glucose accordingly, which may be specifically 1550 nm or 1609 nm. The incident light may be collimated or non-collimated. There may be one or more incident positions of the incident light.

In operation S320, a light intensity value corresponding to each beam of the exit light acquired by M photosensitive surfaces is obtained, so as to obtain T output light intensities. Each output light intensity is obtained by processing the light intensity value of the exit light acquired by one or more photosensitive surfaces, and each photosensitive surface is used to acquire the light intensity value of the exit light exited from the exit position within a predetermined anti-jitter range corresponding to the photosensitive surface. $1<T \leq M$.

According to embodiments of the present disclosure, in order to improve the reliability of the measurement result, each photosensitive surface may acquire the light intensity value of the exit light exited from the exit position within the predetermined anti-jitter range corresponding to the photosensitive surface, which requires the area of the photosensitive surface to be as large as possible. Each photosensitive surface corresponds to a predetermined anti-jitter range, and different photosensitive surfaces correspond to the same predetermined anti-jitter range or different predetermined anti-jitter ranges. A description will be given below from three aspects with examples to illustrate that the greater the area of the photosensitive surface, the better the effect of suppressing the jitter. In the examples, the area of the photosensitive surface A is less than the area of the photosensitive surface B, and both the photosensitive surface A and the photosensitive surface B are square photosensitive surfaces.

In a first aspect, a jitter caused by a pulse beat may be suppressed. The photosensitive surface A and the photosensitive surface B are respectively arranged at a same position in the measurement region, which is a position close to the blood vessel. A measurement result obtained by using the photosensitive surface A may be compared with a measurement result obtained by using the photosensitive surface B when other conditions are the same. The measurement result is represented by a relative variation of the light intensity value or a standard deviation of the light intensity value of the exit light received by the photosensitive surface within a pulsation cycle. The method of calculating the relative variation of the light intensity value is as described above, and will not be repeated here. It was found that the relative variation of the light intensity value of the exit light received by the photosensitive surface B is less than the relative variation of the light intensity value of the exit light received by the photosensitive surface A, and the standard deviation of the light intensity value of the exit light received by the photosensitive surface B is less than the standard deviation of the light intensity value of the exit light received by the photosensitive surface A. Therefore, it may be concluded that the measurement result obtained by using the photosensitive surface B is better than the measurement result obtained by using the photosensitive surface A whether the measurement result is represented by the relative variation of the light intensity value of the exit light received by the photosensitive surface or the measurement result is represented by the standard deviation of the light intensity value of the exit light received by the photosensitive surface.

As the measurement result obtained by using the photosensitive surface B is better than the measurement result obtained by using the photosensitive surface A, and the area of the photosensitive surface B is greater than the area of the photosensitive surface A, it may indicate that the greater the area of the photosensitive surface, the better the effect of suppressing the jitter caused by the pulse beat.

In a second aspect, a jitter caused by a change in the intensity distribution of the light spot irradiated to the measurement region by the incident light may be suppressed. The intensity distribution of the light spot irradiated to the measurement region by the incident light may be changed while other conditions remain unchanged. The measurement result obtained by using the photosensitive surface A may be compared with the measurement result obtained by using the photosensitive surface B. The measurement result is represented by a relative variation of the light intensity value or a standard deviation of the light intensity value of the exit light received by the photosensitive surface within a predetermined period. The method of calculating the relative variation of the light intensity value is as described above, and will not be repeated here. It was found that the relative variation of the light intensity value of the exit light received by the photosensitive surface B is less than the relative variation of the light intensity value of the exit light received by the photosensitive surface A, and the standard deviation of the light intensity value of the exit light received by the photosensitive surface B is less than the standard deviation of the light intensity value of the exit light received by the photosensitive surface A. Therefore, it may be concluded that the measurement result obtained by using the photosensitive surface B is better than the measurement result obtained by using the photosensitive surface A whether the measurement result is represented by the relative variation of the light intensity value of the exit light received by the photosensitive surface or the measurement result is represented by the standard deviation of the light intensity value of the exit light received by the photosensitive surface.

As the measurement result obtained by using the photosensitive surface B is better than the measurement result obtained by using the photosensitive surface A, and the area of the photosensitive surface B is greater than the area of the photosensitive surface A, it may indicate that the greater the area of the photosensitive surface, the better the effect of suppressing the jitter caused by the change in the intensity distribution of the light spot irradiated to the measurement region by the incident light.

In a third aspect, a jitter caused by an uncertainty of the transmission of the incident light may be suppressed. A Monte Carlo simulation method is used, where a central incidence is performed using incident light with a photon number of $10^{15}$, the photosensitive surface A and the photosensitive surface B are respectively arranged at a distance of 2.4 mm from the center of the incident light, and the simulation is performed twenty-two times. The measurement result obtained by using the photosensitive surface A may be compared with the measurement result obtained by using the photosensitive surface B. The measurement result is represented by a standard deviation of the number of exited photons per unit area. The less the standard deviation of the number of exited photons per unit area, the better the suppression effect. FIG. 4 schematically shows a schematic diagram of the measurement result obtained based on the Monte Carlo simulation method according to embodiments of the present disclosure. It was found that the standard deviation of the number of exited photons per unit area corresponding to the photosensitive surface B is less than the standard deviation of the number of exited photons per unit area corresponding to the photosensitive surface A. That is, the measurement result obtained by using the photosensitive surface B is better than the measurement result obtained by using the photosensitive surface A.

As the measurement result obtained by using the photosensitive surface B is better than the measurement result obtained by using the photosensitive surface A, and the area of the photosensitive surface B is greater than the area of the photosensitive surface A, it may indicate that the greater the area of the photosensitive surface, the better the effect of suppressing the jitter caused by the uncertainty of the transmission of the incident light.

Through the examples from the above three aspects, it may indicate that the greater the area of the photosensitive surface, the better the effect of suppressing the adverse influence of the jitter on the measurement result.

According to embodiments of the present disclosure, each photosensitive surface may be an annular photosensitive surface or a non-annular photosensitive surface. The non-annular photosensitive surface may include a sector-annular photosensitive surface, a circular photosensitive surface, a sector photosensitive surface, an elliptical photosensitive surface, or a polygonal photosensitive surface. The polygonal photosensitive surface may include a square photosensitive surface, a rectangular photosensitive surface, or a triangular photosensitive surface.

According to embodiments of the present disclosure, each of the M photosensitive surfaces may be used separately, partially in combination, or all in combination. Used in combination means outputting one output light intensity. In embodiments of the present disclosure, the photosensitive surfaces for outputting one output light intensity are referred to as a homogeneous photosensitive surface. The homogeneous photosensitive surface may include one or more photosensitive surfaces. A condition for using different photosensitive surfaces in combination may be that an average optical path of the exit light received by the photosensitive surfaces is within an average optical path range. The average optical path range may be a range that is greater than or equal to a first average optical path threshold and less than or equal to a second average optical path threshold. The first average optical path threshold and the second average optical path threshold may be determined according to an optical path average value and an optical path change amplitude. The optical path average value is an average value calculated according to the average optical paths of the exit light received at the photosensitive positions of the homogeneous photosensitive surface. In an example, if the optical path average value is a, and the optical path change amplitude is $\pm 30\%$, then the first average optical path threshold may be 0.7a, and the second average optical path threshold may be 1.3a.

The average optical path is explained as follows. A transmission path of light in a tissue may be represented by an optical path and a penetration depth. The optical path is used to represent a total distance that light travels in the tissue, and the penetration depth is used to represent a maximum longitudinal distance that light may reach in the tissue. For a determined source-detection distance, the average optical path is used to represent an average value of the optical path of light in the tissue. A probability distribution function of the optical path may be understood as a function of the source-detection distance and a tissue optical parameter. The source-detection distance represents a radial distance between a center of the incident light and a center of the photosensitive surface. Accordingly, in terms of mathematical expressions, the average optical path may be understood as a function of the source-detection distance and the tissue optical parameter. The tissue optical parameter may include an absorption coefficient, a scattering coefficient, and an anisotropy factor. A factor affecting the average optical path may include the absorption coefficient, the scattering coefficient, the anisotropy factor, and the source-detection distance.

According to embodiments of the present disclosure, the homogeneous photosensitive surface may be an annular photosensitive surface or a non-annular photosensitive surface. The homogeneous photosensitive surface being an annular photosensitive surface may include that the homogeneous photosensitive surface includes one photosensitive surface, and the homogeneous photosensitive surface is an independent annular photosensitive surface; or the homogeneous photosensitive surface includes a plurality of photosensitive surfaces, and the homogeneous photosensitive surface is an annular photosensitive surface formed by combining the plurality of photosensitive surfaces. The homogeneous photosensitive surface being a non-annular photosensitive surface may include that the homogeneous photosensitive surface includes one photosensitive surface, and the homogeneous photosensitive surface is an independent non-annular photosensitive surface; or the homogeneous photosensitive surface includes a plurality of photosensitive surfaces, and the homogeneous photosensitive surface is a non-annular photosensitive surface formed by combining the plurality of photosensitive surfaces.

In operation S330, a concentration of the detected tissue element is determined according to at least one output light intensity corresponding to the predetermined wavelength.

According to embodiments of the present disclosure, after at least one output light intensity corresponding to the predetermined wavelength is obtained, the at least one output light intensity corresponding to the predetermined wavelength may be processed using an interference suppression method to determine the concentration of the detected tissue element. The interference suppression method may include a differential measurement method. The differential measurement method may include a temporal differential measurement method or a position differential measurement method. Alternatively, the at least one output light intensity may also be processed using a non-differential measurement method to determine the concentration of the detected tissue element. Each output light intensity may include a diffusely-scattered light intensity or a diffusely-transmitted light intensity.

According to technical solutions of embodiments of the present disclosure, the photosensitive surface may acquire the light intensity value of the exit light exited from the exit position within the corresponding predetermined anti-jitter range. For the photosensitive surface with the above-mentioned characteristics, a ratio of the area that may stably receive the exit light to the area of the photosensitive surface is increased, so that the stability of receiving the exit light is improved, the adverse influence of the change in the intensity distribution of the exit light caused by the jitter is reduced, and the reliability of the measurement result is improved. Moreover, a real signal of the detected tissue element may be directly obtained by performing a tissue element measurement using the single predetermined wavelength combined with the photosensitive surface with the above-mentioned characteristics. When the single predetermined wavelength is used for the tissue element measurement, the volume and structural complexity of the light source module may be reduced, so that the volume and structural complexity of the device and the apparatus may be reduced to facilitate a portable measurement. The capacity requirement for the power module may also be reduced, so that the production cost may be reduced. In addition, the amount of data processing may also be reduced.

According to embodiments of the present disclosure, a ratio of an average optical path of the exit light received by each photosensitive surface in a target tissue layer to a total optical path is greater than or equal to a ratio threshold. The total optical path is a total distance that the exit light travels in the measurement region.

According to embodiments of the present disclosure, a tissue model of the detected object is generally a layered structure, that is, it may be divided into one or more layers. Different tissue layers carry different information of the detected tissue element. In order to improve the reliability of the measurement result, it is required to ensure that the transmission path of the exit light mainly passes through the tissue layer that carries a rich information of the detected tissue element. A target tissue layer may be understood as the tissue layer that carries a rich information of the detected tissue element, or the tissue layer that is a main source of the detected tissue element. In the following description, a human body is taken as an example of the detected object, and blood glucose is taken as an example of the detected tissue element.

A skin tissue model of the human body may be understood as a three-layer model, including epidermis, dermis and subcutaneous fat layer from outside to inside. The epidermis contains a small amount of interstitial fluid, and does not contain plasma and lymph. The dermis contains a large amount of interstitial fluid, and further contains a large amount of plasma and a small amount of lymph due to the presence of abundant capillaries. The subcutaneous fat layer contains a small amount of cellular fluid, and contains a large amount of plasma and a small amount of lymph due to the presence of blood vessels such as veins and arteries. Therefore, different tissue layers carry different information of the detected tissue element.

Since the epidermis contains a small amount of interstitial fluid, it is not an appropriate source of the blood glucose information. Although the subcutaneous fat layer contains a large amount of plasma and a relatively small amount of interstitial fluid, it is still not an appropriate source of the blood glucose information due to a limitation of the penetration depth of the incident light. The dermis contains abundant capillaries and a large amount of interstitial fluid, and the incident light may easily reach the dermis. Therefore, the dermis may be used as a main source of the blood glucose information. Accordingly, the target tissue layer may be the dermis.

According to embodiments of the present disclosure, the average optical path of the exit light in each tissue layer may be determined according to the optical path and the penetration depth.

In order to ensure that the transmission path of the exit light mainly passes through the target tissue layer, it is required to ensure that a ratio of the average optical path of the exit light received by each photosensitive surface in the target tissue layer to the total optical path is greater than or equal to a ratio threshold. The total optical path may be a total distance that the exit light travels in the measurement region, that is, a total distance of a path that the incident light travels from entering the measurement region, travelling in the measurement region, to reaching the exit position. The ratio threshold is related to the tissue optical parameter and the source-detection distance between the center of the photosensitive surface and the center of the incident light.

It should be noted that, as the ratio of the average optical path of the exit light received by the photosensitive surface in the target tissue layer to the total optical path is limited in embodiments of the present disclosure, the area of the photosensitive surface in embodiments of the present disclosure may not be overly large, and it is a large area within an area range.

According to embodiments of the present disclosure, a total area of the homogeneous photosensitive surface is determined according to a tissue structure feature in the measurement region. The homogeneous photosensitive surface includes one or more photosensitive surfaces, and the homogeneous photosensitive surface is used to output one output light intensity.

According to embodiments of the present disclosure, the total area of the homogeneous photosensitive surface may be determined according to the tissue structure feature in the measurement region. The tissue structure feature may be understood as a structure feature in the measurement region.

For example, when the measurement region is a region where three blood vessels intersect, if the homogeneous photosensitive surface is arranged in the region where the three blood vessels intersect, the total area of the homogeneous photosensitive surface is limited to an area of the region where the three blood vessels intersect, that is, the total area of the homogeneous photosensitive surface needs to be determined according to the area of the region where the three blood vessels intersect.

For another example, when the measurement region is a region where a finger is located, if the homogeneous photosensitive surface is arranged in the region where the finger is located, the total area of the homogeneous photosensitive surface is limited by an area of the region where the finger is located, that is, the total area of the homogeneous photosensitive surface needs to be determined according to the area of the region where the finger is located.

It should be noted that the area of the photosensitive surface in embodiments of the present disclosure may be determined according to the tissue structure feature, and an area that is determined according to the tissue structure feature may generally not be overly large. Therefore, the area of the photosensitive surface in embodiments of the present disclosure may not be overly large, and it is a large area within the area range.

According to embodiments of the present disclosure, a ratio of the area of each photosensitive surface to a circumference of the photosensitive surface is greater than or equal to a ratio threshold.

According to embodiments of the present disclosure, the reason why the influence of the jitter caused by the uncertainty of the transmission of the incident light, the randomness of the light source, the change in the physiological background and the pulse beat on the distribution of the exit light in the measurement region may be reduced by maximizing the ratio of the area of the photosensitive surface to the circumference of the photosensitive surface is as follows.

For ease of explanation, the photosensitive surface is divided into two portions, including an edge portion and a non-edge portion (or inner portion). Generally, the jitter mainly affects the exit light acquired by the edge portion, while the non-edge portion is less affected, that is, the non-edge portion may acquire the exit light relatively stably. From another perspective, when the jitter occurs, the intensity distribution of the exit light in the measurement region may change slightly. The light intensity value of the exit light received by the edge portion may change greatly with the change in the intensity distribution of the exit light, but most of the exit light for the non-edge portion may be acquired by the photosensitive surface relatively stably, so that the light intensity value of the exit light received by the non-edge portion may remain relatively stable. Therefore, it is possible to maximize a ratio of an area corresponding to the non-edge portion to the area of the photosensitive surface in order to effectively suppress the adverse influence of the jitter on the measurement result, and the larger the ratio, the better the effect of reducing the adverse influence. The edge portion may be represented by the circumference of the photosensitive surface, and the non-edge portion may be represented by the area of the photosensitive surface. Thus, the ratio of the area of the photosensitive surface to the circumference of the photosensitive surface is required to be as large as possible.

For example, a photosensitive surface 1 is a circular photosensitive surface, and a photosensitive surface 2 is a square photosensitive surface. In a case of a same circumference, as the area of the photosensitive surface 1 is greater than the area of the photosensitive surface 2, the ratio of the area of the photosensitive surface 1 to the circumference of the photosensitive surface 1 is greater than the ratio of the area of the photosensitive surface 2 to the circumference of the photosensitive surface 2. Therefore, the effect of the photosensitive surface 1 reducing the adverse influence is better than the effect of the photosensitive surface 2 reducing the adverse influence.

It should be noted that the description for the ratio of the area of the photosensitive surface to the circumference of the photosensitive surface being greater than or equal to the ratio threshold is given on the basis of meeting the condition that the area of the photosensitive surface is greater than or equal to the area threshold. For most shapes of photosensitive surfaces, if the ratio of the area of the photosensitive surface to the circumference of the photosensitive surface is greater than or equal to the ratio threshold, a size of the area of the photosensitive surface is actually limited, because for most shapes of patterns, the ratio of the area of the pattern to the circumference of the pattern has a positive correlation with the size of the area, that is, the greater the ratio of the area of the pattern to the circumference of the pattern, the larger the area of the pattern.

For example, in a case of a circle, an area of the circle is $\pi R^2$, and a ratio of the area of the circle to the circumference of the circle is R/2, where R represents a radius. The ratio of the area of the circle to the circumference of the circle is only related to the radius, and the size of the area of the circle is only related to the radius. Therefore, the ratio of the area of the circle to the circumference of the circle has a positive correlation with the size of the area. If the ratio of the area of the circle to the circumference of the circle is defined, then the size of the area of the circle is also defined. For another example, in a case of a square, an area of the square is $a^2$, and a ratio of the area of the square to the circumference of the square is $a$/4, where $a$ represents a side length. The ratio of the area of the square to the circumference of the square is only related to the side length, and the size of the area of the square is only related to the side length. Therefore, the ratio of the area of the square to the circumference of the square has a positive correlation with the size of the area. If the ratio of the area of the square to the circumference of the square is defined, the size of the area of the square is also defined.

According to embodiments of the present disclosure, the ratio threshold is greater than or equal to 0.04 mm.

According to embodiments of the present disclosure, the area of the photosensitive surface in the present disclosure is a relatively large area, that is, the area of the photosensitive surface is a large area within an area range. A description will be given below for this case.

In an aspect, the area of the photosensitive surface may not be too small. The large-area photosensitive surface in embodiments of the present disclosure refers to a photosensitive surface with such area that the photosensitive surface may acquire the light intensity value of the exit light exited from the exit position within the predetermined anti-jitter range. Therefore, the large-area photosensitive surface in embodiments of the present disclosure has a large area used to achieve anti-jitter. Furthermore, the ratio of the area of the photosensitive surface to the circumference of the photosensitive surface may be used to indicate the area of the photosensitive surface allowing the photosensitive surface to acquire the light intensity value of the exit light exited from the exit position within the predetermined anti-jitter range, and the ratio of the area to the circumference of the photosensitive surface generally has a positive correlation with the area of the photosensitive surface. Therefore, if the ratio of the area to the circumference of the photosensitive surface is greater than or equal to the ratio threshold, the size of the area of the photosensitive surface is also actually defined, that is, it is also defined that the area of the photosensitive surface may not be too small by defining that the ratio of the area to the circumference of the photosensitive surface is greater than or equal to the ratio threshold.

In another aspect, the area of the photosensitive surface may not be overly large. In embodiments of the present disclosure, it is required that the ratio of the average optical path of the exit light received by the photosensitive surface in the target tissue layer to the total optical path is greater than or equal to the ratio threshold, and/or the area of the photosensitive surface is determined according to the tissue structure feature, so that the area of the photosensitive surface may not be overly large.

Therefore, the area of the photosensitive surface in embodiments of the present disclosure is a relatively large area, that is, a large area within the area range.

In addition, there may be a case that the photosensitive surface has a large area and also has a large circumference, so that the ratio of the area of the photosensitive surface to the circumference of the photosensitive surface is small, that is, the ratio of the area of the photosensitive surface to the circumference of the photosensitive surface is less than the ratio threshold. Therefore, it is possible that a photosensitive surface having a large absolute area does not meet the anti-jitter requirement. There may also be a case that the photosensitive surface has a small area and a large circumference, so that the ratio of the area of the photosensitive surface to the circumference of the photosensitive surface is less than the ratio threshold. Therefore, the photosensitive surface having a very small area may not meet the anti-jitter requirement.

According to embodiments of the present disclosure, the photosensitive surface is in contact or non-contact with a surface of the measurement region.

According to embodiments of the present disclosure, a form of measuring the tissue element may include a contact measurement and a non-contact measurement. The contact measurement may be implemented to prevent interference light from being received by the photosensitive surface, thereby further improving the reliability of the measurement result. The non-contact measurement may be implemented to avoid an influence of an interference factor such as temperature and pressure on the measurement result, thereby further improving the reliability of the measurement result.

If the photosensitive surface is arranged in contact with the surface of the measurement region, it may be considered that the form of measuring the tissue element is the contact measurement. If the photosensitive surface is arranged in non-contact with the surface of the measurement region, it may be considered that the form of measuring the tissue element is the non-contact measurement.

According to embodiments of the present disclosure, a distance between the photosensitive surface and the surface of the measurement region is less than or equal to a first distance threshold, and the efficiency of the photosensitive surface receiving the exit light is greater than or equal to an efficiency threshold.

According to embodiments of the present disclosure, as the photosensitive surface is made of a photosensitive material and has an area with continuity, a wide range of light intensity values may be received, and the efficiency of receiving the exit light may be improved. Based on this, the efficiency of receiving the exit light may be greater than or equal to the efficiency threshold even when the photosensitive surface is close to the surface of the measurement region, that is, when the distance between the photosensitive surface and the surface of the measurement region is less than or equal to the first distance threshold.

According to embodiments of the present disclosure, the method may further include the following operations before irradiating the measurement region by the incident light having the single predetermined wavelength.

A positioning feature is determined. A measurement region is determined according to the positioning feature, where the measurement region is a region that meets a reproducibility of the controllable measurement condition. The measurement probe is arranged at a position corresponding to the measurement region, where the measurement probe includes M photosensitive surfaces.

According to embodiments of the present disclosure, during the living tissue element measurement, a weak signal of the detected tissue element may be submerged by a change in the measurement condition, and it is difficult to obtain a real signal of the detected tissue element, which causes a great influence on the measurement result.

The inventors found that the changes in the controllable measurement conditions affect the measurement results through different mechanisms, and it is difficult to suppress the influence on the measurement result by using a mathematical algorithm. However, it was found that the reproducibility of the controllable measurement condition may be achieved through an effective control, so that the influence of the controllable measurement condition on the measurement result may be reduced to a negligible level, that is, the influence of the change in the controllable measurement condition on the measurement result is at a similar level to an influence of a random noise on the measurement result. Therefore, for the controllable measurement condition, the inventors propose that the most appropriate processing method is to control the controllable measurement condition to achieve the reproducibility of the controllable measurement condition by using an effective control method. The effective control method is not a mathematical algorithm, and it may be implemented with a hardware design. The reproducibility of the controllable measurement condition may refer to that during each tissue element measurement, the controllable measurement condition is maintained within a predetermined change range, so that the controllable measurement condition remains unchanged or substantially unchanged.

According to embodiments of the present disclosure, by controlling the controllable measurement condition using the effective control method, the influence of the change in the controllable measurement condition on the measurement result may be reduced to a negligible level, and it is avoided to use a complex mathematical algorithm for processing, thereby improving the reliability of the measurement result. In addition, a difficulty in data processing and an amount of data processing may also be reduced.

A description will be given below for achieving the reproducibility of the controllable measurement condition using the effective control method so as to reduce the influence of the change in the controllable measurement condition on the measurement result to a negligible level. Embodiments of the present disclosure mainly focus on a reproducibility of a measurement posture and a reproducibility of a measurement region. The measurement posture refers to a posture of a limb supporting the measurement site. In the related art, no relevant content was found for the measurement posture.

In an aspect, for the reproducibility of the measurement region, a positioning deviation of the measurement region is caused by a non-uniformity of a tissue distribution and a difference in a flatness of a skin surface. When a relative position between the measurement probe and the measurement region deviates, the transmission path of light in the tissue may be changed. Therefore, in order to achieve the reproducibility of the controllable measurement condition, the reproducibility of the measurement region needs to be ensured as much as possible.

In another aspect, for the reproducibility of the measurement posture, it is difficult for the detected object to keep a same measurement posture during the tissue element measurement, and a change in the measurement posture may cause a change in the skin state of the measurement region, which may lead to a change in the transmission path of light in the tissue. Therefore, the change in the measurement posture may cause a positioning deviation and then affect the reliability of the measurement result. The skin state may include a surface shape of the skin and an internal structure of the skin. Therefore, it is necessary to achieve the reproducibility of the measurement posture. A purpose of positioning the measurement posture is to ensure that the measurement posture is a target measurement posture when a tissue element measurement is performed, that is, when the tissue element measurement is performed, if a current measurement posture is not the target measurement posture, it is needed to adjust the current measurement posture to the target measurement posture. The target measurement posture is a measurement posture that meets the reproducibility of the controllable measurement condition.

An importance of achieving the reproducibility of the measurement posture is often overlooked, which is reflected in the following two aspects.

In an aspect, it is not found that the reproducibility of the measurement posture is an important factor affecting the reliability of the measurement result. In the related art, it is generally considered that in terms of the controllable measurement condition, a most important factor affecting the reproducibility of the controllable measurement condition is the reproducibility of the measurement region, that is, it is considered that the improvement of the reliability of the measurement result may be achieved in terms of the controllable measurement condition if the reproducibility of the measurement region is achieved, and that it is not needed to consider other factors. In other words, in the related art, a direction of the improvement revolves around how to improve a positioning accuracy of the measurement region, and it has not been found that in terms of the controllable measurement condition, the reproducibility of the measurement posture is also an important factor affecting the reliability of the measurement result.

Moreover, according to the above analysis, even if the reproducibility of the measurement region is achieved, when the posture of the limb supporting the measurement region changes, the internal structure of the skin at the measurement region may also change, which results in a change in the transmission path of light in the tissue, thereby affecting the reliability of the measurement result. In other words, only ensuring the reproducibility of the measurement region while ignoring the reproducibility of the measurement posture is not conducive to improving the reliability of the measurement result.

In another aspect, no effective method is adopted to achieve the reproducibility of the measurement posture. Due to the lack of deep research on the factors affecting the reliability of the measurement result, the importance of achieving the reproducibility of the measurement posture has not been recognized. In the tissue element measurement, it is considered that the method of keeping a body stability by the detected object is sufficient to achieve a control of the measurement posture, that is, the measurement posture is well controlled if the detected object believes that a body state is not changed. However, in most cases, the change in the measurement posture may not be perceived by the detected object, and such method of achieving the reproducibility of the measurement posture may result in a significant error, which may greatly interfere with the reliability of the measurement result. Even if a method is adopted to control the measurement posture, such method substantially may not ensure the reproducibility of the measurement posture since the importance of achieving the reproducibility of the measurement posture is not recognized.

Therefore, in order to achieve the reproducibility of the measurement region, it is needed to ensure the reproducibility of the measurement posture as much as possible, that is, to accurately position the measurement posture. Based on the above, the reproducibility of the measurement region needs to be based on the reproducibility of the measurement posture. Therefore, the positioning of the measurement region needs to be based on the positioning of the measurement posture.

During a positioning process, the positioning may be performed according to a positioning feature. The positioning feature may include a posture positioning feature and a region positioning feature. The posture positioning feature is used for the positioning of the measurement posture, and the region positioning feature is used for the positioning of the measurement region. The posture positioning feature may be arranged on the detected object or a non-detected object, and the region positioning feature may be arranged on the detected object or the non-detected object. The non-detected object may include the measurement probe or other devices. The positioning feature may include a manually provided positioning feature or an inherent feature of the detected object. The inherent feature may include palm print, fingerprint, birthmark, mole or nevus, etc.

According to embodiments of the present disclosure, if a method of manually providing a positioning feature is adopted, the manually provided positioning feature may gradually fade over time, and it is required to provide the positioning feature again, which may introduce a new error and affect the positioning accuracy. The inherent feature has a good stability and is not prone to an error.

The inherent feature of the detected object may be used as the positioning feature in order to reduce the positioning complexity and improve the positioning accuracy. However, even if the inherent feature of the detected object is used as the posture positioning feature, the internal structure of the skin may be affected by the change in the measurement posture, which may also result in a positioning deviation of the measurement region. Therefore, the position where the positioning feature is arranged on the detected object may not be determined arbitrarily, but according to the measurement site and a bone-muscle relationship between the measurement site and a peripheral site. In an example, if the measurement site is a forearm extensor side, the peripheral site includes a wrist. For the forearm extensor side, a change in a state of the wrist may greatly affect the skin state of the forearm extensor side. In order to improve the positioning accuracy, the positioning features may be arranged on the forearm extensor side and a back of a hand, respectively. It should be noted that the positioning feature may be manually provided if no inherent feature may be used as the positioning feature. For example, the positioning feature may be a point marker or a graphic marker, and the graphic marker may include a cross marker.

According to embodiments of the present disclosure, the positioning feature includes a first posture positioning feature and a region positioning feature. Determining the measurement region according to the positioning feature may include the following operations.

The current measurement posture of the detected object is adjusted to a target measurement posture according to the first posture positioning feature, where the target measurement posture is a measurement posture meeting the reproducibility of the controllable measurement condition. When the current measurement posture is the target measurement posture, the measurement region is determined according to the region positioning feature.

According to embodiments of the present disclosure, when positioning the measurement posture and the measurement region, the positioning of the measurement posture is the basis of the positioning of the measurement region. In a subsequent measurement process after completing the positioning of the measurement region, it is generally not required to reposition the measurement region, but there may be a case that it is still required to position the measurement posture. A condition for completing the positioning of the measurement posture is that the current measurement posture is the target measurement posture. The target measurement posture is a measurement posture that meets the reproducibility of the controllable measurement condition.

According to embodiments of the present disclosure, a cause for the case that it is still required to position the measurement posture lies in that a strategy of allowing the measured site to move within a certain range when no measurement is performed and positioning the measurement posture when a measurement is performed is adopted in embodiments of the present disclosure in order to bring a better user experience to the detected object. When performing a measurement, it is required to ensure that the current measurement posture is the target measurement posture. If the current measurement posture is not the target measurement posture, it is needed to adjust the measurement posture to ensure that the current measurement posture is the target measurement posture.

Based on the above, the positioning may include a first positioning of the measurement posture, a positioning of the measurement region, and a second positioning of the measurement posture. The first positioning of the measurement posture may be understood as a positioning of the measurement posture in cooperation with the implementation of the measurement region. The second positioning of the measurement posture may be understood as a positioning of the measurement posture that is performed in a case that the measurement posture is not the target measurement posture after the measurement probe is arranged at the position corresponding to the measurement region.

According to embodiments of the present disclosure, the region positioning feature is used for the positioning of the measurement region. The posture positioning feature used for the first positioning of the measurement posture is referred to as a first posture positioning feature. The posture positioning feature used for the second positioning of the measurement posture is referred to as a second posture positioning feature. Both the first posture positioning feature and the second posture positioning feature are used for the positioning of the measurement posture. The region positioning feature, the first posture positioning feature and the second posture positioning feature may be all the same, partially the same, or all different from each other. There may be one or more region positioning features, first posture positioning features and second posture positioning features.

When performing the first positioning of the measurement posture and the positioning of the measurement region, the current measurement posture of the detected object may be adjusted according to the first posture positioning feature so that the first posture positioning feature is matched with a predetermined feature. When the first posture positioning feature is matched with the predetermined feature, it may be determined that the current measurement posture is the target measurement posture. When the current measurement posture is the target measurement posture, the measurement region is determined according to the region positioning feature. Then, the positioning of the measurement posture and the positioning of the measurement region are completed.

It should be noted that determining the measurement region according to the region positioning feature may be understood as determining a region corresponding to the region positioning feature as the measurement region, which includes determining a region where the region positioning feature is located as the measurement region, or determining another region having an association with the region positioning feature as the measurement region.

By using the first posture positioning feature and the region positioning feature, the positioning of the measurement region and the positioning of the measurement posture may be achieved synchronously.

According to embodiments of the present disclosure, arranging the measurement probe at the position corresponding to the measurement region may include the following operations.

The measurement probe is arranged at the position corresponding to the measurement region by a fixing portion. The fixing portion is integrated with, partially separated from or completely separated from the measurement probe.

According to embodiments of the present disclosure, the fixing portion is used to fix the measurement probe. The fixing portion may be integrated with, partially separated from or completely separated from the measurement probe, that is, the fixing portion may be a component of the measurement probe, or may be independent of the measurement probe, or partially be a component of the measurement probe and partially be independent of the measurement probe. The fixing portion may include a fixing seat and a first fitting part, or the fixing portion may include a second fitting part. The first fitting part is used to arrange the fixing seat at the position corresponding to the measurement region, and the fixing seat is used to arrange the measurement probe. The second fitting part is used to arrange the measurement probe at the position corresponding to the measurement region.

If the fixing portion includes the fixing seat and the first fitting part, the fixing seat is separated from the measurement probe, and the first fitting part is integrated with or separated from the fixing seat. If the fixing portion includes the second fitting part, the second fitting part is integrated with or separated from the measurement probe.

According to embodiments of the present disclosure, the fixing portion includes the fixing seat and the first fitting part.

Arranging the measurement probe at the position corresponding to the measurement region by the fixing portion may include the following operations.

The fixing seat is arranged at the position corresponding to the measurement region by the first fitting part. The measurement probe is arranged on the fixing seat.

According to embodiments of the present disclosure, the measurement probe is arranged at the position corresponding to the measurement region by the fixing seat, rather than directly arranged at the position corresponding to the measurement region.

During the process of measuring the tissue element, if the measurement probe is arranged at the position corresponding to the measurement region by the fixing seat, the fixing seat may be arranged on the measurement region for a long time without leaving the measurement region, and the measurement probe may be arranged on the fixing seat when a measurement is performed and may be detached from the fixing seat when no measurement is performed. Moreover, since the fixing seat is arranged at the position corresponding to the measurement region, it is possible to maintain a good positioning accuracy and reduce the difficulty of positioning the measurement probe when the measurement probe is detached from the fixing seat and then arranged on the fixing seat.

According to embodiments of the present disclosure, the skin state of the skin at the measurement region meets a first predetermined condition during a process of arranging the fixing seat at the position corresponding to the measurement region by the first fitting part.

According to embodiments of the present disclosure, the skin state of the skin at the measurement region meets a second predetermined condition during a process of arranging the measurement probe on the fixing seat.

According to embodiments of the present disclosure, an action of fixing the fixing seat may affect the skin state of the skin at the corresponding position, and then affect the positioning accuracy of the measurement region. In order to ensure the positioning accuracy of the measurement region, it is possible to ensure that the skin state of the skin at the measurement region meets the first predetermined condition in the process of fixing the fixing seat by the first fitting part. The first predetermined condition may refer to that a change in the skin state of the skin at the corresponding position is within a first predetermined range in the process of fixing the fixing seat by the first fitting part. The change in the skin state may include a skin deformation. Accordingly, the first predetermined range may include a first predetermined deformation range.

According to embodiments of the present disclosure, an action of fixing the measurement probe may affect the skin state of the skin at the corresponding position, and then affect the positioning accuracy of the measurement region. In order to ensure the positioning accuracy of the measurement region, it is possible to ensure that the skin state of the skin at the measurement region meets the second predetermined condition in the process of fixing the measurement probe by the fixing seat. The second predetermined condition may refer to that a change in the skin state of the skin at the corresponding position is within a second predetermined range in the process of fixing the measurement probe by the fixing seat. The change in the skin state may include a skin deformation. Accordingly, the second predetermined range may include a second predetermined deformation range.

According to embodiments of the present disclosure, the measurement probe is not movable on the fixing seat.

According to embodiments of the present disclosure, when the measurement probe is fixed on the fixing seat, there may be a problem of an unstable fixation that may affect the reproducibility of the controllable measurement condition. In order to solve this problem, it is possible to ensure that the measurement probe does not move on the fixing seat during the process of measuring the tissue element.

According to embodiments of the present disclosure, the fixing portion includes the second fitting part.

Arranging the measurement probe at the position corresponding to the measurement region by the fixing portion may include the following operations.

The measurement probe is arranged at the position corresponding to the measurement region by the second fitting part.

According to embodiments of the present disclosure, for the method of arranging the measurement probe at the position corresponding to the measurement region, in addition to the above-mentioned method of arranging the measurement probe at the position corresponding to the measurement region by the fixing seat, it is also possible to adopt a method of directly arranging the measurement probe at the position corresponding to the measurement region, in which the fixing seat is not required, but a cooperation of the second fitting part is required.

It should be noted that the above-mentioned not requiring the fixing seat may include the following two cases. In a first case, the measurement probe is provided with a structure integrated with the measurement probe, which plays a same role as an independent fixing seat. In a second case, the measurement probe is not provided with a structure that plays the same role as an independent fixing seat.

According to embodiments of the present disclosure, the skin state of the skin at the measurement region meets a third predetermined condition during a process of arranging the measurement probe at the position corresponding to the measurement region by the second fitting part.

According to embodiments of the present disclosure, an action of fixing the measurement probe may affect the skin state of the skin at the corresponding position, and then affect the positioning accuracy of the measurement region. In order to ensure the positioning accuracy of the measurement region, it is possible to ensure that the skin state of the skin at the measurement region meets the third predetermined condition in the process of fixing the measurement probe by the second fitting part. The third predetermined condition may refer to that a change in the skin state of the skin at the corresponding position is within a third predetermined range in the process of fixing the measurement probe by the second fitting part. The change in the skin state may include a skin deformation. Accordingly, the third predetermined range may include a third predetermined deformation range.

According to embodiments of the present disclosure, determining the measurement region according to the region positioning feature may include the following operations.

A first projection feature is obtained. When it is determined that the region positioning feature is not matched with the first projection feature, a position of the measurement probe and/or the fixing portion is adjusted until the region positioning feature is matched with the first projection feature. When it is determined that the region positioning feature is matched with the first projection feature, a region corresponding to the measurement probe and/or the fixing portion is determined as the measurement region.

According to embodiments of the present disclosure, in order to ensure a flexibility of use and the positioning accuracy of the measurement region, it is possible to adopt an optical method, that is, to match the region positioning feature with the first projection feature, and determine the measurement region according to a matching result. The first projection feature is formed according to the optical method, that is, by projecting a light spot with a predetermined shape using a light source. The shape of the light spot may be determined according to the region positioning feature. For example, the light spot with the predetermined shape is a cross light spot.

According to embodiments of the present disclosure, after the first projection feature is obtained by using a structure for projecting the first projection feature, it is determined whether the region positioning feature is matched with the first projection feature. When it is determined that the region positioning feature is not matched with the first projection feature, the position of the measurement probe and/or the fixing seat may be adjusted so that the region positioning feature is matched with the first projection feature, until the region positioning feature is matched with the first projection feature. When it is determined that the region positioning feature is matched with the first projection feature, it may indicate that the region where the measurement probe and/or the fixing seat are/is currently located is the measurement region.

According to embodiments of the present disclosure, the structure for projecting the first projection feature may be arranged on the detected object, the measurement probe, the fixing seat or other objects. Other objects may refer to objects other than the measurement probe, the fixing portion and the detected object. The region positioning feature may be arranged on at least one of the measurement probe, the fixing seat, the detected object, or other objects. An adjustment process implemented based on the optical method will be described below from two aspects, including an arrangement position of the structure for projecting the first projection feature and an arrangement position of the region positioning feature.

A description is given below from the aspect of the arrangement position of the structure for projecting the first projection feature.

In an aspect, if the structure for projecting the first projection feature is arranged on the detected object, the region positioning feature may be arranged on at least one of the detected object, the measurement probe, the fixing seat, or other objects. It should be noted that if the region positioning feature is arranged on the detected object or other objects, the positioning of the measurement region may be achieved by a method of adjusting the position of the measurement probe and/or the fixing seat according to the region positioning feature and the first projection feature until the region positioning feature is matched with the first projection feature. The region positioning feature being matched with the first projection feature here means that the region positioning feature is blocked by the measurement probe and/or the fixing seat, so that the first projection feature may not be projected to the position where the region positioning feature is located. If the region positioning feature is not matched with the first posture positioning feature, at least one first projection feature may be projected to the position where the region positioning feature is located.

In another aspect, if the structure for projecting the first projection feature is arranged on the measurement probe, the region positioning feature may not be arranged on the measurement probe, but may be arranged on the detected object, the fixing seat or other objects. It should be noted that if the region positioning feature is arranged on the fixing seat, and the positioning of the measurement probe is achieved by arranging the measurement probe at the position corresponding to the measurement region by the fixing portion provided with the fixing seat, the positioning of the measurement region may be achieved by the method of adjusting the position of the fixing seat. The position of the measurement probe is fixed before the region positioning feature is matched with the first projection feature. The position of the fixing seat is adjusted according to the region positioning feature and the first projection feature until the region positioning feature is matched with the first projection feature. When the two are matched, the region corresponding to the fixing seat is determined as the measurement region, and then the measurement probe may be arranged on the fixing seat.

In another aspect, if the structure for projecting the first projection feature is arranged on the fixing seat, the region positioning feature may not be arranged on the fixing seat, but may be arranged on the detected object, the measurement probe or other objects. It should be noted that if the region positioning feature is arranged on the measurement probe, and the positioning of the measurement probe is achieved by arranging the measurement probe at the position corresponding to the measurement region by the fixing portion provided with the fixing seat, then the positioning of the measurement region may be achieved by the method of adjusting the position of the fixing seat. The position of the measurement probe is fixed before the region positioning feature is matched with the first projection feature. The position of the fixing seat is adjusted according to the region positioning feature and the first projection feature until the region positioning feature is matched with the first projection feature. When the two are matched, the region corresponding to the fixing seat is determined as the measurement region, and then the measurement probe may be arranged on the fixing seat.

In another aspect, if the structure for projecting the first projection feature is arranged on other objects, the region positioning feature may be arranged on at least one of the detected object, the measurement probe, the fixing seat, or other objects. It should be noted that if the region positioning feature is arranged on the detected object or other objects, the positioning of the measurement region may be achieved using a method similar to that of arranging the structure for projecting the first projection feature on the detected object and arranging the region positioning feature on the detected object or other objects, which will not be repeated here.

A description will be given below from the aspect of the arrangement position of the region positioning feature.

In an aspect, if the region positioning feature is arranged on the detected object, the structure for projecting the first projection feature may be arranged on the detected object, the measurement probe, the fixing seat or other objects. It should be noted that if the structure for projecting the first projection feature is arranged on the detected object or other objects, the positioning of the measurement region may be achieved by the method of adjusting the position of the measurement probe and /or the fixing seat according to the region positioning feature and the first projection feature until the region positioning feature is matched with the first projection feature. The region positioning feature being matched with the first projection feature here means that the region positioning feature is blocked by the measurement probe and/or the fixing seat, so that the first projection feature may not be projected to the position where the region positioning feature is located. If the region positioning feature is not matched with the first posture positioning feature, at least one first projection feature may be projected to the position where the region positioning feature is located.

In another aspect, if the region positioning feature is arranged on the measurement probe, the structure for projecting the first projection feature is separated from the measurement probe, and may be arranged on the detected object, the fixing seat or other objects. It should be noted that if the structure for projecting the first projection feature is arranged on the fixing seat, reference may be made to the above description of the corresponding part, and details will not be repeated here.

In another aspect, if the region positioning feature is arranged on the fixing seat, the structure for projecting the first projection feature is separated from the fixing seat, and may be arranged on the detected object, the measurement probe or other objects. It should be noted that if the structure for projecting the first projection feature is arranged on the measurement probe, reference may be made to the above description of the corresponding part, and details will not be repeated here.

In another aspect, if the region positioning feature is arranged on other objects, the structure for projecting the first projection feature may be arranged on the detected object, the measurement probe, the fixing seat or other objects. It should be noted that if the structure for projecting the first projection feature is arranged on the detected object or other objects, reference may be made to the above description of the corresponding part, and details will not be repeated here.

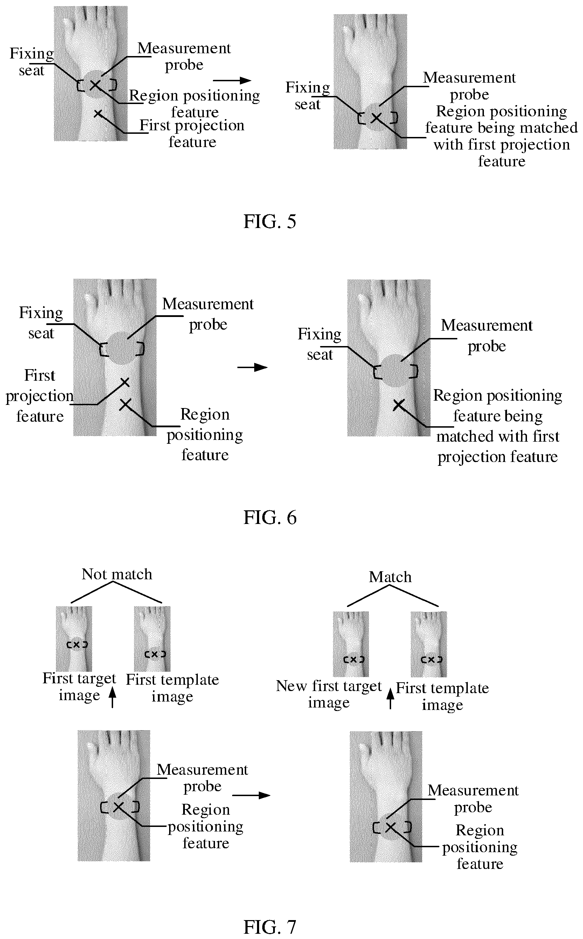

In an example, FIG. 5 schematically shows a schematic diagram of positioning the measurement region based on an optical method according to embodiments of the present disclosure. As shown in FIG. 5, the region positioning feature is arranged on the measurement probe. FIG. 6 schematically shows another schematic diagram of positioning the measurement region based on an optical method according to embodiments of the present disclosure. As shown in FIG. 6, the region positioning feature is arranged on the detected object.

When positioning the measurement region using an optical method, in an aspect, a position and an angle of the light source may be adjusted flexibly and may be easily matched with the region positioning feature. Therefore, the region positioning feature may be arranged flexibly, so that the difficulty in the arrangement of the region positioning feature is reduced. In another aspect, the shape of the emitted light spot may be adjusted to be matched with the region positioning feature better, so that the positioning accuracy may be improved.

According to embodiments of the present disclosure, determining the measurement region according to the region positioning feature may include the following operations.

A first target image is obtained. A first template image is obtained, where the first template image includes the region positioning feature. When it is determined that the first target image is not matched with the first template image, the position of the measurement probe and/or the fixing portion is adjusted to obtain a new first target image until the new first target image is matched with the first template image. When it is determined that the first target image is matched with the first template image, the region corresponding to the measurement probe and/or the fixing portion is determined as the measurement region.

According to embodiments of the present disclosure, in order to ensure the flexibility of use and the positioning accuracy of the measurement region, it is possible to adopt an image matching method, that is, to match the first target image with the first template image, and determine the measurement region according to a matching result. The first template image may include the region positioning feature, and a position of the region positioning feature in the first template image is a predetermined position. In the process of matching the first target image with the first template image, the first target image may be a target image that does not include the region positioning feature, or may be a target image that includes the region positioning feature but the position of the region positioning feature in the first target image is not the predetermined position, or may be a target image that includes the region positioning feature and the position of the region positioning feature in the first target image is the predetermined position. As the first template image includes the region positioning feature at the predetermined position, if the first target image is matched with the first template image, then it may indicate that the first target image includes the region positioning feature and the position of the region positioning feature in the first target image is the predetermined position. In other words, a purpose of matching the first target image with the first template image is to obtain such first target image that the region positioning feature is included in the first target image and the position of the region positioning feature in the first target image is the predetermined position.

According to embodiments of the present disclosure, when it is determined that the first target image is matched with the first template image, it may indicate that the region where the measurement probe and/or the fixing seat are/is currently located is the measurement region. Determining whether the first target image is matched with the first template image may include determining a similarity between the first target image and the first template image. If the similarity is greater than or equal to a similarity threshold, it is determined that the first target image is matched with the first template image. If the similarity is less than the similarity threshold, it is determined that the first target image is not matched with the first template image. Determining the similarity between the first target image and the first template image may include performing a correlation analysis on the first target image and the first template image to obtain a correlation coefficient, and determining the similarity between the first target image and the first template image according to the correlation coefficient.

According to embodiments of the present disclosure, a structure for acquiring the first target image may be arranged on the detected object, the measurement probe, the fixing seat or other objects. Other objects may refer to objects other than the measurement probe, the fixing portion and the detected object. The region positioning feature may be arranged on at least one of the measurement probe, the fixing seat, the detected object, or other objects. For the description of the region positioning feature and the structure for acquiring the first target image, reference may be made to the description of the region positioning feature and the structure for projecting the first projection feature, and details will not be repeated here. A difference is that the region positioning feature may be arranged on at least one of the detected object, the measurement probe, the fixing seat, or other objects if the structure for acquiring the first target image is arranged on the measurement probe; the region positioning feature may be arranged on at least one of the detected object, the measurement probe, the fixing seat, or other objects if the structure for acquiring the first target image is arranged on the fixing seat.

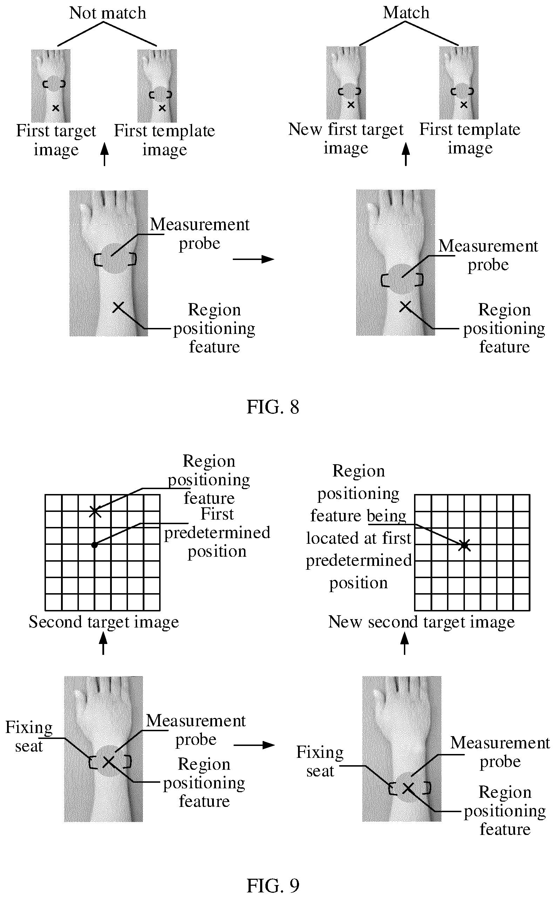

In an example, FIG. 7 schematically shows a schematic diagram of positioning the measurement region based on an image matching method according to embodiments of the present disclosure. As shown in FIG. 7, the region positioning feature is arranged on the measurement probe. FIG. 8 schematically shows another schematic diagram of positioning the measurement region based on an image matching method according to embodiments of the present disclosure. As shown in FIG. 8, the region positioning feature is arranged on the detected object.

According to embodiments of the present disclosure, determining the measurement region according to the region positioning feature may include the following operations.

A second target image is obtained, where the second target image includes the region positioning feature. When it is determined that the position of the region positioning feature in the second target image is not the first predetermined position, the position of the measurement probe and/or the fixing portion is adjusted to obtain a new second target image until the position of the region positioning feature in the new second target image is the first predetermined position. When it is determined that the position of the region positioning feature in the new second target image is the first predetermined position, the region corresponding to the measurement probe and/or the fixing portion is determined as the measurement region.

According to embodiments of the present disclosure, in order to ensure the flexibility of use and the positioning accuracy of the measurement region, it is possible to adopt an imaging method, that is, if the position of the region positioning feature in the second target image is the first predetermined position, it may indicate that the positioning of the measurement region is completed.

According to embodiments of the present disclosure, the process of positioning the measurement region using the imaging method is a process of determining whether the position of the region positioning feature in the second target image is the first predetermined position. If the position of the region positioning feature in the second target image is not the first predetermined position, the position of the measurement probe and/or the fixing seat may be adjusted to obtain a new second target image until the position of the region positioning feature in the new second target image is the first predetermined position. When the position of the region positioning feature in the new second target image is the first predetermined position, it may indicate that the region where the measurement probe and/or the fixing seat are/is currently located is the measurement region.

According to embodiments of the present disclosure, a structure for acquiring the second target image may be arranged on the detected object, the measurement probe, the fixing seat or other objects. Other objects may refer to objects other than the measurement probe, the fixing portion and the detected object. The region positioning feature may be arranged on at least one of the measurement probe, the fixing seat, the detected object, or other objects. For the description of the region positioning feature and the structure for acquiring the second target image, reference may be made to the description of the region positioning feature and the structure for projecting the first projection feature, and details will not be repeated here.

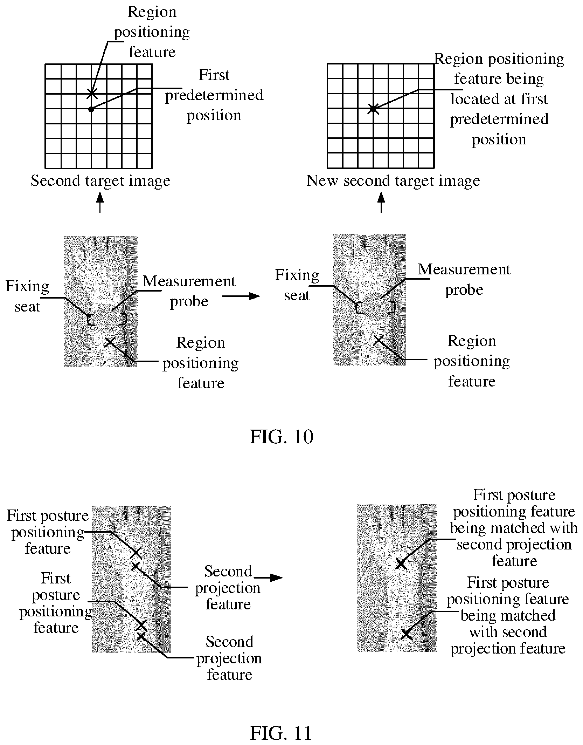

In an example, FIG. 9 schematically shows a schematic diagram of positioning the measurement region based on an imaging method according to embodiments of the present disclosure. As shown in FIG. 9, the region positioning feature is arranged on the measurement probe. FIG. 10 schematically shows another schematic diagram of positioning the measurement region based on an imaging method according to embodiments of the present disclosure. As shown in FIG. 10, the region positioning feature is arranged on the detected object, a movement of the measurement probe and the fixing seat causes a change in a relative position between the two and the region positioning feature, so that the position of the region positioning feature presented in the image is the first predetermined position.

According to embodiments of the present disclosure, adjusting the current measurement posture of the detected object to the target measurement posture according to the first posture positioning feature may include the following operations.

A second projection feature is obtained. When it is determined that the first posture positioning feature is not matched with the second projection feature, the current measurement posture is adjusted until the first posture positioning feature is matched with the second projection feature. When it is determined that the first posture positioning feature is matched with the second projection feature, it is determined that the current measurement posture is the target measurement posture.

According to embodiments of the present disclosure, in order to ensure the flexibility of use and the positioning accuracy of the measurement posture, it is possible to adopt an optical method, that is, to match the first posture positioning feature with the second projection feature, and determine the target measurement posture according to a matching result. The second projection feature is formed according to the optical method, that is, by projecting a light spot with a predetermined shape using a light source. The shape of the light spot may be determined according to the first posture positioning feature. That is, for the detected object, the second projection feature matched with the first posture positioning feature is arranged according to the first posture positioning feature. The current measurement posture with which the first posture positioning feature is matched with the second projection feature is the target measurement posture.

According to embodiments of the present disclosure, the structure for projecting the second projection feature may be arranged on the detected object, the measurement probe, the fixing seat or other objects. Other objects may refer to objects other than the measurement probe, the fixing portion and the detected object. The first posture positioning feature may be arranged on at least one of the measurement probe, the fixing seat, the detected object, or other objects. An adjustment process implemented based on the optical method will be described below from two aspects, including an arrangement position of the structure for projecting the second projection feature and an arrangement position of the first posture positioning feature.

A description will be given below from the aspect of the arrangement position of the structure for projecting the second projection feature.

In an aspect, if the structure for projecting the second projection feature is arranged on the detected object, the first posture positioning feature may be arranged on at least one of the detected object, the measurement probe, the fixing seat, or other objects. It should be noted that if the first posture positioning feature is arranged on the measurement probe, in order to achieve the positioning of the measurement posture, it is required that the position of the measurement probe is fixed in the stage of the first positioning of the measurement posture. Similarly, if the first posture positioning feature is arranged on the fixing seat, in order to achieve the positioning of the measurement posture, it is required that the position of the fixing seat is fixed in the stage of the first positioning of the measurement posture.

In another aspect, if the structure for projecting the second projection feature is arranged on the measurement probe, the first posture positioning feature may not be arranged on the measurement probe, but may be arranged on the detected object, the fixing seat or other objects. It should be noted that the position of the measurement probe needs to be fixed in the stage of the first positioning of the measurement posture. In addition, if the first posture positioning feature is arranged on the fixing seat, the first positioning of the measurement posture may be achieved by the method of adjusting the current measurement posture of the detected object according to the first posture positioning feature and the second projection feature until the first posture positioning feature is matched with the second projection feature. The first posture positioning feature being matched with the second projection feature here means that the first posture positioning feature is blocked by the detected object, so that the second projection feature may not be projected to the position where the first posture positioning feature is located. If the first posture positioning feature is not matched with the second posture positioning feature, at least one second projection feature may be projected to the position where the first posture positioning feature is located. If the first posture positioning feature is arranged on other objects, the positioning of the measurement posture may be achieved by a method similar to that of arranging the first posture positioning feature on the fixing seat, and details will not be repeated here.

In another aspect, if the structure for projecting the second projection feature is arranged on the fixing seat, the first posture positioning feature may not be arranged on the fixing seat, but may be arranged on the detected object, the measurement probe or other objects. It should be noted that the position of the fixing seat may be fixed in the stage of the first positioning of the measurement posture. In addition, if the first posture positioning feature is arranged on the measurement probe or other objects, the positioning of the measurement posture may be achieved by a method similar to that of arranging the structure for projecting the second projection feature on the measurement probe and arranging the first posture positioning feature on the fixing seat or other objects, and details will not be repeated here.

In another aspect, if the structure for projecting the second projection feature is arranged on other objects, the first posture positioning feature may be arranged on at least one of the detected object, the measurement probe, the fixing seat, or other objects. It should be noted that if the first posture positioning feature is arranged on the measurement probe, the fixing seat or other objects, the positioning of the measurement posture may be achieved by a method similar to that of arranging the structure for projecting the second projection feature on the measurement probe and arranging the first posture positioning feature on the fixing seat or other objects, and details will not be repeated here..

A description will be given below from the aspect of the arrangement position of the first posture positioning feature.

In an aspect, if the first posture positioning feature is arranged on the detected object, the structure for projecting the second projection feature may be arranged on the detected object, the measurement probe, the fixing seat or other objects. It should be noted that if the structure for projecting the second projection feature is arranged on the measurement probe, the position of the measurement probe needs to be fixed in the stage of the first positioning of the measurement posture. Similarly, if the structure for projecting the second projection feature is arranged on the fixing seat, the position of the fixing seat needs to be fixed in the stage of the first positioning of the measurement posture.

In another aspect, if the first posture positioning feature is arranged on the measurement probe, the structure for projecting the second projection feature is separated from the measurement probe, and may be arranged on the detected object, the fixing seat or other objects. It should be noted that if the structure for projecting the second projection feature is arranged on the detected object, the fixing seat or other objects, reference may be made to the above description of the corresponding part, and details will not be repeated here.

In another aspect, if the first posture positioning feature is arranged on the fixing seat, the structure for projecting the second projection feature is separated from the fixing seat, and may be arranged on the detected object, the measurement probe or other objects. It should be noted that if the structure for projecting the second projection feature is arranged on the detected object, the measurement probe or other objects, reference may be made to the above description of the corresponding part, and details will not be repeated here.

In another aspect, if the first posture positioning feature is arranged on other objects, the structure for projecting the second projection feature may be arranged on the detected object, the measurement probe, the fixing seat or other objects. It should be noted that if the structure for projecting the second projection feature is arranged on the detected object, the measurement probe, the fixing seat or other objects, reference may be made to the above description of the corresponding part, and details will not be repeated here.

In an example, FIG. 11 schematically shows a schematic diagram of positioning the measurement posture based on an optical method according to embodiments of the present disclosure. As shown in FIG. 11, the first posture positioning feature is arranged on the detected object.

When positioning the measurement posture using an optical method, in an aspect, a position and an angle of the light source may be adjusted flexibly and may be easily matched with the first posture positioning feature. Therefore, the first posture positioning feature may be arranged flexibly, so that the difficulty in the arrangement of the first posture positioning feature is reduced. In another aspect, the shape of the emitted light spot may be adjusted to be matched with the first posture positioning feature better, so that the positioning accuracy may be improved.

According to embodiments of the present disclosure, adjusting the current measurement posture of the detected object to the target measurement posture according to the first posture positioning feature may include the following operations.

A third target image is obtained. A second template image is obtained, where the second template image includes the first posture positioning feature. When it is determined that the third target image is not matched with the second template image, the current measurement posture is adjusted to obtain a new third target image until the new third target image is matched with the second template image. When it is determined that the new third target image is matched with the second template image, it is determined that the current measurement posture is the target measurement posture.

According to embodiments of the present disclosure, in order to ensure the flexibility of use and the positioning accuracy of the measurement posture, it is possible to adopt an image matching method, that is, to match the third target image with the second template image, and determine the target measurement posture according to a matching result. The second template image may include the first posture positioning feature, and the position of the first posture positioning feature in the second template image is a predetermined position. In the process of matching the third target image with the second template image, the third target image may be a target image that does not include the first posture positioning feature, or may be a target image that includes the first posture positioning feature but the position of the first posture positioning feature in the third target image is not the predetermined position, or may be a target image that includes the first posture positioning feature and the position of the first posture positioning feature in the third target image is the predetermined position. As the second template image includes the first posture positioning feature at the predetermined position, if the third target image is matched with the second template image, it may indicate that the third target image includes the first posture positioning feature and the position of the first posture positioning feature in the third target image is the predetermined position. In other words, a purpose of matching the third target image with the second template image is to obtain such third target image that the first posture positioning feature is included in the third target image and the position of the first posture positioning feature in the third target image is the predetermined position.

According to embodiments of the present disclosure, the structure for acquiring the third target image may be arranged on the detected object, the measurement probe, the fixing seat or other objects. Other objects may refer to objects other than the measurement probe, the fixing portion and the detected object. The first posture positioning feature may be arranged on at least one of the measurement probe, the fixing seat, the detected object and other objects. For the description of the first posture positioning feature and the structure for acquiring the third target image, reference may be made to the description of the first posture positioning feature and the structure for projecting the second projection feature, and details will not be repeated here. A difference is that the first posture positioning feature may be arranged on at least one of the detected object, the measurement probe, the fixing seat, or other objects if the structure for acquiring the third target image is arranged on the measurement probe; the first posture positioning feature may be arranged on at least one of the detected object, the measurement probe, the fixing seat, or other objects if the structure for acquiring the third target image is arranged on the fixing seat.

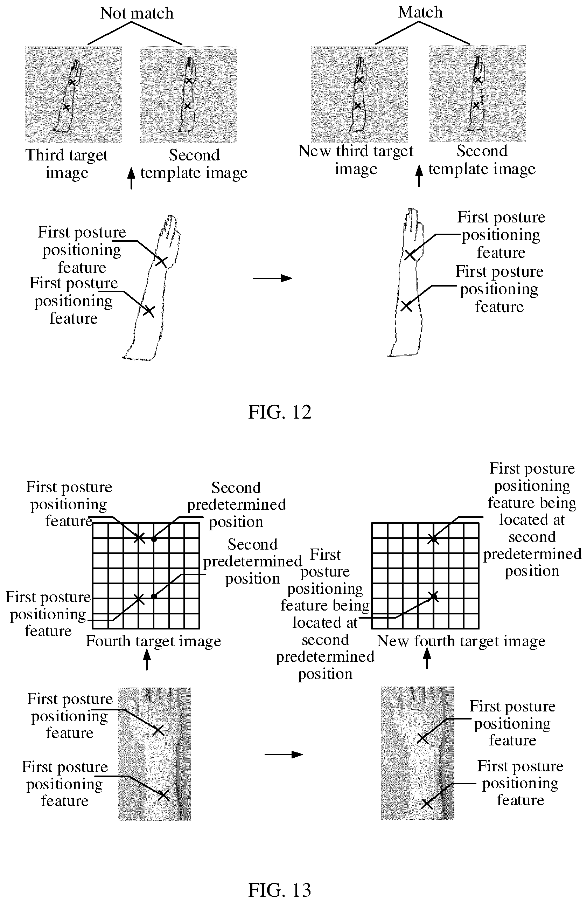

In an example, FIG. 12 schematically shows a schematic diagram of positioning the measurement posture based on an image matching method according to embodiments of the present disclosure. As shown in FIG. 12, the first posture positioning feature is arranged on the detected object.

According to embodiments of the present disclosure, when it is determined that the third target image is matched with the second template image, it may indicate that the current measurement posture is the target measurement posture.

According to embodiments of the present disclosure, adjusting the current measurement posture of the detected object to the target measurement posture according to the first posture positioning feature may include the following operations.

A fourth target image is obtained, where the fourth target image includes the first posture positioning feature. When it is determined that the position of the first posture positioning feature in the fourth target image is not a second predetermined position, the current measurement posture is adjusted to obtain a new fourth target image until the position of the first posture positioning feature in the new fourth target image is the second predetermined position. When it is determined that the position of the first posture positioning feature in the new fourth target image is the second predetermined position, it is determined that the current measurement posture is the target measurement posture.

According to embodiments of the present disclosure, in order to ensure the flexibility of use and the positioning accuracy of the measurement posture, it is possible to adopt an imaging method, that is, if the position of the first posture positioning feature in the fourth target image is the second predetermined position, it may indicate that the positioning of the measurement posture is completed.

According to embodiments of the present disclosure, the process of positioning the measurement posture using the imaging method is a process of determining whether the position of the first posture positioning feature in the fourth target image is the second predetermined position. If the position of the first posture positioning feature in the four target image is not the second predetermined position, the current measurement posture may be adjusted to obtain a new fourth target image until the position of the first posture positioning feature in the new fourth target image is the second predetermined position. When the position of the first posture positioning feature in the new fourth target image is the second predetermined position, it may indicate that the current measurement posture is the target measurement posture.

According to embodiments of the present disclosure, a structure for acquiring the fourth target image may be arranged on the detected object, the measurement probe, the fixing seat or other objects. Other objects may refer to objects other than the measurement probe, the fixing portion, and the detected object. The first posture positioning feature may be arranged on at least one of the measurement probe, the fixing seat, the detected object, or other objects. For the description of the first posture positioning feature and the structure for acquiring the fourth target image, reference may be made to the description of the first posture positioning feature and the structure for projecting the second projection feature, and details will not be repeated here.

In an example, FIG. 13 schematically shows a schematic diagram of positioning the measurement posture based on an imaging method according to embodiments of the present disclosure. As shown in FIG. 13, the first posture positioning feature is arranged on the detected object.

According to embodiments of the present disclosure, the method may further include the following operations.

If the measurement probe is arranged at the position corresponding to the measurement region, a second posture positioning feature is determined when it is determined that the current measurement posture is not the target measurement posture. The current measurement posture is adjusted to the target measurement posture according to the second posture positioning feature.

According to embodiments of the present disclosure, if the measurement probe is arranged at the position corresponding to the measurement region, the above-mentioned second positioning of the measurement posture needs to be performed when it is determined that the current measurement posture is not the target measurement posture. That is, after the positioning of the measurement region is completed, the above-mentioned second positioning of the measurement posture needs to be performed if the current measurement posture is not the target measurement posture. The current measurement posture may be adjusted according to the second posture positioning feature until the current measurement posture is the target measurement posture. The second posture positioning feature may be the same as or different from the first posture positioning feature.

According to embodiments of the present disclosure, adjusting the current measurement posture to the target measurement posture according to the second posture positioning feature may include the following operations.

A third projection feature is obtained. When it is determined that the second posture positioning feature is not matched with the third projection feature, the current measurement posture is adjusted until the second posture positioning feature is matched with the third projection feature. When it is determined that the second posture positioning feature is matched with the third projection feature, it is determined that the current measurement posture is the target measurement posture.

According to embodiments of the present disclosure, in order to ensure the flexibility of use and the positioning accuracy of the measurement posture, it is possible to adopt an optical method, that is, to match the second posture positioning feature with the third projection feature, and determine the target measurement posture according to a matching result. The third projection feature is formed according to an optical method, that is, by projecting a light spot with a predetermined shape using a light source. The shape of the light spot may be determined according to the second posture positioning feature. That is, for the detected object, the third projection feature matched with the second posture positioning feature is provided according to the second posture positioning feature, so that the current measurement posture with which the second posture positioning feature is matched with the third projection feature is the target measurement posture.

According to embodiments of the present disclosure, a structure for projecting the third projection feature may be arranged on the detected object, the measurement probe, the fixing seat or other objects. Other objects may refer to objects other than the measurement probe, the fixing portion and the detected object. The second posture positioning feature may be arranged on at least one of the measurement probe, the fixing seat, the detected object, or other objects. An adjustment process implemented based on the optical method will be described below from two aspects, including an arrangement position of the structure for projecting the third projection feature and an arrangement position of the second posture positioning feature.

A description will be given below from the aspect of the arrangement position of the structure for projecting the third projection feature.

In an aspect, if the structure for projecting the third projection feature is arranged on the detected object, the second posture positioning feature may be arranged on at least one of the detected object, the measurement probe, the fixing seat, or other objects.

In another aspect, if the structure for projecting the third projection feature is arranged on the measurement probe, the second posture positioning feature may not be arranged on the measurement probe and the fixing seat, but may be arranged on the detected object or other objects, because the measurement probe is located on the fixing seat after it is arranged at the position corresponding to the measurement region.

In another aspect, if the structure for projecting the third projection feature is arranged on the fixing seat, the second posture positioning feature may not be arranged on the measurement probe and the fixing seat, but may be arranged on the detected object or other objects, because the measurement probe is located on the fixing seat after it is arranged at the position corresponding to the measurement region.

In another aspect, if the structure for projecting the third projection feature is arranged on other objects, the second posture positioning feature may be arranged on at least one of the detected object, the measurement probe, the fixing seat, or other objects. It should be noted that if the second posture positioning feature is arranged on other objects, the positioning of the measurement posture may be achieved by the method of adjusting the current measurement posture when it is determined that the second posture positioning feature is not matched with the third projection feature, until the second posture positioning feature is matched with the third projection feature, and determining that the current measurement posture is the target measurement posture when it is determined that the second posture positioning feature is matched with the third projection feature. The second posture positioning feature being matched with the third projection feature here means that the second posture positioning feature is blocked by the detected object, so that the third projection feature may not be projected to the position where the second posture positioning feature is located. If the second posture positioning feature is not matched with the third projection feature, at least one third projection feature may be projected to the position where the second posture positioning feature is located.

A description will be given below from the aspect of the arrangement position of the second posture positioning feature.

In an aspect, if the second posture positioning feature is arranged on the detected object, the structure for projecting the third projection feature may be arranged on the detected object, the measurement probe, the fixing seat or other objects.

In another aspect, if the second posture positioning feature is arranged on the measurement probe, the structure for projecting the third projection feature is separated from the measurement probe and the fixing seat, and may be arranged on the detected object or other objects, because the measurement probe is located on the fixing seat after it is arranged at the position corresponding to the measurement region.

In another aspect, if the second posture positioning feature is arranged on the fixing seat, then the structure for projecting the third projection feature is separated from the measurement probe and the fixing seat, and may be arranged on the detected object or other objects, because the measurement probe is located on the fixing seat after it is arranged at the position corresponding to the measurement region.

In another aspect, if the second posture positioning feature is arranged on other objects, the structure for projecting the third projection feature may be arranged on the detected object, the measurement probe, the fixing seat or other objects. It should be noted that if the structure for projecting the third projection feature is arranged on other objects, reference may be made to the above description of the corresponding part, and details will not be repeated here.

When positioning the measurement posture using an optical method, in an aspect, a position and an angle of the light source may be adjusted flexibly and may be easily matched with the second posture positioning feature. Therefore, the second posture positioning feature may be arranged flexibly, so that the difficulty in the arrangement of the second posture positioning feature is reduced. In another aspect, the shape of the emitted light spot may be adjusted to be matched with the second posture positioning feature better, so that the positioning accuracy may be improved.

According to embodiments of the present disclosure, adjusting the current measurement posture to the target measurement posture according to the second posture positioning feature may include the following operations.

A fifth target image is obtained. A third template image is obtained, where the third template image includes the second posture positioning feature. When it is determined that the fifth target image is not matched with the third template image, the current measurement posture is adjusted to obtain a new fifth target image until the new fifth target image is matched with the third template image. When it is determined that the new fifth target image is matched with the third template image, it is determined that the current measurement posture is the target measurement posture.

According to embodiments of the present disclosure, in order to ensure the flexibility of use and the positioning accuracy of the measurement posture, it is possible to adopt an image matching method, that is, to match the fifth target image with the third template image, and determine the target measurement posture according to a matching result. The third template image may include the second posture positioning feature, and the position of the second posture positioning feature in the third template image is a predetermined position. In the process of matching the fifth target image with the third template image, the fifth target image may be a target image that does not include the second posture positioning feature, or may be a target image that includes the second posture positioning feature but the position of the second posture positioning feature in the fifth target image is not the predetermined position, or may be a target image that includes the second posture positioning feature and the position of the second posture positioning feature in the fifth target image is the predetermined position. As the third template image includes the second posture positioning feature at the predetermined position, if the fifth target image is matched with the third template image, it may indicate that the fifth target image includes the second posture positioning feature and the position of the second posture positioning feature in the fifth target image is the predetermined position. In other words, a purpose of matching the fifth target image with the third template image is to obtain such fifth target image that the second posture positioning feature is included in the fifth target image and the position of the second posture positioning feature in the fifth target image is the predetermined position.

According to embodiments of the present disclosure, when it is determined that the fifth target image is matched with the third template image, it may indicate that the current measurement posture is the target measurement posture.

According to embodiments of the present disclosure, a structure for acquiring the fifth target image may be arranged on the detected object, the measurement probe, the fixing seat, or other objects. Other objects may refer to objects other than the measurement probe, the fixing portion, and the detected object. The second posture positioning feature may be arranged on at least one of the measurement probe, the fixing seat, the detected object, or other objects. For the description of the second posture positioning feature and the structure for acquiring the fifth target image, reference may be made to the description of the second posture positioning feature and the structure for projecting the third projection feature, and details will not be repeated here. A difference is that the second posture positioning feature may be arranged on at least one of the detected object, the measurement probe, the fixing seat, or other objects if the structure for acquiring the fifth target image is arranged on the measurement probe; the second posture positioning feature may be arranged on at least one of the detected object, the measurement probe, the fixing seat, or other objects if the structure for acquiring the fifth target image is arranged on the fixing seat.

According to embodiments of the present disclosure, adjusting the current measurement posture to the target measurement posture according to the second posture positioning feature may include the following operations.

A sixth target image is obtained, where the sixth target image includes the second posture positioning feature. When it is determined that the position of the second posture positioning feature in the sixth target image is not a third predetermined position, the current measurement posture is adjusted to obtain a new sixth target image until the position of the second posture positioning feature in the new sixth target image is the third predetermined position. When it is determined that the position of the second posture positioning feature in the new sixth target image is the third predetermined position, it is determined that the current measurement posture is the target measurement posture.

According to embodiments of the present disclosure, in order to ensure the flexibility of use and the positioning accuracy of the measurement posture, it is possible to adopt an imaging method, that is, if the position of the second posture positioning feature in the sixth target image is the third predetermined position, it may indicate that the positioning of the measurement posture is completed.

According to embodiments of the present disclosure, the process of positioning the measurement posture using the imaging method is a process of determining whether the position of the second posture positioning feature in the sixth target image is the third predetermined position. If the position of the second posture positioning feature in the sixth target image is not the third predetermined position, the current measurement posture may be adjusted to obtain a new sixth target image until the position of the second posture positioning feature in the new sixth target image is the third predetermined position. When the position of the second posture positioning feature in the new sixth target image is the third predetermined position, it may indicate that the current measurement posture is the target measurement posture.

According to embodiments of the present disclosure, a structure for acquiring the sixth target image may be arranged on the detected object, the measurement probe, the fixing seat, or other objects. Other objects may refer to objects other than the measurement probe, the fixing portion, and the detected object. The second posture positioning feature may be arranged on at least one of the measurement probe, the fixing seat, the detected object, or other objects. For the description of the second posture positioning feature and the structure for acquiring the sixth target image, reference may be made to the description of the second posture positioning feature and the structure for projecting the third projection feature, and details will not be repeated here.

According to embodiments of the present disclosure, the method may further include the following operations.

A prompt information is generated. The prompt information is used to prompt a completion of the positioning of the measurement posture and/or a completion of the positioning of the measurement region. A form of the prompt information includes at least one of an image, a speech, or a vibration.

According to embodiments of the present disclosure, in order to allow a user to timely know whether the positioning of the measurement posture and/or the positioning of the measurement region are/is completed, the prompt information may be generated after the completion of the positioning of the measurement posture and/or the completion of the positioning of the measurement region. A specific representation form of the prompt information may include at least one of an image, a speech, or a vibration.

According to embodiments of the present disclosure, the method may further include the following operations.

The measurement probe is arranged on the fixing seat when it is determined that the fixing seat is arranged at the position corresponding to the measurement region and that the measurement probe is not arranged on the fixing portion. When it is determined that the fixing seat is not arranged at the position corresponding to the measurement region, the fixing seat is arranged at the position corresponding to the measurement region by the first fitting part, and the measurement probe is arranged on the fixing seat.

According to embodiments of the present disclosure, if the measurement probe is arranged at the position corresponding to the measurement region by the fixing seat, then during the process of measuring the tissue element, the fixing seat may be detached from the measurement region, and the measurement probe may be detached from the fixing seat. When it is required to perform a measurement, if the fixing seat is not arranged at the position corresponding to the measurement region, then the fixing seat may be arranged at the position corresponding to the measurement region by the first fitting part, and the measurement probe may be arranged on the fixing seat. If the fixing seat is arranged at the position corresponding to the measurement region and the measurement probe is not arranged on the fixing seat, then the measurement probe may be arranged on the fixing seat.

In an example, for a short-term measurement at any time, the fixing seat may be arranged at the position corresponding to the measurement region, and the measurement probe may be detached from the fixing seat; when it is required to perform a measurement, the measurement probe may be arranged on the fixing seat. For a long-term measurement, the fixing seat may be detached from the measurement region, and the measurement probe may be detached from the fixing seat; when it is required to perform a measurement, the fixing seat may be arranged at the position corresponding to the measurement region by the first fitting part, and the measurement probe may be arranged on the fixing seat.

According to embodiments of the present disclosure, the method may further include the following operations.

When it is determined that the measurement probe is not arranged at the position corresponding to the measurement region, the measurement probe is arranged at the position corresponding to the measurement region by the second fitting part.

According to embodiments of the present disclosure, if the measurement probe is directly arranged at the position corresponding to the measurement region, then during the process of measuring the tissue element, the measurement probe may be detached from the measurement region, and when it is required to perform a measurement, the measurement probe may be arranged at the position corresponding to the measurement region by the second fitting part.

According to embodiments of the present disclosure, determining the concentration of the detected tissue element according to the at least one output light intensity corresponding to the predetermined wavelength may include the following operations.

A first output light intensity and a second output light intensity are determined from the at least one output light intensity corresponding to the predetermined wavelength. The concentration of the detected tissue element is determined according to the first output light intensity and the second output light intensity corresponding to the predetermined wavelength.

According to embodiments of the present disclosure, the average optical path of the exit light corresponding to the first output light intensity is different from the average optical path of the exit light corresponding to the second output light intensity.

According to embodiments of the present disclosure, determining the concentration of the detected tissue element according to the first output light intensity and the second output light intensity corresponding to the predetermined wavelength may include the following operations.

A differential processing is performed on the first output light intensity and the second output light intensity corresponding to the predetermined wavelength to obtain a differential signal. The concentration of the detected tissue element is determined according to the differential signal corresponding to the predetermined wavelength.

According to embodiments of the present disclosure, as the change in the uncontrollable measurement condition is unpredictable and uncontrollable, it is difficult to reduce the influence of the change in the uncontrollable measurement condition on the measurement result by achieving a reproducibility using an effective control method. In order to improve the reliability of the measurement result, the inventors found that the influence of the change in the uncontrollable measurement condition on the measurement result may be reduced by using a proper mathematical algorithm, so that the influence on the measurement result may be reduced to a negligible level, that is, the influence of the change in the uncontrollable measurement condition on the measurement result is at a similar level to an influence of a random noise on the measurement result.

In order to reduce the influence of the change in the uncontrollable measurement condition on the measurement result, it is possible to adopt an interference suppression method, which may include a differential measurement method. The differential measurement method may include a temporal differential measurement method and a position differential measurement method. The differential measurement method may be implemented to reduce the influence of the change in the uncontrollable measurement condition on the measurement result for the following reasons. If the output light intensities corresponding to different average optical paths carry substantially the same interference information, that is, the interference has substantially the same influence on the output light intensities corresponding to different average optical paths, then a differential processing may be performed on the output light intensities corresponding to two average optical paths (that is, the first output light intensity and the second output light intensity ) to obtain a differential signal, as the output light intensities corresponding to different average optical paths carry different valid information. The concentration of the detected tissue element may be determined according to the differential signal. The interference information may be understood as a response of the output light intensity to the interference. The valid information may be understood as a response of the output light intensity to the detected tissue element.

According to embodiments of the present disclosure, the differential processing performed on the first output light intensity and the second output light intensity corresponding to the predetermined wavelength may include a processing method in terms of hardware and a processing method in terms of software. The processing method in terms of hardware may include processing by using a differential circuit. The processing method in terms of software may include performing a differential operation by using a differential algorithm. The differential algorithm may include a direct differential operation and a logarithmic differential operation. The direct differential operation refers to directly performing a differential processing on two parameters. The logarithmic differential operation refers to performing a logarithmic operation on two parameters to obtain logarithmic parameters, and then performing a differential processing on the two logarithmic parameters.

According to embodiments of the present disclosure, it is possible to effectively weaken a common mode interference information through the differential measurement method, thereby improving the reliability of the measurement result.

According to embodiments of the present disclosure, performing the differential processing on the first output light intensity and the second output light intensity corresponding to the predetermined wavelength to obtain the differential signal may include the following operations.

The first output light intensity and the second output light intensity corresponding to the predetermined wavelength are processed using a differential circuit, so as to obtain the differential signal.

According to embodiments of the present disclosure, the differential processing on the first output light intensity and the second output light intensity may be performed using a differential circuit, so as to directly obtain the differential signal.

According to embodiments of the present disclosure, performing the differential processing on the first output light intensity and the second output light intensity corresponding to the predetermined wavelength to obtain the differential signal may include the following operations.

The first output light intensity and the second output light intensity corresponding to the predetermined wavelength are processed using a differential algorithm, so as to obtain the differential signal.

According to embodiments of the present disclosure, processing the first output light intensity and the second output light intensity corresponding to the predetermined wavelength using a differential algorithm to obtain the differential signal may include the following operations.

A direct differential operation is performed on the first output light intensity and the second output light intensity corresponding to the predetermined wavelength, so as to obtain the differential signal.

According to embodiments of the present disclosure, processing the first output light intensity and the second output light intensity corresponding to the predetermined wavelength using a differential algorithm to obtain the differential signal may include the following operations.

A logarithmic processing is performed on the first output light intensity and the second output light intensity corresponding to the predetermined wavelength, so as to obtain a first logarithmic light intensity and a second logarithmic light intensity. A direct differential operation is performed on the first logarithmic light intensity and the second logarithmic light intensity corresponding to the predetermined wavelength, so as to obtain the differential signal.

According to embodiments of the present disclosure, the first logarithmic light intensity represents a logarithm of the first output light intensity, and the second logarithmic light intensity represents a logarithm of the second output light intensity.

The differential signal may be determined by Equation (1),

$$A_D = \ln\left(I\left(\overline{L_1}\right)\right) - \ln\left(I\left(\overline{L_2}\right)\right) \qquad (1)$$

where $A_D$ represents the differential signal, $I(\overline{L_1})$ represents the first output light intensity, $I(\overline{L_2})$ represents the second output light intensity, $\overline{L_1}$ represents the average optical path corresponding to the first output light intensity, and $\overline{L_2}$ represents the average optical path corresponding to the second output light intensity.

According to embodiments of the present disclosure, determining the concentration of the detected tissue element according to the differential signal corresponding to the predetermined wavelength may include the following operations.

The differential signal corresponding to the predetermined wavelength is input into a first concentration prediction model for the tissue element to output the concentration of the detected tissue element.

According to embodiments of the present disclosure, the first concentration prediction model for the tissue element may be obtained by training a chemometric model. The chemometric model may include a regression model, the regression model may include a linear regression model, and the linear regression model may include a unary linear regression model.

According to embodiments of the present disclosure, a set of first training samples is obtained, where the set of first training samples includes a plurality of first training samples, and each first training sample includes a first true concentration of the detected tissue element and a differential signal corresponding to the first true concentration. The first concentration prediction model for the tissue element is established according to the set of first training samples.

According to embodiments of the present disclosure, the first concentration prediction model for the tissue element may be generated as follows. A set of first training samples is obtained, where the set of first training samples includes a plurality of first training samples, and the first training sample includes a first true concentration of the detected tissue element and a differential signal corresponding to the first true concentration. A mathematical model to be trained is trained by using the differential signal corresponding to the first true concentration as an input variable and using the first true concentration as an output variable, so as to obtain the first concentration prediction model for the tissue element.

According to embodiments of the present disclosure, establishing the first concentration prediction model for the tissue element according to the set of first training samples may include the following operations.

The set of first training samples is pre-processed to obtain a processed set of first training samples. The first concentration prediction model for the tissue element is established according to the processed set of first training samples.

According to embodiments of the present disclosure, in order to improve a prediction accuracy of the model, the set of first training samples may be pre-processed to determine an abnormal training sample in the set of first training samples, and the first concentration prediction model for the tissue element may be established according to the set of first training samples after a removal of the abnormal training sample.

According to embodiments of the present disclosure, the method may further include the following operations.

When a fourth predetermined condition is met, the first concentration prediction model for the tissue element is corrected to obtain a corrected first concentration prediction model for the tissue element, so as to process a new differential signal using the corrected first concentration prediction model for the tissue element to obtain a new concentration of the detected tissue element.

According to embodiments of the present disclosure, in order to improve the prediction accuracy of the model, it is needed to adjust the first concentration prediction model for the tissue element according to an actual condition during the process of measuring the tissue element, that is, if the actual condition meets the fourth predetermined condition, the first concentration prediction model for the tissue element may be corrected to obtain the corrected first concentration prediction model for the tissue element. The fourth predetermined condition may include that a time interval between a current tissue element measurement and the establishment of the first concentration prediction model for the tissue element is less than or equal to a time threshold, and/or a state of the detected object does not change significantly. The state of the detected object may include a physical state of the detected object and/or a skin state of the detected object, such as a skin burn.

An inapplicability of the first concentration prediction model for the tissue element caused by a change in the tissue element of the detected object and a change in the state of the measurement device within a short period of time or without a significant change in the state of the detected object may be generally solved by correcting the first concentration prediction model for the tissue element.

After the corrected first concentration prediction model for the tissue element is obtained, a new differential signal may be obtained and input into the corrected first concentration prediction model for the tissue element to output a new concentration of the detected tissue element.

According to embodiments of the present disclosure, correcting the first concentration prediction model for the tissue element may include the following operations.

A first target concentration of the detected tissue element is obtained. A differential signal corresponding to the first target concentration is obtained. The first concentration prediction model for the tissue element is corrected according to the first target concentration and the differential signal corresponding to the first target concentration.

According to embodiments of the present disclosure, the first concentration prediction model for the tissue element may be trained based on a unary linear regression model. Two input parameters of the unary linear regression model include a slope and an intercept, and a model training process is to determine the slope and the intercept. Through researches, it has been found that if the fourth predetermined condition is met, the slope of the first concentration prediction model for the tissue element remains unchanged but the intercept changes. In this case, it is needed to re-determine the intercept. Then, the first concentration prediction model for the tissue element may be corrected to obtain a new intercept according to the first target concentration and the differential signal corresponding to the first target concentration, and then the corrected first concentration prediction model for the tissue element is obtained.

According to embodiments of the present disclosure, the method may further include the following operations.

When a fifth predetermined condition is met, a new differential signal is processed by using a new first concentration prediction model for the tissue element, so as to obtain a new concentration of the detected tissue element.

According to embodiments of the present disclosure, in order to improve the prediction accuracy of the model, it is needed to determine, according to the actual condition during the process of measuring the tissue element, whether to re-establish a new first concentration prediction model for the tissue element, that is, a new first concentration prediction model for the tissue element may be established if the actual condition meets the fifth predetermined condition. The fifth predetermined condition may include that a time interval between a current tissue element measurement and the establishment of the first concentration prediction model for the tissue element is greater than a time threshold, and/or a state of the detected object changes significantly. The state of the detected object may include the physical state of the detected object and/or the skin state of the detected object, such as a skin burn.

The above-mentioned method is adopted because there is generally a significant change in the tissue element of the detected object and the state of the measurement device after a long period of time or in a case of a great change in the state of the detected object, so that the original first concentration prediction model for the tissue element is not applicable and it is not possible to make it applicable by correcting the original first concentration prediction model for the tissue element. Then, it is needed to re-establish a new first concentration prediction model for the tissue element.

According to embodiments of the present disclosure, the new first concentration prediction model for the tissue element may be generated as follows. A new set of first training samples is obtained, where the new set of first training samples includes a plurality of new first training samples, and each new first training sample includes a new first true concentration of the detected tissue element and a differential signal corresponding to the new first true concentration. The new first concentration prediction model for the tissue element is established according to the new set of first training samples.

According to embodiments of the present disclosure, determining the concentration of the detected tissue element according to the differential signal corresponding to the predetermined wavelength may include the following operations.

A current interference parameter value of each interference parameter of a plurality of interference parameters is obtained. A plurality of current interference parameter values and the differential signal corresponding to the predetermined wavelength are input into a second concentration prediction model for the tissue element to output the concentration of the detected tissue element.

According to embodiments of the present disclosure, the measurement of the concentration of the tissue element may be affected by an interference parameter. The interference parameter may include a temperature and a pressure, etc. An interference parameter-based second concentration prediction model for the tissue element may be established in order to improve the reliability of the measurement result.

Processing the plurality of current interference parameter values and the differential signal corresponding to the predetermined wavelength using the second concentration prediction model for the tissue element to obtain the concentration of the detected tissue element may include the following operations. The plurality of current interference parameter values are input into the second concentration prediction model for the tissue element to output a differential interference signal. The differential signal corresponding to the predetermined wavelength is corrected by using the difference interference signal, so as to obtain a correction signal corresponding to the predetermined wavelength. The concentration of the detected tissue element is determined according to the correction signal corresponding to the predetermined wavelength.

According to embodiments of the present disclosure, the reliability of the measurement result is further improved by establishing the interference parameter-based second concentration prediction model for the tissue element for predicting the concentration of the tissue element.

According to embodiments of the present disclosure, the method may further include the following operations.

A set of second training samples is obtained, where the set of second training samples includes a plurality of second training samples, and each second training sample includes a second true concentration of the detected tissue element and a differential signal corresponding to the second true concentration. A set of third training samples is obtained, where the set of third training samples includes a plurality of third training samples, and each third training sample includes a training interference parameter value of each interference parameter of a plurality of interference parameters and a differential signal corresponding to each training interference parameter value. A tissue element concentration prediction model to be corrected is established according to the set of second training samples. A correction parameter model is established according to the set of third training samples. The second concentration prediction model for the tissue element is obtained according to the tissue element concentration prediction model to be corrected and the correction parameter model.

According to embodiments of the present disclosure, the correction parameter model is a mathematical model between an interference parameter and a differential signal corresponding to the interference parameter. The tissue element concentration prediction model to be corrected is a mathematical model between the tissue element concentration and the differential signal corresponding to the tissue element concentration. Obtaining the second concentration prediction model for the tissue element according to the tissue element concentration prediction model to be corrected and the correction parameter model may include: obtaining the differential signal corresponding to the interference parameter according to the correction parameter model, correcting the differential signal corresponding to the tissue element concentration by using the differential signal corresponding to the interference parameter, and establishing the second concentration prediction model for the tissue element according to the tissue element concentration and the corrected differential signal. During the model training process, the tissue element concentration is the second true concentration, and the differential signal corresponding to the tissue element concentration is the differential signal corresponding to the second true concentration.

According to embodiments of the present disclosure, in order to improve the prediction accuracy of the model, both the set of second training samples and the set of third training samples may be pre-processed to determine an abnormal training sample in the set of second training samples and an abnormal training sample in the set of third training samples. The tissue element concentration prediction model to be corrected may be established according to the set of second training samples after a removal of the abnormal training sample. The correction parameter model may be established according to the set of third training samples after a removal of the abnormal training sample.

According to embodiments of the present disclosure, the method may further include the following operations.

When the fourth predetermined condition is met, the second concentration prediction model for the tissue element is corrected to obtain a corrected second concentration prediction model for the tissue element, so as to process a new differential signal and a plurality of new current interference parameter values by using the corrected second concentration prediction model for the tissue element to obtain a new concentration of the detected tissue element.

According to embodiments of the present disclosure, in order to improve the prediction accuracy of the model, it is needed to adjust the second concentration prediction model for the tissue element according to the actual condition during the process of measuring the tissue element, that is, the second concentration prediction model for the tissue element may be corrected to obtain a corrected second concentration prediction model for the tissue element if the actual condition meets the fourth predetermined condition. The fourth predetermined condition may include that a time interval between a current tissue element measurement and the establishment of the second concentration prediction model for the tissue element is less than or equal to a time threshold, and/or a state of the detected object does not change significantly. The state of the detected object may include the physical state of the detected object and/or the skin state of the detected object, such as a skin burn.

An inapplicability of the second concentration prediction model for the tissue element caused by a change in the tissue element of the detected object and a change in the state of the measurement device within a short period of time or without a significant change in the state of the detected object may be generally solved by correcting the second concentration prediction model for the tissue element.

After the corrected second concentration prediction model for the tissue element is obtained, a new differential signal and a plurality of new current interference parameter values may be obtained and input into the corrected second concentration prediction model for the tissue element to output a new concentration of the detected tissue element.

According to embodiments of the present disclosure, correcting the second concentration prediction model for the tissue element may include the following operations.

A second target concentration of the detected tissue element is obtained. A differential signal corresponding to the second target concentration is obtained. A current interference parameter value of each interference parameter of a plurality of interference parameters is obtained. The second concentration prediction model for the tissue element is corrected according to the second target concentration, a plurality of interference parameter values, and the differential signal corresponding to the second target concentration.

According to embodiments of the present disclosure, the second concentration prediction model for the tissue element may be trained based on a unary linear regression model. Two input parameters of the unary linear regression model include a slope and an intercept, and a model training process is to determine the slope and the intercept. Through researches, it has been found that that if the first predetermined condition is met, the slope of the second concentration prediction model for the tissue element remains unchanged but the intercept changes. In this case, it is needed to re-determine the intercept. Then, the second concentration prediction model for the tissue element may be corrected to obtain a new intercept according to the second target concentration, the differential signal corresponding to the second target concentration and a plurality of interference parameter values, and then the corrected second concentration prediction model for the tissue element is obtained.

According to embodiments of the present disclosure, the method may further include the following operations.

When a fifth predetermined condition is met, a new differential signal and a plurality of new current interference parameter values are processed by using a new second concentration prediction model for the tissue element, so as to obtain a new concentration of the detected tissue element.

According to embodiments of the present disclosure, in order to improve the prediction accuracy of the model, it is needed to determine, according to the actual condition during the process of measuring the tissue element, whether to re-establish a new second concentration prediction model for the tissue element, that is, a new second concentration prediction model for the tissue element may be established if the actual condition meets the fifth predetermined condition. The fifth predetermined condition may include that a time interval between a current tissue element measurement and the establishment of the second concentration prediction model for the tissue element is greater than a time threshold, and/or a state of the detected object changes significantly. The state of the detected object may include the physical state of the detected object and/or the skin state of the detected object, such as a skin burn.

The above-mentioned method is adopted because there is generally a significant change in the tissue element of the detected object and the state of the measurement device after a long period of time or in a case of a great change in the state of the detected object, so that the original second concentration prediction model for the tissue element is not applicable and it is not possible to make it applicable by correcting the original second concentration prediction model for the tissue element. Then, it is needed to re-establish a new second concentration prediction model for the tissue element.

According to embodiments of the present disclosure, the new second concentration prediction model for the tissue element may be generated as follows. A new set of second training samples is obtained, where the new set of second training samples includes a plurality of new second training samples, and each new second training sample includes a new second true concentration of the detected tissue element and a differential signal corresponding to the new second true concentration. A new set of third training samples is obtained, where the new set of third training samples includes a plurality of new third training samples, and each new third training sample includes a new training interference parameter value of each interference parameter of a plurality of interference parameters and a differential signal corresponding to each new training interference parameter value. A new tissue element concentration prediction model to be corrected is established according to the new set of second training samples. A new correction parameter model is established according to the new set of third training samples. The new second concentration prediction model for the tissue element is obtained according to the new tissue element concentration prediction model to be corrected and the new correction parameter model.

According to embodiments of the present disclosure, the first output light intensity and the second output light intensity are acquired at different time instants by the same homogeneous photosensitive surface or different homogeneous photosensitive surfaces. The first output light intensity is a light intensity for a contraction period, and the second output light intensity is a light intensity for a relaxation period. The homogeneous photosensitive surface includes one or more photosensitive surfaces, and the homogeneous photosensitive surface is used to output one output light intensity.

According to embodiments of the present disclosure, in a case that the first output light intensity and the second output light intensity are acquired at different time instants by the same homogeneous photosensitive surface or different homogeneous photosensitive surfaces, the tissue element measurement may be performed using a pulse wave-based temporal differential measurement method.

A pulse beat, also known as an arterial pulsation, refers to a periodic contraction and relaxation with the beating of the heart, a pressure inside an aorta causes a pulsatile change in a diameter of a blood vessel, and a blood flow in the blood vessel also changes regularly and periodically. Each pulse waveform includes an ascending branch and a descending branch. The ascending branch represents a dilation of the artery during the contraction period of the ventricle, and the descending branch represents a retraction of the artery during the relaxation period of the ventricle. A contraction and a relaxation of the ventricle represent a pulsation cycle.

According to embodiments of the present disclosure, as the pulse wave-based temporal differential measurement method is adopted, it is required to utilize an information of the pulse beat as much as possible. In order to improve the reliability of the measurement result, the photosensitive surface may be arranged at a position as close as possible to the target site (such as a target blood vessel). That is, the homogeneous photosensitive surface for outputting the first output light intensity and the second output light intensity may be arranged at a position from which a distance to the target site is less than or equal to a fourth distance threshold. The fourth distance threshold may be zero, that is, the homogeneous photosensitive surface may be arranged on the target site. The homogeneous photosensitive surface for outputting the first output light intensity and the second output light intensity being arranged at the position from which the distance to the target site is less than or equal to the fourth distance threshold means that the distance from the target site to each photosensitive surface in the homogeneous photosensitive surface for outputting the first output light intensity and the second output light intensity is less than or equal to the fourth distance threshold. The distance from the target site to each photosensitive surface in the homogeneous photosensitive surface being less than or equal to the fourth distance threshold may mean that a distance from the target blood vessel to an edge of the photosensitive surface farthest away from the target site in the homogeneous photosensitive surface is less than or equal to the fourth distance threshold.

It should be noted that utilizing the information of the pulse beat as much as possible when adopting the pulse wave-based temporal differential measurement method is not contradictory to the above-mentioned reducing the adverse influences of the pulse beat on the measurement result by using the large-area photosensitive surface. The former is to utilize a useful information brought by the pulse beat as much as possible, while the latter is to minimize the adverse influence brought by the pulse beat. In addition, the first output light intensity may also be the light intensity for the relaxation period, and the second output light intensity may also be the light intensity for the contraction period. The first output light intensity and the second output light intensity corresponding to the predetermined wavelength may be output light intensities in a same pulsation cycle, or may be output light intensities in different pulsation cycles.

According to embodiments of the present disclosure, the first output light intensity corresponding to the predetermined wavelength is acquired by a first homogeneous photosensitive surface corresponding to the predetermined wavelength, and the second output light intensity corresponding to the predetermined wavelength is acquired by a second homogeneous photosensitive surface corresponding to the predetermined wavelength. The first homogeneous photosensitive surface includes one or more photosensitive surfaces, and the second homogeneous photosensitive surface includes one or more photosensitive surfaces.

For the predetermined wavelength, embodiments of the present disclosure provide the first homogeneous photosensitive surface corresponding to the predetermined wavelength and the second homogeneous photosensitive surface corresponding to the predetermined wavelength. The first homogeneous photosensitive surface is used to output the first output light intensity corresponding to the predetermined wavelength, and the second homogeneous photosensitive surface is used to output the second output light intensity corresponding to the predetermined wavelength. Both the first homogeneous photosensitive surface and the second homogeneous photosensitive surface may include one or more photosensitive surfaces.

According to embodiments of the present disclosure, the first output light intensity and the second output light intensity may be processed using a position differential measurement method, so as to determine the concentration of the detected tissue element.

According to embodiments of the present disclosure, it is needed to avoid the target site (such as the target blood vessel) as much as possible when the position differential measurement method is adopted. In order to improve the reliability of the measurement result, the photosensitive surface may be arranged as far away from the target site as possible. The first homogeneous photosensitive surface for outputting the first output light intensity may be arranged at a position from which a distance to the target site is greater than or equal to a fifth distance threshold, that is, the distance between each photosensitive surface in the first homogeneous photosensitive surface and the target site is greater than or equal to the fifth distance threshold. The distance between each photosensitive surface in the first homogeneous photosensitive surface and the target site being greater than or equal to the fifth distance threshold may mean that the distance between an edge of the photosensitive surface closest to the target site in the first homogeneous photosensitive surface and the target site is greater than or equal to the fifth distance threshold. Alternatively, the first homogeneous photosensitive surface is in non-contact with the target site, and a distance between a center of the photosensitive surface closest to the target site in the first homogeneous photosensitive surface and the target site is greater than or equal to the fifth distance threshold. The second homogeneous photosensitive surface for outputting the second output light intensity is arranged at a position from which a distance to the target site is greater than or equal to a sixth distance threshold. For the understanding of the second homogeneous photosensitive surface for outputting the second output light intensity being arranged at a position from which the distance to the target site is greater than or equal to the sixth distance threshold, reference may be made to the description of the first homogeneous photosensitive surface for outputting the first output light intensity, and details will not be repeated here.

According to embodiments of the present disclosure, the first homogeneous photosensitive surface and the second homogeneous photosensitive surface are the same photosensitive surface, and the exit light received by the first homogeneous photosensitive surface and the exit light received by the second homogeneous photosensitive surface are obtained through a transmission of the incident light incident at different incident positions.

According to embodiments of the present disclosure, the first homogeneous photosensitive surface and the second homogeneous photosensitive surface are different homogeneous photosensitive surfaces.

According to embodiments of the present disclosure, the number of the incident positions of the incident light may be at least one. If the number of the incident positions of the incident light is at least two, the first homogeneous photosensitive surface and the second homogeneous photosensitive surface may be the same photosensitive surface. If the homogeneous photosensitive surface is used to receive the exit light corresponding to the first output light intensity, that is, it is used as the first homogeneous photosensitive surface, then the incident position corresponding to the exit light is a first incident position. If the homogeneous photosensitive surface is used to receive the exit light corresponding to the second output light intensity, that is, it is used as the second homogeneous photosensitive surface, then the incident position corresponding to the exit light is a second incident position. The first incident position and the second incident position are different incident positions.

According to embodiments of the present disclosure, the first homogeneous photosensitive surface and the second homogeneous photosensitive surface may also be different homogeneous photosensitive surfaces.

According to embodiments of the present disclosure, an average optical path of the exit light received at different photosensitive positions of each photosensitive surface in the first homogeneous photosensitive surface is within a first average optical path range. The first average optical path range is determined according to a first optical path average value, and the first optical path average value is an average value calculated according to the average optical paths of the exit light received at the photosensitive positions of the first homogeneous photosensitive surface. An average optical path of the exit light received at different photosensitive positions of each photosensitive surface in the second homogeneous photosensitive surface is within a second average optical path range. The second average optical path range is determined according to a second optical path average value, and the second optical path average value is an average value calculated according to the average optical paths of the exit light received at the photosensitive positions of the second homogeneous photosensitive surface.

According to embodiments of the present disclosure, in order to improve the reliability of the measurement result obtained by performing a tissue element measurement using the position differential measurement method, it is needed to ensure that the exit light received by the first homogeneous photosensitive surface has a characteristic of a short optical path, and the exit light received by the second homogeneous photosensitive surface also has a characteristic of a short optical path. The short optical path may be understood as that the average optical path of the exit light is within an average optical path range.

For the first homogeneous photosensitive surface, the average optical path of the exit light received at different photosensitive positions of each photosensitive surface in the first homogeneous photosensitive surface is within the first average optical path range. The first average optical path range is determined as follows. A first optical path average value for the average optical paths of the exit light received at the photosensitive positions of the first homogeneous photosensitive surface is determined, and a first optical path change amplitude is determined. The first average optical path range is determined according to the first optical path average value and the first optical path change amplitude. For example, if the first optical path average value is b, and the first optical path change amplitude is $\pm 40\%$, then the first average optical path range may be greater than or equal to 0.6b and less than or equal to 1.4b.

For the second homogeneous photosensitive surface, the average optical path of the exit light received at different photosensitive positions of each photosensitive surface in the second homogeneous photosensitive surface is within the second average optical path range. The second average optical path range is determined as follows. A second optical path average value for the average optical paths of the exit light received at the photosensitive positions of the second homogeneous photosensitive surface is determined, and a second optical path change amplitude is determined. The second average optical path range is determined according to the second optical path average value and the second optical path change amplitude.

According to embodiments of the present disclosure, an absolute value of a difference between the first optical path average value and the second optical path average value is within a first optical path difference range.

According to embodiments of the present disclosure, in order to improve the reliability of the measurement result obtained by performing a tissue element measurement based on the differential measurement method, it is needed to arrange the first homogeneous photosensitive surface and the second homogeneous photosensitive surface within an appropriate position range. A description will be given below by taking blood glucose as an example of the detected tissue element. In a case that the detected tissue element is blood glucose, the target tissue layer is the dermis, and the output light intensity is required to be the output light intensity that mainly carries the tissue element information in the dermis.

In an aspect, if the distance between the position of the photosensitive surface and the center of the incident light is too small, the output light intensity of the exit light may mainly carry the tissue element information in the epidermis. If the distance between the position of the photosensitive surface and the center of the incident light is overly large, the output light intensity of the exit light may mainly carry the tissue element information in the subcutaneous fat layer. As the dermis is located between the epidermis and the subcutaneous fat layer, an arrangement position of the first homogeneous photosensitive surface and the second homogeneous photosensitive surface needs to be selected within an appropriate position range, and the distance between the first homogeneous photosensitive surface and the second homogeneous photosensitive surface may not be overly large.

In another aspect, although the differential measurement method may be implemented to effectively weaken the common mode interference, it may also lose part of the valid information, i.e., the blood glucose information, while weakening the common mode interference. If two positions are extremely close, it is possible all valid information may be lost. Therefore, the arrangement position of the first homogeneous photosensitive surface and the second homogeneous photosensitive surface needs to be selected within an appropriate position range, and the distance between the first homogeneous photosensitive surface and the second homogeneous photosensitive surface may not be too small.

In order to arrange the first homogeneous photosensitive surface and the second homogeneous photosensitive surface within an appropriate position range, a determination may be made according to a principle of valid information measurement, a principle of optimization of differential measurement precision, and a principle of effective elimination of interference signals. The principle of valid information measurement may mean that the exit light at two positions may carry as much tissue element information as possible in the target tissue layer. Therefore, the two positions should be within a rational position range. The principle of optimization of differential measurement precision may mean that there should be a certain distance between two positions, so as to ensure that as much valid information as possible is retained after the differential operation. The principle of effective elimination of interference signals may mean that the distance between two positions should be as small as possible to improve the effect of the differential measurement method in eliminating the common mode interference.

Reflected in the optical path, arranging the first homogeneous photosensitive surface and the second homogeneous photosensitive surface within a rational position range requires that the absolute value of the difference between the first optical path average value corresponding to the first homogeneous photosensitive surface and the second optical path average value corresponding to the second homogeneous photosensitive surface is within the first optical path difference range. The first optical path difference range is determined according to an optimal differential optical path. The optimal differential optical path may be determined according to at least one of the above three principles.

It may be understood that the requirement for the position arrangement of the first homogeneous photosensitive surface and the second homogeneous photosensitive surface also require that the area of the photosensitive surface may not be overly large, otherwise a differential effect may be affected, thereby affecting the reliability of the measurement result.

According to embodiments of the present disclosure, the first average optical path range is less than or equal to the first optical path difference range, and the second average optical path range is less than or equal to the first optical path difference range.

According to embodiments of the present disclosure, reflected in the optical path, arranging the first homogeneous photosensitive surface and the second homogeneous photosensitive surface within a rational position range further requires that the first average optical path range is less than or equal to the first optical path difference range, and the second average optical path range is less than or equal to the first optical path difference range. It may be concluded that an absolute value of a difference between the first optical path average value corresponding to the first homogeneous photosensitive surface and the second optical path average value corresponding to the second homogeneous photosensitive surface is within the first optical path difference range, the first average optical path range is less than or equal to the first optical path difference range, and the second average optical path range is less than or equal to the first optical path difference range.

According to embodiments of the present disclosure, the first optical path difference range is determined according to the optimal differential optical path corresponding to the predetermined wavelength.

According to embodiments of the present disclosure, when the measurement region of the detected object is determined, there exists an optimal differential sensitivity corresponding to the predetermined wavelength. The optimal differential sensitivity may represent a sensitivity in a case of a maximum change in the differential signal caused by the change in the concentration of a unit detected tissue element. The optimal differential optical path may be determined according to the optimal differential sensitivity, that is, the optimal differential optical path may be determined according to the principle of optimization of differential measurement precision. Therefore, the optical path corresponding to the optimal differential sensitivity is referred to as the optimal differential optical path.

According to embodiments of the present disclosure, after the optimal differential optical path corresponding to the predetermined wavelength is determined, an up and down adjustment amplitude may be provided, and the first optical path difference range corresponding to the predetermined wavelength is determined according to the optimal differential optical path corresponding to the predetermined wavelength and the up and down adjustment amplitude.

According to embodiments of the present disclosure, a source-detection distance of each photosensitive surface in the first homogeneous photosensitive surface corresponding to the predetermined wavelength from the center of the incident light is within a predetermined source-detection distance range corresponding to the predetermined wavelength. The predetermined source-detection distance range is determined according to a source-detection distance of a floating reference position corresponding to the predetermined wavelength from the center of the incident light.

According to embodiments of the present disclosure, in order to further improve the reliability of the measurement result, the position of the photosensitive surface may be arranged based on a floating reference method. A description is given below for the floating reference method.

For the detected object, when the incident light enters the tissue, absorption and scattering may occur, the absorption may directly cause an attenuation of light energy, and the scattering may affect a distribution of the exit light by changing a transmitting direction of photons, and the distribution of the exit light is a result of a combined effect of the absorption and the scattering. Based on the floating reference method, for the detected tissue element, there is a position away from the center of the incident light at which the absorption and the scattering have a same influence on the output light intensity of the exit light and opposite directions, and therefore the exit light is insensitive to the change in the concentration of the detected tissue element. Such position with above characteristics may be referred to as a benchmark position (or a reference position). The output light intensity of the exit light at the benchmark position reflects a response to the interference other than the detected tissue element during the measurement process. Moreover, for the detected tissue element, there is also a position away from the center of the incident light at which the sensitivity of the output light intensity of the exit light to the change in the concentration of the detected tissue element is greater than or equal to a sensitivity threshold. Such position with above characteristics may be referred to as a measurement position. The output light intensity of the exit light at the measurement position reflects a response to the detected tissue element during the measurement process and a response to the interference other than the detected tissue element. The benchmark position and the measurement position may vary depending on the wavelength, the detected object, and the measurement region. Therefore, the benchmark position may be called a floating reference position.

According to embodiments of the present disclosure, as the output light intensity of the exit light exited from the floating reference position mainly carries the response to the interference other than the detected tissue element during the measurement process, the output light intensity of the exit light exited from the floating reference position may be introduced into the differential measurement to minimize the common mode interference and minimize the loss of valid information. Based on the above, when the measurement region of the detected object is determined, for the predetermined wavelength, the source-detection distance of at least one of the M photosensitive surfaces from the center of the incident light may be within the predetermined source-detection distance range corresponding to the predetermined wavelength. The predetermined source-detection distance range is determined according to the source-detection distance of the floating reference position corresponding to the predetermined wavelength from the center of the incident light. In embodiments of the present disclosure, the source-detection distance of each photosensitive surface in the first homogeneous photosensitive surface from the center of the incident light may be within the predetermined source-detection distance range corresponding to the predetermined wavelength.

In an example, for the measurement region B of the detected object A, the distance between the floating reference position corresponding to the predetermined wavelength $\lambda 1$ and the center of the incident light is 1.7 mm, then the predetermined source-detection distance range corresponding to the predetermined wavelength $\lambda 1$ may be 1.5 mm to 1.9 mm.

Based on the above, the homogeneous photosensitive surface corresponding to the reference position and the homogeneous photosensitive surface corresponding to the measurement position may be determined. The output light intensity acquired by the homogeneous photosensitive surface corresponding to the reference position is called the first output light intensity, and the output light intensity acquired by the homogeneous photosensitive surface corresponding to the measurement region is called the second output light intensity. Alternatively, the output light intensity acquired by the homogeneous photosensitive surface corresponding to the measurement region is called the first output light intensity, and the output light intensity acquired by the homogeneous photosensitive surface corresponding to the reference position is called the second output light intensity.

According to embodiments of the present disclosure, determining the concentration of the detected tissue element according to the at least one output light intensity corresponding to the predetermined wavelength may include the following operations.

A third output light intensity is determined from the at least one output light intensity corresponding to the predetermined wavelength. The concentration of the detected tissue element is determined according to the third output light intensity corresponding to the predetermined wavelength.

According to embodiments of the present disclosure, the tissue element measurement may be performed using a non-differential measurement method, that is, the concentration of the detected tissue element is determined according to the third output light intensity corresponding to the predetermined wavelength.

According to embodiments of the present disclosure, the third output light intensity corresponding to the predetermined wavelength is acquired by the homogeneous photosensitive surface corresponding to the predetermined wavelength, and a difference between the average optical path of the exit light received at different photosensitive positions of each photosensitive surface in the homogeneous photosensitive surface and the optimal optical path corresponding to the predetermined wavelength is within the second optical path difference range.

According to embodiments of the present disclosure, in order to improve the reliability of the measurement result, when the measurement region of the detected object is determined, for the predetermined wavelength, the average optical path of the exit light received at different photosensitive positions of the homogeneous photosensitive surface for acquiring the third output light intensity may be close to the optimal optical path corresponding to the predetermined wavelength, that is, an absolute value of a difference between the average optical path of the exit light received at different photosensitive positions of the homogeneous photosensitive surface for acquiring the third output light intensity and the optimal optical path corresponding to the predetermined wavelength is less than or equal to the second optical path difference range. The optimal optical path corresponding to the predetermined wavelength may be understood as the optical path corresponding to the maximum sensitivity to the detected tissue element at the predetermined wavelength.

According to embodiments of the present disclosure, determining the concentration of the detected tissue element according to the third output light intensity corresponding to the predetermined wavelength may include the following operations.

The third output light intensity corresponding to the predetermined wavelength is input into a third concentration prediction model for the tissue element to output the concentration of the detected tissue element.

According to embodiments of the present disclosure, the third concentration prediction model for the tissue element may be generated as follows. A set of fourth training samples is obtained, where the set of fourth training samples includes a plurality of fourth training samples, and each fourth training sample includes a third true concentration of the detected tissue element and an output light intensity corresponding to the third true concentration. The third concentration prediction model for the tissue element is established according to the set of fourth training samples.

When the fourth predetermined condition is met, a third target concentration of the detected tissue element is obtained, the output light intensity corresponding to the third target concentration is obtained, and the third concentration prediction model for the tissue element is corrected according to the third target concentration and the output light intensity corresponding to the third target concentration, so as to process a new output light intensity using the corrected third concentration prediction model for the tissue element to obtain a new concentration of the detected tissue element.

When the fifth predetermined condition is met, a new output light intensity is processed using a new third concentration prediction model for the tissue element to obtain a new concentration of the detected tissue element.

According to embodiments of the present disclosure, determining the concentration of the detected tissue element according to the third output light intensity corresponding to the predetermined wavelength may include the following operations.

A current interference parameter value of each interference parameter of a plurality of interference parameters is obtained. A plurality of current interference parameter values and the third output light intensity corresponding to the predetermined wavelength are input into a fourth concentration prediction model for the tissue element to output the concentration of the detected tissue element.

According to embodiments of the present disclosure, the fourth concentration prediction model for the tissue element may be generated as follows. A set of fifth training samples is obtained, where the set of fifth training samples includes a plurality of fifth training samples, and each fifth training sample includes a fourth true concentration of the detected tissue element and an output light intensity corresponding to the fourth true concentration. A set of sixth training samples is obtained, where the set of sixth training samples includes a plurality of sixth training samples, and each sixth training sample includes a training interference parameter value of each interference parameter of the plurality of interference parameters and the light intensity value corresponding to each training interference parameter value. A tissue element concentration prediction model to be corrected is established according to the set of sixth training samples. A correction parameter model is established according to the set of sixth training samples. The fourth concentration prediction model for the tissue element is obtained according to the tissue element concentration prediction model to be corrected and the correction parameter model.

When the fourth predetermined condition is met, a fourth target concentration of the detected tissue element is obtained, an output light intensity corresponding to the fourth target concentration is obtained, and the current interference parameter value of each interference parameter in the plurality of interference parameters is obtained. The fourth concentration prediction model for the tissue element is corrected according to the fourth target concentration, a plurality of interference parameter values, and the output light intensity corresponding to the fourth target concentration, so as to process a new output light intensity and a plurality of new current interference parameter values using the corrected fourth concentration prediction model for the tissue element to obtain a new concentration of the detected tissue element.

When the fifth predetermined condition is met, a new output light intensity and a plurality of new current interference parameter values are processed using a new fourth concentration prediction model for the tissue element to obtain a new concentration of the detected tissue element.

According to embodiments of the present disclosure, each photosensitive surface includes an annular photosensitive surface or a non-annular photosensitive surface, and different photosensitive surfaces have the same shape or different shapes.

According to embodiments of the present disclosure, each photosensitive surface may be made of a photosensitive material. The annular photosensitive surface may avoid a problem of an orientation positioning, and may also achieve a large area design within a small source-detection distance range. It should be noted that the source-detection distance is generally an important physical quantity in the living tissue element measurement, and it is very meaningful to achieve a larger area design within a small source-detection distance.

According to embodiments of the present disclosure, in some cases, using a non-annular photosensitive surface has the following beneficial effects.

In a first aspect, due to the influence of the measurement region on the measurement result, a photosensitive surface arranged in a measurement region conducive to the measurement may generally obtain a better measurement result than a photosensitive surface arranged in a measurement region interfering with the measurement. Therefore, the photosensitive surface may be arranged at an appropriate position according to the tissue structure feature. The non-annular photosensitive surface may easily avoid the measurement region that interferes with the measurement, such as a blood vessel or a wound region. Therefore, a good effect may be achieved by using the non-annular photosensitive surface.

In a second aspect, due to the non-uniformity of the tissue, the same incident light may have different transmission paths in the tissue, and then the exit light exited from different exit positions correspond to different average optical paths. Taking blood glucose as an example of the detected tissue element, a main source of a blood glucose signal is generally the dermis. Therefore, it is required that the exit light is obtained after the incident light is mainly transmitted in the dermis. Accordingly, there are some requirements for the average optical path corresponding to the exit light.

Assuming that an annular photosensitive surface with a corresponding size is designed according to the requirements for the average optical path, it may be considered that the exit light received at different photosensitive positions of the annular photosensitive surface correspond to substantially similar average optical paths and mainly passes through the dermis. The average optical path is within an average optical path range C. In this case, if the skin tissue is uniform, the above conclusion is in line with an actual situation. However, the skin tissue is usually not uniform, and there is a significant difference in the average optical paths corresponding to the exit light received at different photosensitive positions of the same annular photosensitive surface. For example, the exit light received at some photosensitive positions of the annular photosensitive surface corresponds to substantially similar average optical paths, which are within the average optical path range C, while the average optical paths corresponding to the exit light received at the other photosensitive positions of the annular photosensitive surface are quite different from the above, and are not within the average optical path range C. The average optical path of the exit light being within the average optical path range C may indicate that the exit light mainly passes through the dermis, and the average optical path of the exit light being not within the average optical path range C may indicate that the exit light does not mainly pass through the dermis. Since the annular photosensitive surface outputs one output light intensity, the output light intensity obtained using the annular photosensitive surface may have a low signal quality in a case of uneven skin tissue, thereby affecting the reliability of the measurement result.

The non-annular photosensitive surface may be arranged according to the actual situation. For the above example, if the average optical path not within the average optical path range C is within an average optical path range D, then two non-annular photosensitive surfaces may be used. One non-annular photosensitive surface is used to receive the light intensity value of the exit light of which the average optical path is within the average optical path range C, and the other non-annular photosensitive surface is used to receive the light intensity value of the exit light of which the average optical path is within the average optical path range D. The output light intensities of the two non-annular photosensitive surfaces are consistent with the actual situation, which is beneficial to ensure the reliability of the measurement result.

In a third aspect, when the tissue element measurement is performed using the pulse wave-based temporal differential measurement method, it is required to make full use of a pulse signal, that is, to maximize a difference between the light intensity for the contraction period and the light intensity for the relaxation period. However, most of the annular photosensitive surface may not be located above the blood vessel, and an effect of acquiring the pulse signal may be affected, so that the difference between the light intensity for the contraction period and the light intensity for the relaxation period is reduced. Therefore, the difference between the light intensity for the contraction period and the light intensity for the relaxation period obtained by using the annular photosensitive surface is less than the difference between the light intensity for the contraction period and the light intensity for the relaxation period obtained by using the non-annular photosensitive surface.

In a fourth aspect, due to a non-uniformity of the tissue and the influence of the change in the physiological background on the exit light, there may be a difference between the average optical paths of the exit light received at different photosensitive surfaces having a same source-detection distance from the center of the incident light. Therefore, a differential operation may be performed on the output light intensities acquired at different photosensitive surfaces having the same source-detection distance from the center of the incident light for the tissue element measurement. The above may be implemented by the non-annular photosensitive surface, that is, for the same source-detection distance, it is possible to separately arrange at least two non-annular photosensitive surfaces with the center of the incident light as a center, so as to output two output light intensities.

In a fifth aspect, the manufacturing process is not difficult and the manufacturing cost is not high.

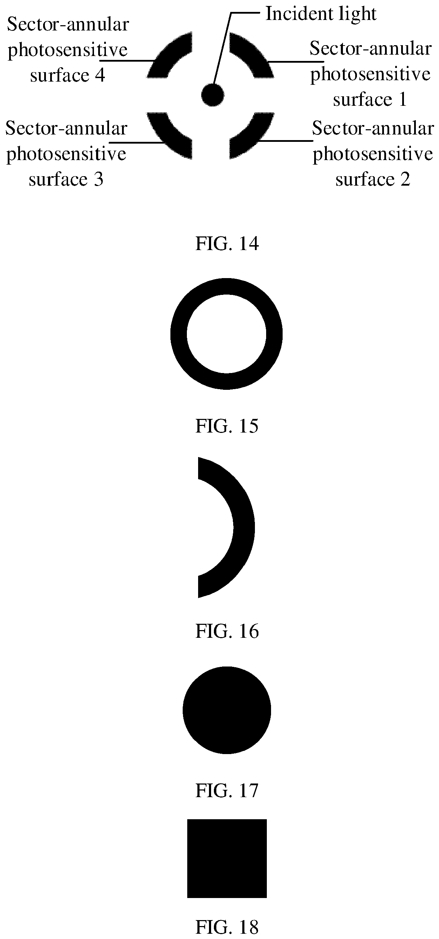

The fourth aspect will be described below with reference to FIG. 14. FIG. 14 schematically shows a schematic diagram of a differential measurement according to embodiments of the present disclosure. As shown in FIG. 14, four sector-annular photosensitive surfaces are shown in FIG. 14, including a sector-annular photosensitive surface 1, a sector-annular photosensitive surface 2, a sector-annular photosensitive surface 3, and a sector-annular photosensitive surface 4. The four sector-annular photosensitive surfaces are separately used, and each sector-annular photosensitive surface has a corresponding output light intensity. Centers of the four sector-annular photosensitive surfaces have a same distance from the center of the incident light, that is, the four sector-annular photosensitive surfaces have the same source-detection distance. Due to the non-uniformity of the tissue, the average optical path corresponding to the exit light received by the sector-annular photosensitive surface 1 is different from the average optical path corresponding to the exit light received by the sector-annular photosensitive surface 2. A differential operation may be performed according to the output light intensity acquired by the sector-annular photosensitive surface 1 and the output light intensity acquired by the sector-annular photosensitive surface 2, so as to perform a differential measurement.

According to embodiments of the present disclosure, the non-annular photosensitive surface includes a sector-annular photosensitive surface, a circular photosensitive surface, a sector photosensitive surface, an elliptical photosensitive surface, or a polygonal photosensitive surface.

According to embodiments of the present disclosure, the polygonal photosensitive surface includes a square photosensitive surface, a rectangular photosensitive surface, or a triangular photosensitive surface.

According to embodiments of the present disclosure, it is possible to design a central angle according to the actual situation, so as to obtain the corresponding sector-annular photosensitive surface, such as a sector-annular photosensitive surface with a central angle of 90°, a sector-annular photosensitive surface with a central angle of 180°, and a sector-annular photosensitive surface with a central angle of 45°.

According to embodiments of the present disclosure, FIG. 15 schematically shows a schematic diagram of an annular photosensitive surface according to embodiments of the present disclosure. FIG. 16 schematically shows a schematic diagram of a sector-annular photosensitive surface according to embodiments of the present disclosure. FIG. 17 schematically shows a schematic diagram of a circular photosensitive surface according to embodiments of the present disclosure. FIG. 18 schematically shows a schematic diagram of a square photosensitive surface according to embodiments of the present disclosure.

According to embodiments of the present disclosure, the homogeneous photosensitive surface includes an annular photosensitive surface or a non-annular photosensitive surface, the homogeneous photosensitive surface includes one or more photosensitive surfaces, and the homogeneous photosensitive surface is used to output one output light intensity.

According to embodiments of the present disclosure, the homogeneous photosensitive surface may be an annular photosensitive surface or a non-annular photosensitive surface, that is, the homogeneous photosensitive surface appears as an annular photosensitive surface or a non-annular photosensitive surface as a whole. According to the number of photosensitive surfaces included in the homogeneous photosensitive surface, it may be determined whether the whole shape is formed by a single photosensitive surface or a combination of a plurality of photosensitive surfaces. Each photosensitive surface in the homogeneous photosensitive surface may be an annular photosensitive surface or a non-annular photosensitive surface.

According to embodiments of the present disclosure, the homogeneous photosensitive surface being an annular photosensitive surface may include that: the homogeneous photosensitive surface is an independent annular photosensitive surface in a case that the homogeneous photosensitive surface includes one photosensitive surface; or the homogeneous photosensitive surface is an annular photosensitive surface formed by combining a plurality of photosensitive surfaces in a case that the homogeneous photosensitive surface includes the plurality of photosensitive surfaces. The homogeneous photosensitive surface being a non-annular photosensitive surface may include that: the homogeneous photosensitive surface is an independent non-annular photosensitive surface in a case that the homogeneous photosensitive surface includes one photosensitive surface; or the homogeneous photosensitive surface is a non-annular photosensitive surface formed by combining a plurality of photosensitive surfaces in a case that the homogeneous photosensitive surface includes the plurality of photosensitive surfaces.

According to embodiments of the present disclosure, the combined plurality of photosensitive surfaces are closely arranged to ensure as much as possible that there is no gap between adjacent photosensitive surfaces. At present, circular photosensitive surfaces or quadrangular photosensitive surfaces are commonly used with low difficulty in manufacturing process and low production cost, while photosensitive surfaces of other shapes usually need to be customized with high difficulty in manufacturing process and high production cost. Therefore, in a case of limited production cost, a combination method may be used to combine a plurality of circular photosensitive surfaces and/or a plurality of square photosensitive surfaces to form a homogeneous photosensitive surface with other shapes. The quadrangular photosensitive surface may include a square one and a rectangular one.

In addition, the production cost of the photosensitive surface is also related to the area of the photosensitive surface. Generally, the larger the area of the photosensitive surface, the higher the production cost. If a large-area photosensitive surface is required and there are currently a plurality of small-area photosensitive surfaces, the plurality of small-area photosensitive surfaces may be combined to obtain a large-area photosensitive surface in order to reduce the production cost.

According to embodiments of the present disclosure, when it is determined that a distance between the homogeneous photosensitive surface and a target site is greater than or equal to a second distance threshold, the homogeneous photosensitive surface includes an annular photosensitive surface, a sector-annular photosensitive surface, a sector photosensitive surface, a circular photosensitive surface, or a square photosensitive surface.

According to embodiments of the present disclosure, when it is determined that the distance between the homogeneous photosensitive surface and the target site is greater than or equal to the second distance threshold, a photosensitive surface with an appropriate shape may be selected according to the actual jitter of the exit light, so as to minimize the adverse influence of the jitter on the measurement.

The target site may be a site where the jitter occurs. The pulse beat is one of sources of jitter, and the pulse beat is related to a blood vessel. Therefore, the target site may be a blood vessel. Generally, a jitter distribution of the exit light close to the blood vessel has a particular directionality, while a jitter distribution of the exit light far away from the blood vessel is uniform and has no directionality.

If the homogeneous photosensitive surface is far away from the target site (such as the target blood vessel), the jitter distribution of the exit light may be uniform. In this case, an annular photosensitive surface, a sector-annular photosensitive surface, a sector photosensitive surface, a circular photosensitive surface or a square photosensitive surface may be selected. The homogeneous photosensitive surface being far away from the target site may be understood as that the distance between each photosensitive surface in the homogeneous photosensitive surface and the target site is greater than or equal to the second distance threshold. The distance between each photosensitive surface in the homogeneous photosensitive surface and the target site being greater than or equal to the second distance threshold may include that a distance between an edge of the photosensitive surface closest to the target site in the homogeneous photosensitive surface and the target site is greater than or equal to the second distance threshold value, or the homogeneous photosensitive surface is in non-contact with the target site and the distance between a center of the photosensitive surface closest to the target site in the homogeneous photosensitive surface and the target site is greater than or equal to the second distance threshold value.

In a case that the homogeneous photosensitive surface is far away from the target site, if the average optical path of the exit light received at different photosensitive positions of each photosensitive surface in the homogeneous photosensitive surface is less than or equal to an optical path threshold, it may indicate that the jitter of the exit light is affected by a size of the optical path. The larger the average optical path of the exit light, the more obvious the jitter of the exit light, or otherwise, the less obvious the jitter of the exit light. In this case, it may be designed that the farther away from the center of the incident light, the longer the arc length. Then, it is possible to select an annular photosensitive surface, a sector-annular photosensitive surface, or a sector photosensitive surface.

In a case that the homogeneous photosensitive surface is far away from the target site, if the average optical path of the exit light received at different photosensitive positions of each photosensitive surface in the homogeneous photosensitive surface is greater than the optical path threshold, it may indicate that the jitter of the exit light is almost independent of the size of the optical path. In this case, it is possible to select a circular photosensitive surface or a square photosensitive surface.

According to embodiments of the present disclosure, in a case that the homogeneous photosensitive surface is a sector-annular photosensitive surface, if the homogeneous photosensitive surface includes one photosensitive surface, the sector-annular photosensitive surface is an independent sector-annular photosensitive surface; if the homogeneous photosensitive surface includes a plurality of photosensitive surfaces, the sector-annular photosensitive surface is a photosensitive surface formed by combining the plurality of photosensitive surfaces. Similarly, for the case that the homogeneous photosensitive surface includes an annular photosensitive surface, a circular photosensitive surface, a square photosensitive surface or a sector photosensitive surface, the homogeneous photosensitive surface may be an independently formed homogeneous photosensitive surface or a combined homogeneous photosensitive surface.

It should be noted that at present, circular photosensitive surfaces or quadrangular photosensitive surfaces are commonly used with low difficulty in manufacturing process and low production cost, while photosensitive surfaces of other shapes usually need to be customized with high difficulty in manufacturing process and high production cost. Therefore, in a case of limited production cost, the homogeneous photosensitive surface may include a circular photosensitive surface or a quadrangular photosensitive surface if it is determined that the distance between the homogeneous photosensitive surface and the target site is greater than or equal to the second distance threshold.

According to embodiments of the present disclosure, when it is determined that the distance between the homogeneous photosensitive surface and the target site is less than or equal to a third distance threshold, the shape of the homogeneous photosensitive surface is determined according to the jitter distribution of the exit light.

According to embodiments of the present disclosure, if the homogeneous photosensitive surface is close to the target site (such as the target blood vessel), it may indicate that the jitter distribution of the exit light has a particular directionality. In this case, the shape of the homogeneous photosensitive surface may be determined according to the jitter distribution of the exit light. Optionally, the shape of the homogeneous photosensitive surface is a similar pattern to the jitter distribution of the exit light. In an example, if the jitter distribution of the exit light is an ellipse, the homogeneous photosensitive surface may be designed as an elliptical photosensitive surface. Alternatively, if the jitter distribution of the exit light is a rectangle, the homogeneous photosensitive surface may be designed as a rectangular photosensitive surface. Alternatively, if the jitter distribution of the exit light is a rhombus, the homogeneous photosensitive surface may be designed as a rhombic photosensitive surface.

According to embodiments of the present disclosure, the jitter distribution of the exit light may be decomposed into a jitter distribution in a first direction and a jitter distribution in a second direction perpendicular to the first direction. A ratio of a length of the homogeneous photosensitive surface in the first direction to a length of the homogeneous photosensitive surface in the second direction is determined according to a ratio of a jitter amplitude of the exit light in the first direction to a jitter amplitude of the exit light in the second direction. The exit light has a maximum jitter amplitude in the first direction.

According to embodiments of the present disclosure, if the jitter distribution of the exit light includes jitter distributions in two mutually perpendicular directions that are obtained by decomposing the jitter distribution of the exit light into the two mutually perpendicular directions which are called the first direction and the second direction respectively, and the exit light has the maximum jitter amplitude in the first direction, then the ratio of the length of the homogeneous photosensitive surface in the first direction to the length of the homogeneous photosensitive surface in the second direction may be determined according to the ratio of the jitter amplitude of the exit light in the first direction to the jitter amplitude of the exit light in the second direction, so that the ratio of the length of the homogeneous photosensitive surface in the first direction to the length of the homogeneous photosensitive surface in the second direction is greater than or equal to the ratio of the jitter amplitude of the exit light in the first direction to the jitter amplitude of the exit light in the second direction.

In an example, if the first direction and the second direction are a Y-axis direction and an X-axis direction in a rectangular coordinate system respectively, then a ratio of the jitter amplitude of the exit light in the Y-axis direction to the jitter amplitude of the exit light in the X-axis direction may be represented by $\dfrac{V_y}{V_x}$ , and a ratio of the length of the homogeneous photosensitive surface in the Y-axis direction to the length of the homogeneous photosensitive surface in the X-axis direction may be represented by $\dfrac{dy}{dx}$ , $\dfrac{dy}{dx} \geqslant \dfrac{V_y}{V_x}$ .

According to embodiments of the present disclosure, the homogeneous photosensitive surface includes a rectangular photosensitive surface or an elliptical photosensitive surface. A ratio of a length to a width of the rectangular photosensitive surface is determined according to the ratio of the jitter amplitude of the exit light in the first direction to the jitter amplitude of the exit light in the second direction. A ratio of a major axis to a minor axis of the elliptical photosensitive surface is determined according to the ratio of the jitter amplitude of the exit light in the first direction to the jitter amplitude of the exit light in the second direction.

According to embodiments of the present disclosure, if the distance between the homogeneous photosensitive surface and the target site is less than or equal to the third distance threshold, and the jitter distribution of the exit light is in the first direction and the second direction that are perpendicular to each other, then the homogeneous photosensitive surface may include a rectangular photosensitive surface or an elliptical photosensitive surface. A ratio of a length to a width of the rectangular photosensitive surface is greater than or equal to the ratio of the jitter amplitude of the exit light in the first direction to the jitter amplitude of the exit light in the second direction. A ratio of a major axis to a minor axis of the elliptical photosensitive surface is greater than or equal to the ratio of the jitter amplitude of the exit light in the first direction to the jitter amplitude of the exit light in the second direction.

According to embodiments of the present disclosure, each output light intensity being obtained by processing the light intensity value of the exit light acquired by one or more photosensitive surfaces may include the following operations.

One or more photosensitive surfaces are used in combination to output one output light intensity. Alternatively, each of the one or more photosensitive surfaces is used separately, and the light intensity values of the exit light acquired by the photosensitive surfaces are calculated to obtain one output light intensity.

According to embodiments of the present disclosure, a photosensitive surface for outputting one output light intensity is referred to as a homogeneous photosensitive surface, and the homogeneous photosensitive surface may include one or more photosensitive surfaces. A condition for using different photosensitive surfaces in combination may be that the average optical path of the exit light received by the photosensitive surfaces is within the average optical path range. The average optical path range may be a range greater than or equal to a first average optical path threshold and less than or equal to a second average optical path threshold. The first average optical path threshold and the second average optical path threshold may be determined according to the optical path average value and the optical path change amplitude. The optical path average value is an average value calculated according to the average optical paths of the exit light received at the photosensitive positions of the homogeneous photosensitive surface.

The photosensitive surface is generally used in cooperation with an amplification circuit corresponding to the photosensitive surface, so as to output one light intensity value. In order to cause the homogeneous photosensitive surface to output an accurate output light intensity, it is required that a product of a light response rate of each photosensitive surface in the homogeneous photosensitive surface and an amplification factor of the amplification circuit used in cooperation with the photosensitive surface is a predetermined value. When the product of the light response rate of each photosensitive surface and the amplification factor of the amplification circuit used in cooperation with the photosensitive surface is a same predetermined value, the homogeneous photosensitive surface may output one output light intensity. If the product of the light response rate of a photosensitive surface and the amplification factor of the amplification circuit used in cooperation with the photosensitive surface is a different predetermined value, it is needed to adopt a corresponding method so that the product is the same predetermined value.

The homogeneous photosensitive surface outputting one output light intensity may be achieved by a hardware method or a software method.

In a first method, which is the hardware method, cathodes of different photosensitive surfaces in the homogeneous photosensitive surface may be electrically connected to each other, and anodes of different photosensitive surfaces in the homogeneous photosensitive surface may be electrically connected to each other, that is, a common-cathode and common-anode electrical connection between different photosensitive surfaces may be achieved. In this case, it is equivalent to connecting different photosensitive surfaces in parallel so that one or more photosensitive surfaces are used in combination to output one output light intensity. It should be noted that the light response rates of different photosensitive surfaces need to be as identical as possible to obtain an accurate output light intensity.

In a second method, which is the software method, the cathodes of different photosensitive surfaces in the homogeneous photosensitive surface are not connected to each other, and the anodes of different photosensitive surfaces in the homogeneous photosensitive surface are not connected to each other, that is, each photosensitive surface is used separately to output one light intensity value. After the light intensity value corresponding to each photosensitive surface is obtained, a weight summation may be performed on the light intensity values of the photosensitive surfaces in the homogeneous photosensitive surface by using a corresponding algorithm, so as to obtain one output light intensity.

Alternatively, the output light intensity corresponding to the homogeneous photosensitive surface may be determined by Equation (2) and Equation (3):

$$I = \sum_{i=1}^{N} \alpha_i I_i \qquad (2);$$

$$\alpha_i = \frac{H}{\beta_i \gamma_i} \qquad (3),$$

where $I$ represents the output light intensity corresponding to the homogeneous photosensitive surface, $I_i$ represents the light intensity value corresponding to the photosensitive surface $i$, $i \in \{1,2,......, N\text{-}1, N\}$, $N$ represents the number of photosensitive surfaces included in the homogeneous photosensitive surface, $1 \leq N \leq M$, $M$ represents a total number of photosensitive surfaces, $\alpha_i$ represents a weighting coefficient corresponding to the photosensitive surface $i$, $H$ represents the predetermined value, $\beta_i$ represents the light response rate corresponding to the photosensitive surface $i$, $\gamma_i$ represents the amplification factor of the amplification circuit used in cooperation with the photosensitive surface $i$.

According to embodiments of the present disclosure, determining the concentration of the detected tissue element according to the at least one output light intensity corresponding to the predetermined wavelength may include the following operations.

At least one added light intensity corresponding to the predetermined wavelength is determined, where the added light intensity is obtained by adding a plurality of output light intensities corresponding to the predetermined wavelength. The concentration of the detected tissue element is determined according to the at least one added light intensity corresponding to the predetermined wavelength.

According to embodiments of the present disclosure, when determining the concentration of the detected tissue element, it is possible to flexibly use the obtained data according to the actual situation, such as a small value of a single output light intensity, or a low signal-to-noise ratio of the output light intensity, so as to improve the reliability of the measurement result.

Based on the above, the concentration of the detected tissue element may be determined according to the at least one added light intensity corresponding to the predetermined wavelength. The added light intensity may be obtained by adding a plurality of output light intensities. The output light intensity is a light intensity of diffused light (that is, the exit light).

According to embodiments of the present disclosure, the predetermined wavelength is a wavelength sensitive to the detected tissue element.

According to embodiments of the present disclosure, in order to improve the reliability of the measurement result, the predetermined wavelength may be a wavelength sensitive to the detected tissue element. In an example, the predetermined wavelength may be 1550 nm or 1609 nm.

According to embodiments of the present disclosure, the temperature of the measurement region is maintained within a predetermined temperature range during the process of measuring the tissue element.

According to embodiments of the present disclosure, since a change in the temperature of the measurement region may affect the reliability of the measurement result, in order to ensure the reliability of the measurement result as much as possible, the temperature of the measurement region may be controlled to be maintained within the predetermined temperature range based on a temperature control method.

According to embodiments of the present disclosure, the photosensitive surface is obtained by providing a mask on an initial photosensitive surface, and a light transmittance of the mask is less than or equal to a light transmittance threshold.

According to embodiments of the present disclosure, a shape of the mask is determined according to the shape of the jitter distribution of the exit light.

According to embodiments of the present disclosure, circular photosensitive surfaces or quadrangular photosensitive surfaces are commonly used with a low difficulty in manufacturing process and a low production cost, while photosensitive surfaces of other shapes usually need to be customized with a high difficulty in manufacturing process and a high production cost. Therefore, in a case of limited production cost, it is possible to adopt a method of providing a mask on the initial photosensitive surface. A part of the initial photosensitive surface that is blocked by the mask is difficult to receive a light intensity value because the light transmittance of the mask is less than or equal to the light transmittance threshold.

Based on the above, the shape and the position of the mask may be determined according to actually desired shape and area, so as to obtain a photosensitive surface with a predetermined shape and area. The actually required shape and area may be determined according to the jitter distribution of the exit light.

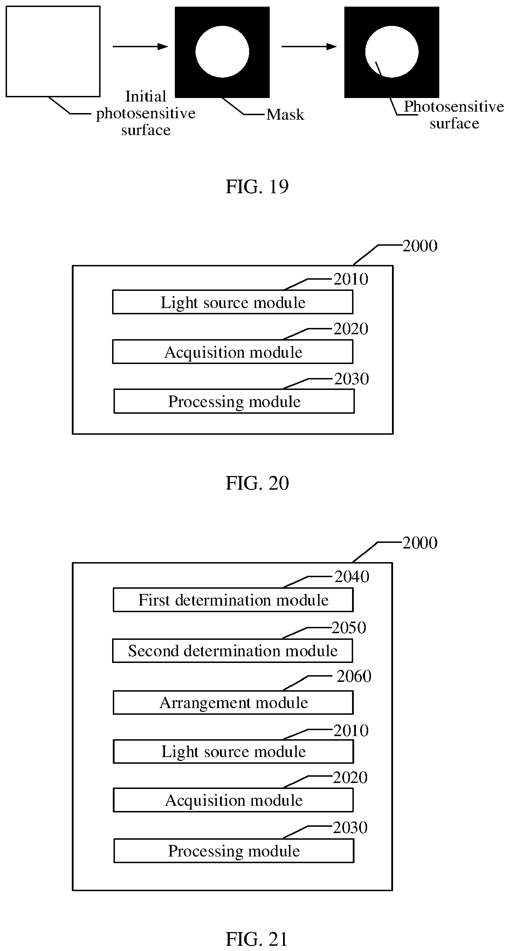

In an example, FIG. 19 schematically shows a schematic diagram of providing a mask on an initial photosensitive surface to obtain a photosensitive surface according to embodiments of the present disclosure. As shown in FIG. 19, the initial photosensitive surface is a square photosensitive surface, and the photosensitive surface is a circular photosensitive surface.

According to embodiments of the present disclosure, the light spot irradiated to the measurement region by the incident light has a uniform intensity distribution.

According to embodiments of the present disclosure, in order to relax the requirement for the detected object when performing the tissue element measurement so as to ensure the reliability of the measurement result better, it is possible to adopt a method of ensuring a uniform intensity distribution of the light spot irradiated to the measurement region by the incident light. Furthermore, the more uniform the intensity distribution of the light spot irradiated to the measurement region by the incident light, the lower the requirement for the reproducibility of the controllable measurement condition, and the better the effect of reducing the influence of the uncontrollable measurement condition on the measurement result by using the differential measurement method, thereby ensuring the reliability of the measurement result better. Moreover, when a measure to obtain a uniform intensity distribution of the light spot irradiated to the measurement region by the incident light is adopted, a light energy of the incident light may be attenuated to a certain extent. However, the tissue element measurement requires that the light energy of the incident light may not be too small. Therefore, it is needed to ensure a minimum attenuation of the light energy of the incident light while ensuring the uniform intensity distribution of the light spot irradiated to the measurement region by the incident light. In addition, if the incident light is transmitted by an optical fiber, the uniform intensity distribution of the light spot irradiated to the measurement region by the incident light may also reduce an adverse influence of an optical fiber jitter on the measurement result.

According to embodiments of the present disclosure, an area of the light spot irradiated to the measurement region by the incident light is greater than or equal to a light spot area threshold.

According to embodiments of the present disclosure, in order to relax the requirement for the detected object when performing the tissue element measurement so as to ensure the reliability of the measurement result better, it is possible to make the area of the light spot irradiated to the measurement region by the incident light greater than or equal to the light spot area threshold. Furthermore, within a certain range, the greater the area of the light spot irradiated to the measurement region by the incident light, the lower the requirement for the reproducibility of the controllable measurement condition, and the better the effect of reducing the influence of the uncontrollable measurement condition on the measurement result by using the differential measurement method, thereby ensuring the reliability of the measurement result better. The light spot area threshold may be determined according to actual situation, and is not specifically limited here. In addition, if the incident light is transmitted by an optical fiber, the area of the light spot irradiated to the measurement region by the incident light that is greater than or equal to the light spot area threshold may reduce the adverse influence of the optical fiber jitter on the measurement result.

It should be noted that in order to improve the reliability of the measurement result, it is required to ensure three aspects as much as possible, including: a device of measuring the tissue element having an ability to sense an expected change in the concentration of the tissue element; reducing the influence of the uncontrollable measurement condition on the measurement result; and controlling the controllable measurement condition. The technical solutions provided by embodiments of the present disclosure may ensure the above three aspects.

For the aspect of the device of measuring the tissue element having an ability to sense an expected change in the concentration of the tissue element, a large-area photosensitive surface is used to achieve a high stability and efficiency of receiving the exit light. For the aspect of reducing the influence of the uncontrollable measurement condition on the measurement result, it is achieved by adopting a differential measurement method. For the aspect of controlling the controllable measurement condition, it is achieved by adopting an effective control method.

FIG. 20 schematically shows a block diagram of a device of measuring a tissue element according to embodiments of the present disclosure.

As shown in FIG. 20, a device 2000 of measuring a tissue element includes a light source module 2010, an acquisition module 2020, and a processing module 2030.

The light source module 2010 is used to irradiate a measurement region with incident light having a single predetermined wavelength. Each beam of incident light passes through the measurement region to form at least one beam of exit light exited from at least one exit position, and the number of incident positions of the incident light is at least one.

The acquisition module 2020 includes M photosensitive surfaces, and each photosensitive surface may acquire a light intensity value of the exit light exited from the exit position within a predetermined anti-jitter range corresponding to the photosensitive surface. The acquisition module 2020 is used to obtain a light intensity value corresponding to each beam of exit light acquired by the M photosensitive surfaces, so as to obtain T output light intensities. Each output light intensity is obtained by processing the light intensity value of the exit light acquired by one or more photosensitive surfaces, where $1 \leq T \leq M$.

The processing module 2030 is used to determine a concentration of the detected tissue element according to at least one output light intensity corresponding to the predetermined wavelength.

According to the technical solutions of embodiments of the present disclosure, the photosensitive surface may acquire the light intensity value of the exit light exited from the exit position within the corresponding predetermined anti-jitter range. In the photosensitive surface with the above-mentioned characteristics, a ratio of the area that may stably receive the exit light to the area of the photosensitive surface is increased, so that the stability of receiving the exit light is improved, the adverse influence of the change in the intensity distribution of the exit light caused by the jitter is reduced, and the reliability of the measurement result is improved. Moreover, a real signal of the detected tissue element may be directly obtained by performing the tissue element measurement using the single predetermined wavelength combined with the photosensitive surface with the above-mentioned characteristics. When the single predetermined wavelength is used for the tissue element measurement, the volume and structural complexity of the light source module may be reduced, so that the volume and structural complexity of the device and the apparatus may be reduced to facilitate a portable measurement. The capacity requirement for the power module may also be reduced, so that the production cost may be reduced. In addition, the amount of data processing may also be reduced.

According to embodiments of the present disclosure, a ratio of an average optical path of the exit light received by each photosensitive surface in the target tissue layer to a total optical path is greater than or equal to a ratio threshold. The total optical path is a total distance that the exit light travels within the measurement region.

According to embodiments of the present disclosure, a total area of the homogeneous photosensitive surface is determined according to the tissue structure feature in the measurement region. The homogeneous photosensitive surface includes one or more photosensitive surfaces, and the homogeneous photosensitive surface is used to output one output light intensity.

According to embodiments of the present disclosure, a ratio of an area of each photosensitive surface to a circumference of the photosensitive surface is greater than or equal to a ratio threshold.

According to embodiments of the present disclosure, the ratio threshold is greater than or equal to 0.04 mm.

According to embodiments of the present disclosure, the photosensitive surface is in contact or non-contact with the surface of the measurement region.

According to embodiments of the present disclosure, a distance between the photosensitive surface and the surface of the measurement region is less than or equal to a first distance threshold, and an efficiency of the photosensitive surface receiving the exit light is greater than or equal to an efficiency threshold.

As shown in FIG. 21, according to embodiments of the present disclosure, the device 2000 of measuring the tissue element further includes a first determination module 2040, a second determination module 2050, and an arrangement module 2060.

The first determination module 2040 is used to determine a positioning feature. The second determination module 2050 is used to determine a measurement region according to the positioning feature, where the measurement region is a region that meets a reproducibility of a controllable measurement condition. The arrangement module 2060 is used to arrange a measurement probe at a position corresponding to the measurement region. The measurement probe includes M photosensitive surfaces.

According to embodiments of the present disclosure, the positioning feature includes a first posture positioning feature and a region positioning feature.

The second determination module includes a first adjustment unit and a first determination unit.

The first adjustment unit is used to adjust a current measurement posture of the detected object to a target measurement posture according to the first posture positioning feature. The target measurement posture is a measurement posture that meets the reproducibility of the controllable measurement condition. The first determination unit is used to determine the measurement region according to the region positioning feature when the current measurement posture is the target measurement posture.

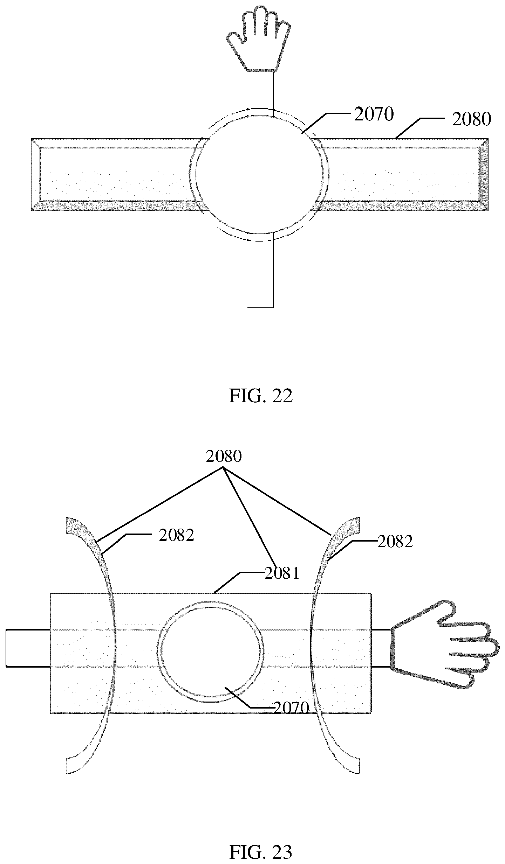

As shown in FIG. 22, according to embodiments of the present disclosure, the device 2000 of measuring the tissue element further includes a fixing portion 2080 used to arrange the measurement probe 2070 at the position corresponding to the measurement region. The fixing portion 2080 is integrated with, partially separated from or completely separated from the measurement probe 2070.

According to embodiments of the present disclosure, the fixing portion and the measurement probe shown in FIG. 22 may be integrated or separated.

As shown in FIG. 23, according to embodiments of the present disclosure, the fixing portion 2080 includes a fixing seat 2081 and a first fitting part 2082.

The first fitting part 2082 is used to arrange the fixing seat 2081 at the position corresponding to the measurement region. The fixing seat 2081 is used to fix the measurement probe 2070. According to embodiments of the present disclosure, a hardness of the first fitting part 2082 includes a first hardness and a second hardness. The first hardness is less than the second hardness. The first hardness is a corresponding hardness in a process of fixing the fixing seat 2081 by the first fitting part 2082, and the second hardness is a corresponding hardness after the fixing seat 2081 is fixed by the first fitting part 2082.

According to embodiments of the present disclosure, in order to enable the first fitting part 2082 to fix the fixing seat 2081, the first fitting part 2082 is required to be rigid. Furthermore, in order to minimize an influence generated when fixing the fixing seat 2081 by the first fitting part 2082, the first fitting part 2082 is required to have a certain flexibility. Therefore, a requirement is put forward on the hardness of the first fitting part 2081.

In order to solve the above problems, it is possible to adopt a method of changing the hardness of the first fitting part 2082, that is, the hardness of the first fitting part 2082 includes the first hardness and the second hardness. The first hardness represents the corresponding hardness in the process of fixing the fixing seat 2081 by the first fitting part 2082, the second hardness represents the corresponding hardness after the fixing seat 2081 is fixed by the first fitting part 2082, and the first hardness is less than the second hardness. In this way, it may not only ensure that the first fitting part achieves the fixation function, but also minimize the influence generated when fixing the fixing seat 2081 by the first fitting part 2082.

According to embodiments of the present disclosure, the first fitting part 2082 includes a first Velcro or a first elastic belt.

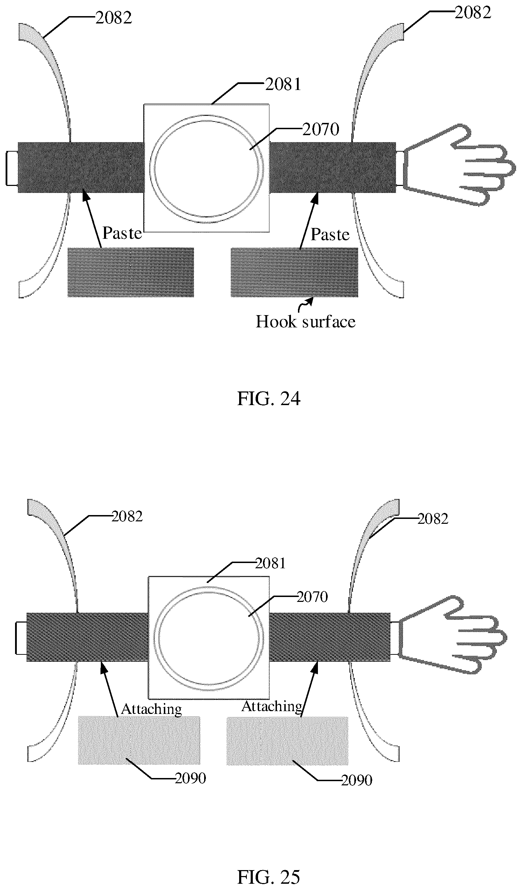

In an example, FIG. 24 schematically shows a schematic diagram of the first fitting part according to embodiments of the present disclosure. The first fitting part 2082 shown in FIG. 24 is a Velcro. A material of a matte surface of the Velcro is very soft, so that the influence generated when fixing the fixing seat 2081 by the first fitting part 2082 may be reduced. At this time, the hardness of the first fitting part 2082 is the first hardness. Meanwhile, in order to enable the first fitting part 2082 to achieve the fixation function, after the fixing seat 2081 is fixed by the first fitting part 2082, a hook surface may be pasted on the matte surface to increase the hardness of the first fitting part 2082. At this time, the hardness of the first fitting part 2082 is the second hardness.

According to embodiments of the present disclosure, the corresponding hardness in the process of fixing the fixing seat 2081 by the first fitting part 2082 is the first hardness, which may reduce the influence generated when fixing the fixing seat 2081 by the first fitting part 2082. Therefore, it may be ensured as much as possible that the skin state of the skin at the measurement region meets a first predetermined condition in the process of arranging the fixing seat at the position corresponding to the measurement region by the first fitting part 2082.

According to embodiments of the present disclosure, the hardness of the first fitting part 2082 is greater than or equal to a first hardness threshold and less than or equal to a second hardness threshold.

According to embodiments of the present disclosure, in order to meet the hardness requirement of the first fitting part 2082, in addition to the method described above, it is also possible to adopt a method of manufacturing the first fitting part 2082 using a material having a hardness greater than or equal to the first hardness threshold and less than or equal to the second hardness threshold, which may also enable the first fitting part 2082 to fix the fixing seat 2081 while minimizing the influence generated when fixing the fixing seat 2081 by the first fitting part 2082. It should be noted that the first hardness threshold and the second hardness threshold may be determined according to actual situations, and are not specifically limited here.

As shown in FIG. 25, according to embodiments of the present disclosure, the device 2000 of measuring the tissue element may further include a first magnetic portion 2090. The first fitting part 2082 is entirely or partially a metal hinge, and the first magnetic portion 2090 fits with the first fitting part 2082 to fix the fixing seat 2081.

According to embodiments of the present disclosure, in order to meet the hardness requirement of the first fitting part 2082, in addition to the method described above, it is also possible to adopt a method of designing the first fitting part 2082 to be entirely or partially a metal hinge, which may also enable the first fitting part 2082 to fix the fixing seat 2081 while minimizing the influence generated when fixing the fixing seat 2081 by the first fitting part 2082.

The fixation function may be achieved as follows. After the first fitting part 2082 completes the fixing of the fixing seat 2081, the first magnetic portion 2090 may be attached to the first fitting part 2082, so that the first magnetic portion 2090 fits with the first fitting part 2082 to fix the fixing seat 2081, thereby achieving the fixation function. Referring to FIG. 25, FIG. 25 schematically shows a schematic diagram of another first fitting part according to embodiments of the present disclosure. The first fitting part 2082 shown in FIG. 25 is entirely a metal hinge. The first magnetic portion 2090 may be attached to the first fitting part 2082 after the first fitting part 2082 completes the fixing of the fixing seat 2081. The first magnetic portion 2090 may be a miniature electromagnet.

In addition, as the metal hinge is a ferromagnetic metal and a metal is easy to absorb heat, a direct contact between the metal hinge and the skin may produce a great influence on a skin temperature. In order to avoid an influence of a heat absorption of the metal on the skin temperature, it is possible to place a thermal insulator under the metal hinge. Optionally, the thermal insulator may be flannelette.

The above may be achieved because a good flexibility of the metal hinge may reduce the influence generated when fixing the fixing seat 2081 by the first fitting part 2082. Moreover, after the first fitting part 2082 completes the fixing of the fixing seat 2081, as the first magnetic portion 2090 is attached to the first fitting part 2082, a cooperation of the two makes the first fitting part 2082 become harder, so that the fixation function may be achieved.

It should be noted that the first fitting part 2082 is entirely or partially a metal hinge, and a good flexibility of the metal hinge may reduce the influence generated when fixing the fixing seat 2081 by the first fitting part 2082. Therefore, it may be ensured as much as possible that the skin state of the skin at the measurement region meets the first predetermined condition in the process of arranging the fixing seat 2081 at the position corresponding to the measurement region by the first fitting part 2082.

According to embodiments of the present disclosure, a surface of the first fitting part 2082 is provided with a hole.

According to embodiments of the present disclosure, the measurement probe 2070 is fixed on the fixing seat 2081 in at least one manner selected from: the measurement probe 2070 being fixed on the fixing seat 2081 by an adhesive tape; the measurement probe 2070 being fixed on the fixing seat 2081 by a fastener; the measurement probe 2070 being fixed on the fixing seat 2081 by a magnetic force; or a friction coefficient between the measurement probe 2070 and the fixing seat 2081 being greater than or equal to a friction coefficient threshold.

According to embodiments of the present disclosure, in order to fix the measurement probe 2070 on the fixing seat 2081 and ensure that the measurement probe 2070 does not move on the fixing seat 2081, at least one of the following methods may be adopted.

In a first method, the measurement probe 2070 may be fixed on the fixing seat 2081 by an adhesive tape. In a second method, the measurement probe 2070 may be fixed on the fixing seat 2081 by a fastener. In a third method, the measurement probe 2070 may be fixed on the fixing seat 2081 by a magnetic force. In a fourth method, the friction coefficient between the measurement probe 2070 and the fixing seat 2081 may be greater than or equal to the friction coefficient threshold. Optionally, a material of the fixing seat 2081 includes rubber, aluminum, or plastic.

According to embodiments of the present disclosure, the fixing portion 2080 includes a second fitting part.

The second fitting part is used to arrange the measurement probe 2070 at a position corresponding to the measurement region.

According to embodiments of the present disclosure, a hardness of the second fitting part includes a third hardness and a fourth hardness. The third hardness is less than the fourth hardness. The third hardness is a corresponding hardness in a process of fixing the measurement probe 2070 by the second fitting part, and the fourth hardness is a corresponding hardness after the measurement probe 2070 is fixed by the second fitting part.

According to embodiments of the present disclosure, the second fitting part includes a second Velcro or a second elastic belt.

According to embodiments of the present disclosure, the hardness of the second fitting part is greater than or equal to a third hardness threshold and less than or equal to a fourth hardness threshold.

According to embodiments of the present disclosure, the device 2000 of measuring the tissue element further includes a second magnetic portion, the second fitting part is entirely or partially a metal hinge, and the second magnetic portion fits with the second fitting part to fix the measurement probe 2070.

According to embodiments of the present disclosure, a surface of the second fitting part is provided with a hole.

According to embodiments of the present disclosure, for the relevant description of the second fitting part, reference may be made to the above description of the first fitting part 2082, and details will not be repeated here. A difference is that the second fitting part is used to fix the measurement probe 2070.

According to embodiments of the present disclosure, the region positioning feature is arranged on at least one of the measurement probe 2070, the fixing portion 2080, or the detected object.

The first determination unit is used to obtain a first projection feature. When it is determined that the region positioning feature is not matched with the first projection feature, the position of the measurement probe 2070 and/or the fixing portion 2080 is adjusted until the region positioning feature is matched with the first projection feature. When it is determined that the region positioning feature is matched with the first projection feature, a region corresponding to the measurement probe 2070 and/or the fixing portion 2080 is determined as the measurement region.

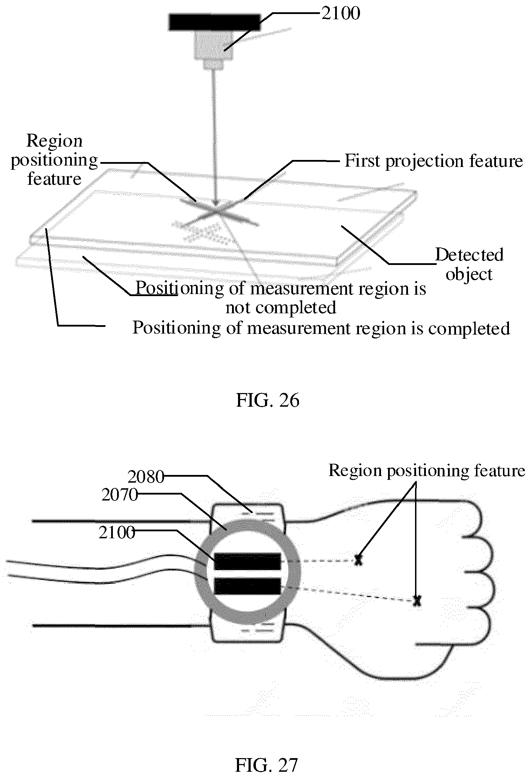

As shown in FIG. 26 and FIG. 27, according to embodiments of the present disclosure, the device 2000 of measuring the tissue element further includes a region positioning portion 2100 arranged on the detected object, the measurement probe 2070, the fixing portion 2080 or other objects. The region positioning portion 2100 is used to project the first projection feature.

According to embodiments of the present disclosure, when it is determined that the region positioning portion 2100 is arranged on the measurement probe 2070, the region positioning feature is not arranged on the measurement probe 2070. When it is determined that the region positioning portion 2100 is arranged on the fixing portion 2080, the region positioning feature is not arranged on the fixing portion 2080.

According to embodiments of the present disclosure, FIG. 26 schematically shows a schematic diagram of a region positioning portion according to embodiments of the present disclosure. The measurement probe 2070 and the fixing portion 2080 are not shown in FIG. 26. The region positioning portion 2100 is used to project the first projection feature, which is a cross light spot. The region positioning feature is a cross marker.

FIG. 27 schematically shows a schematic diagram of another region positioning portion according to embodiments of the present disclosure. As shown in FIG. 27, the region positioning portion 2100 is integrated with the measurement probe 2070 and the fixing portion 2080, and the region positioning feature is arranged on a back of a hand of the detected object. The region positioning portion 2100 is used to project the first projection feature, and the first projection feature is a cross light spot.

According to embodiments of the present disclosure, the region positioning portion 2100 includes a first laser.

According to embodiments of the present disclosure, the first laser may project a light spot having a predetermined shape, so as to form the first projection feature.

According to embodiments of the present disclosure, the first determination unit is used to obtain a first target image, and obtain a first template image, where the first template image includes the region positioning feature. When it is determined that the first target image is not matched with the first template image, the position of the measurement probe 2070 and/or the fixing portion 2080 is adjusted to obtain a new first target image until the new first target image is matched with the first template image. When it is determined that the first target image is matched with the first template image, the region corresponding to the measurement probe 2070 and/or the fixing portion 2080 is determined as the measurement region.

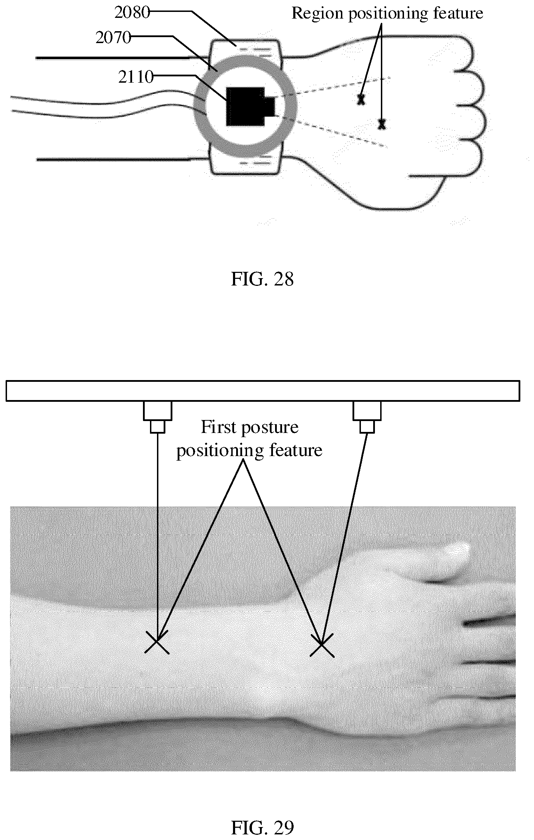

As shown in FIG. 28, according to embodiments of the present disclosure, the device 2000 of measuring the tissue element further includes a first image acquisition portion 2110 arranged on the detected object, the measurement probe 2070, the fixing portion 2080 or other objects. The first image acquisition portion 2110 is used to acquire the first target image.

According to embodiments of the present disclosure, FIG. 28 schematically shows a schematic diagram of a first image acquisition portion according to embodiments of the present disclosure. As shown in FIG. 28, the first image acquisition portion 2110 is integrated with the measurement probe 2070 and the fixing portion 2080, and the region positioning feature is arranged on the back of the hand of the detected object. The first image acquisition portion 2110 is used to acquire the first target image. The first image acquisition portion 2110 may be an image sensor.

According to embodiments of the present disclosure, the first determination unit is used to obtain a second target image, where the second target image includes the region positioning feature. When it is determined that the position of the region positioning feature in the second target image is not the first predetermined position, the position of the measurement probe 2070 and/or the fixing portion 2080 is adjusted to obtain a new second target image until the position of the region positioning feature in the second target image is the first predetermined position. When it is determined that the position of the region positioning feature in the new second target image is the first predetermined position, the region corresponding to the measurement probe 2070 and/or the fixing portion 2080 is determined as the measurement region.

According to embodiments of the present disclosure, the device 2000 of measuring the tissue element further includes a second image acquisition portion arranged on the detected object, the measurement probe 2070, the fixing unit 2080 or other objects. The second image acquisition portion is used to acquire the second target image.

According to embodiments of the present disclosure, the second image acquisition portion is the same as or different from the first image acquisition portion 2110.

According to embodiments of the present disclosure, when it is determined that the second image acquisition portion is arranged on the measurement probe 2070, the region positioning feature is not arranged on the measurement probe 2070. When it is determined that the second image acquisition portion is arranged on the fixing portion 2080, the region positioning feature is not arranged on the fixing portion 2080.

According to embodiments of the present disclosure, the first adjustment unit is used to obtain a second projection feature. When it is determined that the first posture positioning feature is not matched with the second projection feature, the current measurement posture is adjusted until the first posture positioning feature is matched with the second projection feature. When it is determined that the first posture positioning feature is matched with the second projection feature, it is determined that the current measurement posture is the target measurement posture.

As shown in FIG. 29, according to embodiments of the present disclosure, the device 2000 of measuring the tissue element further includes a first posture positioning portion 2120 arranged on the detected object, the measurement probe 2070, the fixing portion 2080 or other objects. The first posture positioning portion 2120 is used to project the second projection feature.

According to embodiments of the present disclosure, when it is determined that the first posture positioning portion 2120 is arranged on the measurement probe 2070, the first posture positioning feature is not arranged on the measurement probe 2070. When it is determined that the first posture positioning portion 2120 is arranged on the fixing portion 2080, the first posture positioning feature is not arranged on the fixing portion 2080.

According to embodiments of the present disclosure, FIG. 29 schematically shows a schematic diagram of a first posture positioning portion according to embodiments of the present disclosure. The measurement probe 2070 and the fixing portion 2080 are not shown in FIG. 29. The first posture positioning portion 2120 is used to project the second projection feature, which is a cross light spot. The first posture positioning feature is a cross marker.

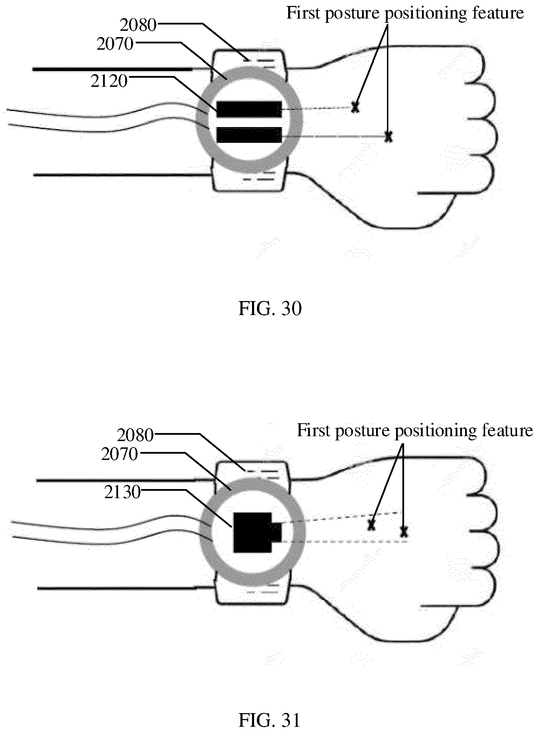

FIG. 30 schematically shows a schematic diagram of another first posture positioning portion according to embodiments of the present disclosure. As shown in FIG. 30, the first posture positioning portion 2120 is integrated with the measurement probe 2070 and the fixing portion 2080, and the first posture positioning feature is arranged on the back of the hand of the detected object. The first posture positioning portion 2120 is used to project the second projection feature, which is a cross light spot.

According to embodiments of the present disclosure, the first posture positioning portion 2120 includes a second laser.

According to embodiments of the present disclosure, the second laser may project a light spot having a predetermined shape, so as to form the second projection feature.

According to embodiments of the present disclosure, the first adjustment unit is used to obtain a third target image, and obtain a second template image, where the second template image includes the first posture positioning feature. When it is determined that the third target image is not matched with the second template image, the current measurement posture is adjusted to obtain a new third target image until the new third target image is matched with the second template image. When it is determined that the new third target image is matched with the second template image, it is determined that the current measurement posture is the target measurement posture.

As shown in FIG. 31, according to embodiments of the present disclosure, the device 2000 of measuring the tissue element further includes a third image acquisition portion 2130 arranged on the detected object, the measurement probe 2070, the fixing portion 2080 or other objects. The third image acquisition portion 2130 is used to acquire the third target image.

According to embodiments of the present disclosure, FIG. 31 schematically shows a schematic diagram of a third image acquisition portion according to embodiments of the present disclosure. As shown in FIG. 31, the third image acquisition portion 2130 is integrated with the measurement probe 2070 and the fixing portion 2080, and the first posture positioning feature is arranged on the back of the hand of the detected object. The third image acquisition portion 2130 is used to acquire the third target image. The third image acquisition portion 2130 may be an image sensor.

According to embodiments of the present disclosure, the third image acquisition portion 2130, the first image acquisition portion 2110 and the second image acquisition portion may be different, partially the same, or completely the same.

According to embodiments of the present disclosure, the first adjustment unit is used to obtain a fourth target image, where the fourth target image includes the first posture positioning feature. When it is determined that the position of the first posture positioning feature in the fourth target image is not the second predetermined position, the current measurement posture is adjusted to obtain a new fourth target image until the position of the first posture positioning feature in the new fourth target image is the second predetermined position. When it is determined that the position of the first posture positioning feature in the new fourth target image is the second predetermined position, it is determined that the current measurement posture is the target measurement posture.

According to embodiments of the present disclosure, the device 2000 of measuring the tissue element further includes a fourth image acquisition portion arranged on the detected object, the measurement probe 2070, the fixing portion 2080 or other objects. The fourth image acquisition portion is used to acquire the fourth target image.

According to embodiments of the present disclosure, the fourth image acquisition portion, the third image acquisition portion 2130, the first image acquisition portion 2110 and the second image acquisition portion may be different, partially the same, or completely the same.

According to embodiments of the present disclosure, when it is determined that the fourth image acquisition portion is arranged on the measurement probe 2070, the first posture positioning feature is not arranged on the measurement probe 2070. When it is determined that the fourth image acquisition portion is arranged on the fixing portion 2080, the first posture positioning feature is not arranged on the fixing portion 2080.

According to embodiments of the present disclosure, the device 2000 of measuring the tissue element further includes a third determination module and an adjustment module.

The third determination module is used to determine a second posture positioning feature in response to the current measurement posture being not the target measurement posture if the measurement probe 2070 is arranged at the position corresponding to the measurement region. The adjustment module is used to adjust the current measurement posture to the target measurement posture according to the second posture positioning feature.

According to embodiments of the present disclosure, the second posture positioning feature is arranged on at least one of the measurement probe 2070, the fixing portion 2080, or the detected object.

The adjustment module includes a first obtaining unit, a second adjustment unit, and a second determination unit.

The first obtaining unit is used to obtain a third projection feature. The second adjustment unit is used to adjust, when it is determined that the second posture positioning feature is not matched with the third projection feature, the current measurement posture until the second posture positioning feature is matched with the third projection feature. The second determination unit is used to determine that the current measurement posture is the target measurement posture when it is determined that the second posture positioning feature is matched with the third projection feature.

According to embodiments of the present disclosure, the device 2000 of measuring the tissue element further includes a second posture positioning portion arranged on the detected object, the measurement probe 2070, the fixing portion 2080 or other objects. The second posture positioning portion is used to project the third projection feature.

According to embodiments of the present disclosure, when it is determined that the second posture positioning portion is arranged on the measurement probe 2070, the second posture positioning feature is not arranged on the measurement probe 2070 and the fixing portion 2080. When it is determined that the second posture positioning portion is arranged on the fixing portion 2080, the second posture positioning feature is not arranged on the measurement probe 2070 and the fixing portion 2080.

According to embodiments of the present disclosure, the second posture positioning portion is the same as or different from the first posture positioning portion 2120.

According to embodiments of the present disclosure, the second posture positioning portion includes a third laser.

According to embodiments of the present disclosure, the third laser may project a light spot having a predetermined shape, so as to form the third projection feature.

According to embodiments of the present disclosure, the adjustment module includes a second obtaining unit, a third obtaining unit, a third adjustment unit, and a third determination unit.

The second obtaining unit is used to obtain a fifth target image. The third obtaining unit is used to obtain a third template image, where the third template image includes the second posture positioning feature. The third adjustment unit is used to adjust, when it is determined that the fifth target image is not matched with the third template image, the current measurement posture to obtain a new fifth target image until the new fifth target image is matched with the third template image. The third determination unit is used to determine that the current measurement posture is the target measurement posture when it is determined that the new fifth target image is matched with the third template image.

According to embodiments of the present disclosure, the device of measuring the tissue element further includes a fifth image acquisition portion arranged on the detected object, the measurement probe 2070, the fixing portion 2080 or other objects. The fifth image acquisition portion is used to acquire the fifth target image.

According to embodiments of the present disclosure, the adjustment module includes a fourth obtaining unit, a fourth adjustment unit, and a fourth determination unit.

The fourth obtaining unit is used to obtain a sixth target image, where the sixth target image includes the second posture positioning feature. The fourth adjustment unit is used to adjust, when it is determined that the position of the second posture positioning feature in the sixth target image is not a third predetermined position, the current measurement posture to obtain a new sixth target image until the position of the second posture positioning feature in the new sixth target image is the third predetermined position. The fourth determination unit is used to determine that the current measurement posture is the target measurement posture when it is determined that the position of the second posture positioning feature in the new sixth target image is the third predetermined position.

According to embodiments of the present disclosure, the device 2000 of measuring the tissue element further includes a sixth image acquisition portion arranged on the detected object, the measurement probe 2070, the fixing portion 2080 or other objects. The sixth image acquisition portion is used to acquire the sixth target image.

According to embodiments of the present disclosure, when it is determined that the sixth image acquisition portion is arranged on the measurement probe 2070, the second posture positioning feature is not arranged on the measurement probe 2070 and the fixing portion 2080. When it is determined that the sixth image acquisition portion is arranged on the fixing portion 2080, the second posture positioning feature is not arranged on the measurement probe 2070 and the fixing portion 2080.

According to embodiments of the present disclosure, the sixth image acquisition portion, the fifth image acquisition portion, the fourth image acquisition portion, the third image acquisition portion 2130, the first image acquisition portion 2110 and the second image acquisition portion may be different, partially the same, or completely the same.

According to embodiments of the present disclosure, when the measurement region and the measurement posture are positioned using an optical method, the region positioning portion 2100, the first posture positioning portion 2120 and the second posture positioning portion may be all the same, partially the same, or completely different. The above-mentioned partially the same means that two of the above three structures are the same. If the three structures are all the same, it may indicate that a same structure may be used to generate the first projection feature, the second projection feature and the third projection feature. The above method may simplify the complexity of the positioning structure.

When the measurement region and the measurement posture are positioned using an image matching method, the first image acquisition portion 2110, the third image acquisition portion 2130 and the fifth image acquisition portion may be all the same, partially the same or completely different. The above-mentioned partially the same means that two of the above three structures are the same. If the three structures are all the same, it may indicate that a same structure may be used to generate the first target image, the third target image and the fifth target image. The above method may simplify the complexity of the positioning structure.

When the measurement region and the measurement posture are positioned using an imaging method, the second image acquisition portion, the fourth image acquisition portion and the sixth image acquisition portion may be all the same, partially the same or completely different. The above-mentioned partially the same means that two of the above three structures are the same. If the three structures are all the same, it may indicate that a same structure may be used to generate the second target image, the fourth target image and the sixth target image. The above method may simplify the complexity of the positioning structure.

A description will be given below by taking the optical method as an example.

The following description is for a case that the region positioning portion 2100, the first posture positioning portion 2120 and the second posture positioning portion are the same structure, the second posture positioning feature is completely identical to the region positioning feature and is partially identical to the first posture positioning feature, and the measurement region is a forearm extensor side.

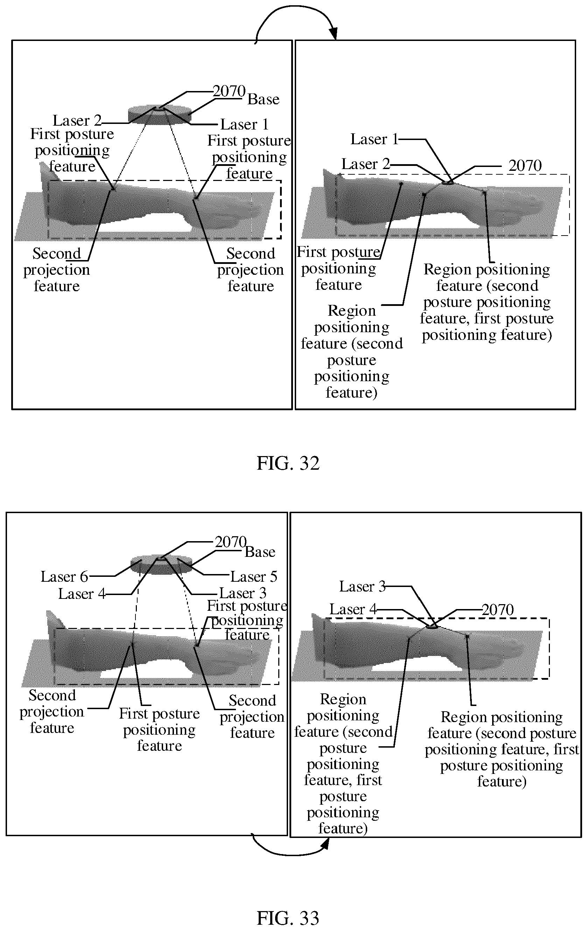

FIG. 32 schematically shows a schematic diagram of positioning the measurement posture and the measurement region according to embodiments of the present disclosure. As shown in FIG. 32, the region positioning portion 2100, the first posture positioning portion 2120 and the second posture positioning portion all include a laser 1 and a laser 2. The laser 1 and the laser 2 are arranged on the measurement probe 2070.

When a first positioning of the measurement posture is performed, the measurement probe 2070 is arranged on a base, and the position of the measurement probe 2070 is fixed before the first positioning of measurement posture is completed. The current measurement posture is adjusted according to the first posture positioning feature and the second projection feature until the first posture positioning feature is matched with the second projection feature. When the two are matched, it may indicate that the first positioning of the measurement posture is completed.

When the positioning of the measurement region is performed, the measurement probe 2070 is arranged on the detected object. The position of the measurement probe 2070 is adjusted according to the region positioning feature and the first projection feature until the region positioning feature is matched with the first projection feature. When the two are matched, it may indicate that the positioning of the measurement region is completed.

After the measurement probe 2070 is arranged on the detected object, if the current measurement posture is not the target posture, it is needed to perform a second positioning of the measurement posture before the measurement is performed. The current measurement posture is adjusted according to the second posture positioning feature and the third projection feature until the second posture positioning feature is matched with the third projection feature. When the two are matched, it may indicate that the second positioning of the measurement posture is completed.

The following description is for a case that the region positioning portion 2100, the first posture positioning portion 2120 and the second posture positioning portion are the same structure, the region positioning feature is completely identical to the second posture positioning feature and partially identical to the first posture positioning feature, and the measurement region is the forearm extensor side.

FIG. 33 schematically shows another schematic diagram of positioning the measurement posture and the measurement region according to embodiments of the present disclosure. As shown in FIG. 33, both the region positioning portion 2100 and the second posture positioning portion include a laser 3 and a laser 4. The first posture positioning portion 2120 includes a laser 5 and a laser 6. The laser 3 and the laser 4 are arranged on the measurement probe 2070. The laser 5 and the laser 6 are arranged on the base.

According to embodiments of the present disclosure, the device of measuring the tissue element further includes a prompt module.

The prompt module is used to generate a prompt information, which is used to prompt a completion of the positioning of the measurement posture and/or the positioning of the measurement region. A form of the prompt information includes at least one of an image, a speech, or a vibration.

According to embodiments of the present disclosure, when it is determined that the fixing seat 2081 is arranged at a position corresponding to the measurement region and the measurement probe 2070 is not arranged on the fixing portion 2080, the measurement probe 2070 is arranged on the fixing seat 2081.

When it is determined that the fixing seat 2081 is not arranged at the position corresponding to the measurement region, the fixing seat 2081 is arranged at the position corresponding to the measurement region by the first fitting part 2082, and the measurement probe 2070 is arranged on the fixing seat 2081.

According to embodiments of the present disclosure, when it is determined that the measurement probe 2070 is not arranged at the position corresponding to the measurement region, the measurement probe 2070 is arranged at the position corresponding to the measurement region by the second fitting part.

According to embodiments of the present disclosure, the processing module 2030 is used to determine a first output light intensity and a second output light intensity from at least two output light intensities corresponding to the predetermined wavelength, and determine the concentration of the detected tissue element according to the first output light intensity and the second output light intensity corresponding to the predetermined wavelength.

According to embodiments of the present disclosure, determining the concentration of the detected tissue element according to the first output light intensity and the second output light intensity corresponding to the predetermined wavelength may include the following operations.

A differential processing is performed on the first output light intensity and the second output light intensity corresponding to the predetermined wavelength to obtain a differential signal. The concentration of the detected tissue element is determined according to the differential signal corresponding to the predetermined wavelength.

Performing the differential processing on the first output light intensity and the second output light intensity corresponding to the predetermined wavelength to obtain the differential signal may include the following operations. The first output light intensity and the second output light intensity corresponding to the predetermined wavelength are processed using a differential circuit, so as to obtain the differential signal.

According to embodiments of the present disclosure, performing the differential processing on the first output light intensity and the second output light intensity corresponding to the predetermined wavelength to obtain the differential signal may include the following operations. The first output light intensity and the second output light intensity corresponding to the predetermined wavelength are processed using a differential algorithm, so as to obtain the differential signal.

According to embodiments of the present disclosure, processing the first output light intensity and the second output light intensity corresponding to the predetermined wavelength using a differential algorithm to obtain the differential signal may include the following operations. A direct differential operation is performed on the first output light intensity and the second output light intensity corresponding to the predetermined wavelength, so as to obtain the differential signal.

According to embodiments of the present disclosure, processing the first output light intensity and the second output light intensity corresponding to the predetermined wavelength by using a differential algorithm to obtain the differential signal may include the following operations. A logarithmic processing is performed on the first output light intensity and the second output light intensity corresponding to the predetermined wavelength, so as to obtain a first logarithmic light intensity and a second logarithmic light intensity. A direct differential operation is performed on the first logarithmic light intensity and the second logarithmic light intensity corresponding to the predetermined wavelength, so as to obtain the differential signal.

According to embodiments of the present disclosure, determining the concentration of the detected tissue element according to the differential signal corresponding to the predetermined wavelength may include the following operations.

The differential signal corresponding to the predetermined wavelength is input into the first concentration prediction model for the tissue element to output the concentration of the detected tissue element.

According to embodiments of the present disclosure, the device 2000 of measuring the tissue element may be further used to perform the following operations.

A set of first training samples is obtained, where the set of first training samples includes a plurality of first training samples, and each first training sample includes a first true concentration of the detected tissue element and a differential signal corresponding to the first true concentration. The first concentration prediction model for the tissue element is established according to the set of first training samples.

According to embodiments of the present disclosure, establishing the first concentration prediction model for the tissue element according to the set of first training samples may include the following operations.

The set of first training samples is pre-processed to obtain a processed set of first training samples. The first concentration prediction model for the tissue element is established according to the processed set of first training samples.

According to embodiments of the present disclosure, the device of measuring the tissue element may be further used to perform the following operations.

When a fourth predetermined condition is met, the first concentration prediction model for the tissue element is corrected to obtain a corrected first concentration prediction model for the tissue element, so as to process a new differential signal using the corrected first concentration prediction model for the tissue element to obtain a new concentration of the detected tissue element.

According to embodiments of the present disclosure, correcting the first concentration prediction model for the tissue element may include the following operations.

A first target concentration of the detected tissue element is obtained. A differential signal corresponding to the first target concentration is obtained. The first concentration prediction model for the tissue element is corrected according to the first target concentration and the differential signal corresponding to the first target concentration.

According to embodiments of the present disclosure, the device of measuring the tissue element may be further used to perform the following operations.

When a fifth predetermined condition is met, a new differential signal is processed using a new first concentration prediction model for the tissue element, so as to obtain a new concentration of the detected tissue element.

According to embodiments of the present disclosure, determining the concentration of the detected tissue element according to the differential signal corresponding to the predetermined wavelength may include the following operations.

A current interference parameter value of each interference parameter of a plurality of interference parameters is obtained. A plurality of current interference parameter values and the differential signal corresponding to the predetermined wavelength are input into a second concentration prediction model for the tissue element to output the concentration of the detected tissue element.

According to embodiments of the present disclosure, the device of measuring the tissue element may be further used to perform the following operations.

A set of second training samples is obtained, where the set of second training samples includes a plurality of second training samples, and each second training sample includes a second true concentration of the detected tissue element and a differential signal corresponding to the second true concentration. A set of third training samples is obtained, where the set of third training samples includes a plurality of third training samples, and each third training sample includes a training interference parameter value of each interference parameter of a plurality of interference parameters and a differential signal corresponding to each training interference parameter value. A tissue element concentration prediction model to be corrected is established according to the set of second training samples. A correction parameter model is established according to the set of third training samples. The second concentration prediction model for the tissue element is obtained according to the tissue element concentration prediction model to be corrected and the correction parameter model.

According to embodiments of the present disclosure, the device of measuring the tissue element may be further used to perform the following operations.

When the fourth predetermined condition is met, the second concentration prediction model for the tissue element is corrected, so as to process a new differential signal and a plurality of new current interference parameter value by using the corrected second concentration prediction model for the tissue element to obtain a new concentration of the detected tissue element.

According to embodiments of the present disclosure, correcting the second concentration prediction model for the tissue element may include the following operations.

A second target concentration of the detected tissue element is obtained. A differential signal corresponding to the second target concentration is obtained. A current interference parameter value of each interference parameter of a plurality of interference parameters is obtained. The second concentration prediction model for the tissue element is corrected according to the second target concentration, the plurality of interference parameter values, and the differential signal corresponding to the second target concentration.

According to embodiments of the present disclosure, the device of measuring the tissue element may be further used to perform the following operations.

When the fifth predetermined condition is met, a new differential signal and a plurality of new current interference parameter values are processed by using a new second concentration prediction model for the tissue element to obtain a new concentration of the detected tissue element.

According to embodiments of the present disclosure, the M photosensitive surfaces include one or more homogeneous photosensitive surfaces corresponding to the predetermined wavelength. The homogeneous photosensitive surface is used to acquire the first output light intensity and/or the second output light intensity corresponding to the predetermined wavelength at different time instants. The first output light intensity and the second output light intensity corresponding to the predetermined wavelength are output light intensities in a same pulsation cycle. The first output light intensity is a light intensity for a contraction period, and the second output light intensity is a light intensity for a relaxation period. The homogeneous photosensitive surface includes one or more photosensitive surfaces. The processing module 2030 is used to determine the concentration of the detected tissue element according to the first output light intensity and the second output light intensity corresponding to the predetermined wavelength.

According to embodiments of the present disclosure, the M photosensitive surfaces include a first homogeneous photosensitive surface and a second homogeneous photosensitive surface corresponding to the predetermined wavelength. The first homogeneous photosensitive surface is used to acquire the first output light intensity corresponding to the predetermined wavelength, and the second homogeneous photosensitive surface is used to acquire the second output light intensity corresponding to the predetermined wavelength. The first homogeneous photosensitive surface includes one or more photosensitive surfaces, and the second homogeneous photosensitive surface includes one or more photosensitive surfaces. The processing module 2030 is used to determine the concentration of the detected tissue element according to the first output light intensity and the second output light intensity corresponding to the predetermined wavelength.

According to embodiments of the present disclosure, the first homogeneous photosensitive surface and the second homogeneous photosensitive surface are the same homogeneous photosensitive surface, and the exit light received by the first homogeneous photosensitive surface and the exit light received by the second homogeneous photosensitive surface are obtained by a transmission of the incident light incident on different incident positions.

According to embodiments of the present disclosure, the first homogeneous photosensitive surface and the second homogeneous photosensitive surface are different homogeneous photosensitive surfaces.

According to embodiments of the present disclosure, the average optical length of the exit light received at different photosensitive positions of each photosensitive surface in the first homogeneous photosensitive surface is within a first average optical path range, the first average optical path range is determined according to the first optical path average value, and the first optical path average value is an average value calculated according to the average optical path of the exit light received at the photosensitive positions of the first homogeneous photosensitive surface. The average optical path of the exit light received at different photosensitive positions of each photosensitive surface in the second homogeneous photosensitive surface is within a second average optical path range, the second average optical path range is determined according to a second optical path average value, and the second optical path average value is an average value calculated according to the average optical path of the exit light received at the photosensitive positions of the second homogeneous photosensitive surface.

According to embodiments of the present disclosure, an absolute value of a difference between the first optical path average value and the second optical path average value is within a first optical path difference range.

According to embodiments of the present disclosure, the first average optical path range is less than or equal to the first optical path difference range, and the second average optical path range is less than or equal to the first optical path difference range.

According to embodiments of the present disclosure, the first optical path difference range is determined according to an optimal differential optical path corresponding to the predetermined wavelength.

According to embodiments of the present disclosure, a source-detection distance of each photosensitive surface in the first homogeneous photosensitive surface corresponding to the predetermined wavelength from the center of the incident light is within a predetermined source-detection distance range corresponding to the predetermined wavelength. The predetermined source-detection distance range is determined according to a source-detection distance of a floating reference position corresponding to the predetermined wavelength from the center of the incident light.

According to embodiments of the present disclosure, the M photosensitive surfaces include a homogeneous photosensitive surface corresponding to the predetermined wavelength. The homogeneous photosensitive surface is used to acquire a third output light intensity corresponding to the predetermined wavelength, and the homogeneous photosensitive surface includes one or more photosensitive surfaces. The processing module 2030 is used to determine the concentration of the detected tissue element according to the third output light intensity corresponding to the predetermined wavelength.

According to embodiments of the present disclosure, a difference between the average optical path of the exit light received at different photosensitive positions of each homogeneous photosensitive surface in the homogeneous photosensitive surface and the optimal optical path corresponding to the predetermined wavelength is within a second optical path difference range.

According to embodiments of the present disclosure, each photosensitive surface includes an annular photosensitive surface or a non-annular photosensitive surface, and different photosensitive surfaces have the same or different shapes.

According to embodiments of the present disclosure, the non-annular photosensitive surface includes a sector-annular photosensitive surface, a circular photosensitive surface, a sector photosensitive surface, an elliptical photosensitive surface, or a polygonal photosensitive surface.

According to embodiments of the present disclosure, the polygonal photosensitive surface includes a square photosensitive surface, a rectangular photosensitive surface, or a triangular photosensitive surface.

According to embodiments of the present disclosure, the homogeneous photosensitive surface includes an annular photosensitive surface or a non-annular photosensitive surface. The homogeneous photosensitive surface includes one or more photosensitive surfaces, and the homogeneous photosensitive surface is used to output one output light intensity.

According to embodiments of the present disclosure, the homogeneous photosensitive surface being an annular photosensitive surface includes that: the homogeneous photosensitive surface is an independent annular photosensitive surface in a case that the homogeneous photosensitive surface includes one photosensitive surface; or the homogeneous photosensitive surface is an annular photosensitive surface formed by combining a plurality of photosensitive surfaces in a case that the homogeneous photosensitive surface includes the plurality of photosensitive surfaces. The homogeneous photosensitive surface being a non-annular photosensitive surface includes that: the homogeneous photosensitive surface is an independent non-annular photosensitive surface in a case that the homogeneous photosensitive surface includes one photosensitive surface; or the homogeneous photosensitive surface is a non-annular photosensitive surface formed by combining a plurality of photosensitive surfaces in a case that the homogeneous photosensitive surface includes the plurality of photosensitive surfaces.

According to embodiments of the present disclosure, when it is determined that the distance between the homogeneous photosensitive surface and the target site is greater than or equal to the second distance threshold, the homogeneous photosensitive surface includes an annular photosensitive surface, a sector-annular photosensitive surface, a sector photosensitive surface, a circular photosensitive surface, or a square photosensitive surface.

According to embodiments of the present disclosure, when it is determined that the distance between the homogeneous photosensitive surface and the target site is less than or equal to the third distance threshold, the shape of the homogeneous photosensitive surface is determined according to the jitter distribution of the exit light.

According to embodiments of the present disclosure, the jitter distribution of the exit light may be decomposed into a jitter distribution in the first direction and a jitter distribution in the second direction perpendicular to the first direction. A ratio of a length of the homogeneous photosensitive surface in the first direction to a length of the homogeneous photosensitive surface in the second direction is determined according to a ratio of a jitter amplitude of the exit light in the first direction to a jitter amplitude of the exit light in the second direction. The exit light has a maximum jitter amplitude in the first direction.

According to embodiments of the present disclosure, the homogeneous photosensitive surface includes a rectangular photosensitive surface or an elliptical photosensitive surface. A ratio of a length to a width of the rectangular photosensitive surface is determined according to the ratio of the jitter amplitude of the exit light in the first direction to the jitter amplitude of the exit light in the second direction. A ratio of a major axis to a minor axis of the elliptical photosensitive surface is determined according to the ratio of the jitter amplitude of the exit light in the first direction to the jitter amplitude of the exit light in the second direction.

According to embodiments of the present disclosure, anodes of different photosensitive surfaces among the M photosensitive surfaces are not electrically connected to each other, anodes of at least two photosensitive surfaces among the M photosensitive surfaces are electrically connected to each other, or anodes of the M photosensitive surfaces are electrically connected to each other.

According to embodiments of the present disclosure, the anodes of different photosensitive surfaces among the M photosensitive surfaces are not electrically connected to each other, the anodes of some photosensitive surfaces are electrically connected to each other, or the anodes of all the photosensitive surfaces are electrically connected to each other.

According to embodiments of the present disclosure, each of the M photosensitive surfaces may be used independently. In this case, the anodes of different photosensitive surfaces among the M photosensitive surfaces are not electrically connected to each other.

Some photosensitive surfaces among the M photosensitive surfaces may be used in combination. In this case, the anodes of the different photosensitive surfaces used in combination are electrically connected to each other.

All the photosensitive surfaces of the M photosensitive surfaces may be used in combination. In this case, the anodes of the different photosensitive surfaces used in combination are electrically connected to each other.

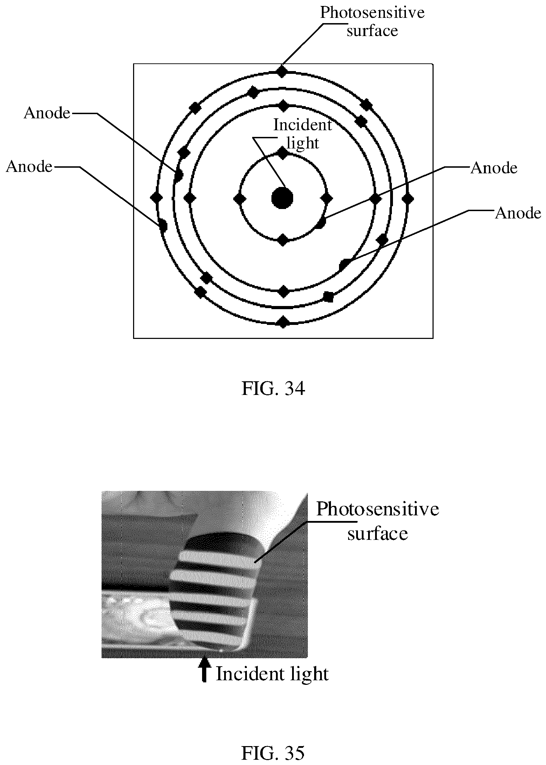

According to embodiments of the present disclosure, FIG. 34 schematically shows a schematic diagram of an electrical connection of the anodes of different photosensitive surfaces according to embodiments of the present disclosure. As shown in FIG. 34, the anodes of all the photosensitive surfaces are electrically connected to each other.

According to embodiments of the present disclosure, different parts of a photosensitive surface may be on a same plane or on different planes.

According to embodiments of the present disclosure, the photosensitive surface may be a planar photosensitive surface or a three-dimensional photosensitive surface. If different parts of the photosensitive surface are on the same plane, the photosensitive surface is a planar photosensitive surface. If different parts of the photosensitive surface are on different planes, the photosensitive surface is a three-dimensional photosensitive surface. Whether to use the planar photosensitive surface or the three-dimensional photosensitive surface may be specifically determined according to actual desires, which is not specifically limited here.

Optionally, for a contact measurement, in order to improve the reliability of the measurement result, it is needed to ensure a good fitting state between a target surface of the photosensitive surface and a skin surface at the measurement region as much as possible. The target surface of the photosensitive surface represents a surface facing the measurement region. A flatness of the skin surface at the measurement region may not be high. If a planar photosensitive surface is used, it may be difficult to achieve a good fitting state between the target surface of the photosensitive surface and the skin surface at the measurement region. The three-dimensional photosensitive surface is a photosensitive surface with different parts on different planes and may be used for the contact measurement. A specific form of the three-dimensional photosensitive surface may be determined according to the tissue structure feature at the measurement region.

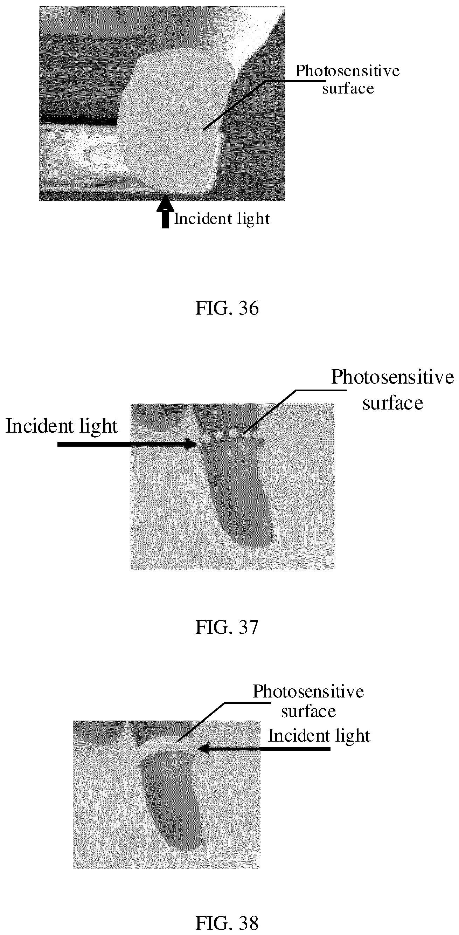

FIG. 35 schematically shows a schematic diagram of a three-dimensional photosensitive surface in a form of a glove according to embodiments of the present disclosure. FIG. 36 schematically shows a schematic diagram of another three-dimensional photosensitive surface in a form of a glove according to embodiments of the present disclosure.

FIG. 37 schematically shows a schematic diagram of a three-dimensional photosensitive surface in a form of a finger ring according to embodiments of the present disclosure. FIG. 38 schematically shows a schematic diagram of another three-dimensional photosensitive surface in a form of a finger ring according to embodiments of the present disclosure. In FIG. 38, $h_1$ and $h_2$ represent distances between different parts of the photosensitive surface and a predetermined plane.

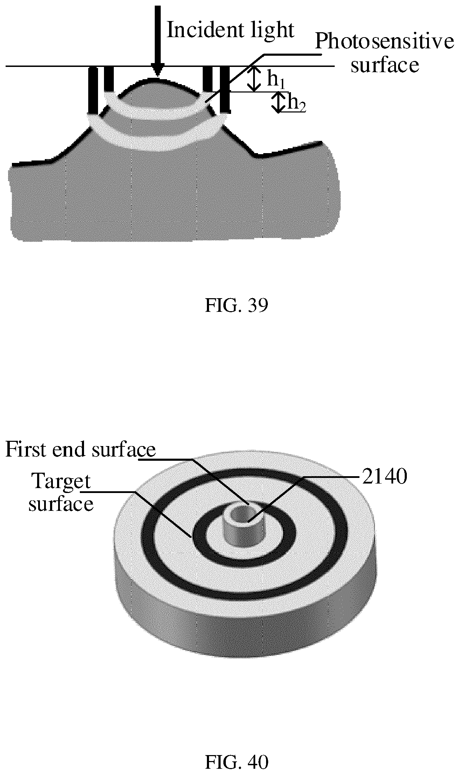

FIG. 39 schematically shows a schematic diagram of a three-dimensional photosensitive surface for an on-arm measurement according to embodiments of the present disclosure. In FIG. 39, the distances between different parts of the photosensitive surface and the predetermined plane may be determined according to a tissue structure feature of the arm.

According to embodiments of the present disclosure, a set of photosensitive surfaces is on a same plane or on different planes, and the set of photosensitive surfaces includes a plurality of photosensitive surfaces.

According to embodiments of the present disclosure, each photosensitive surface included in the set of photosensitive surfaces may be a planar photosensitive surface or a three-dimensional photosensitive surface. If the set of photosensitive surfaces includes a plurality of planar photosensitive surfaces, a photosensitive surface form of three-dimensional photosensitive surface may be presented by the set of photosensitive surfaces by arranging some or all of the plurality of planar photosensitive surfaces on different planes.

It should be noted that a three-dimensional photosensitive surface formed by a plurality of planar photosensitive surfaces may also achieve the above-mentioned effect for the contact measurement, and details will not be repeated here.

According to embodiments of the present disclosure, the predetermined wavelength is a wavelength sensitive to the detected tissue element.

According to embodiments of the present disclosure, the device of measuring the tissue element further includes a temperature control module, which is used to control the temperature of the measurement region to be maintained within a predetermined temperature range during the process of measuring the tissue element.

According to embodiments of the present disclosure, the device of measuring the tissue element further includes a mask arranged on an initial photosensitive surface. A light transmittance of the mask is less than or equal to a light transmittance threshold. The mask is used to be provided on the initial photosensitive surface to obtain the photosensitive surface.

According to embodiments of the present disclosure, a shape of the mask is determined according to the shape of the jitter distribution of the exit light.

As shown in FIG. 40, according to embodiments of the present disclosure, the measurement probe 2070 is provided with a first sleeve 2140. A first end surface of the first sleeve exceeds the target surface of the measurement probe 2070. The first end surface represents an end surface facing the measurement region, and the target surface of the measurement probe 2070 represents a surface facing the measurement region.

According to embodiments of the present disclosure, in order to shield interference light, the first sleeve 2140 may be arranged on the measurement probe 2070 so that the end surface of the first sleeve 2140 facing the measurement region exceeds the target surface of the measurement probe 2070. The interference light may include surface-reflected light and/or diffracted light.

According to embodiments of the present disclosure, a second end surface and/or an inner region of the first sleeve 2140 are/is provided with a scatterer. The first end surface and the second end surface are two opposite end surfaces, and the inner region includes a partial inner region or an entire inner region.

According to embodiments of the present disclosure, in order to make the light spot irradiated to the measurement region by the incident light have a uniform intensity distribution, it is possible to adopt a method of providing a scatterer at a corresponding part of the first sleeve 2140. The scatterer may include sulfuric acid paper, silica gel, or a target mixture. The target mixture may include a mixture of polydimethylsiloxane and titanium dioxide particles.

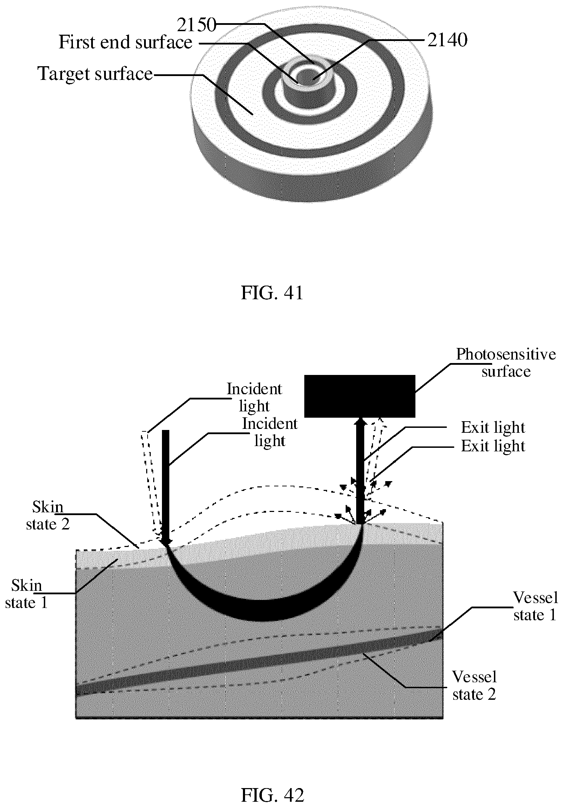

As shown in FIG. 41, according to embodiments of the present disclosure, the device 2000 of measuring the tissue element further includes a second sleeve 2150 arranged outside a target region of the first sleeve 2140. The target region represents a partial region or an entire region of the first sleeve 2140 exceeding the measurement probe 2070.

According to embodiments of the present disclosure, in order to make the light spot irradiated to the measurement region by the incident light as large as possible, it is possible to adopt a method of arranging the second sleeve 2150 outside the target region of the first sleeve 2140.

According to embodiments of the present disclosure, the second sleeve 2150 is provided with a scatterer.

According to embodiments of the present disclosure, if the second sleeve 2150 is provided, it is possible to adopt a method of providing the scatterer at a corresponding part of the second sleeve 2150 in order to make the light spot irradiated to the measurement region by the incident light have a uniform intensity distribution.

According to embodiments of the present disclosure, an inner diameter of the first sleeve 2140 is greater than or equal to an inner diameter threshold.

According to embodiments of the present disclosure, an opening of the first end surface of the first sleeve 2140 is greater than or equal to an opening of the second end surface of the first sleeve 2140.

According to embodiments of the present disclosure, in order to make the light spot irradiated to the measurement region by the incident light as large as possible, it is possible to design that the inner diameter of the first sleeve 2140 is greater than or equal to an inner diameter threshold, and/or the opening of the first end surface of the first sleeve 2140 is greater than or equal to the opening of the second end surface of the first sleeve 2140, that is, the opening of the end surface of the first sleeve 2140 facing the measurement region is greater than or equal to the opening of the end surface of the first sleeve 2140 away from the measurement region.

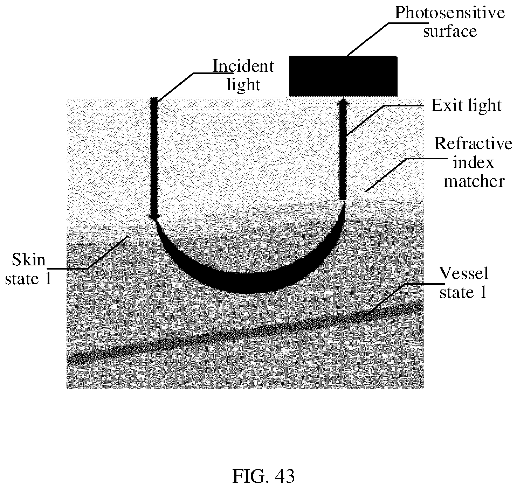

As shown in FIG. 43 to FIG. 44, according to embodiments of the present disclosure, a refractive index matcher is filled between the photosensitive surface and the measurement region.

According to embodiments of the present disclosure, the jitter may cause an unstable skin surface at the measurement region, and then cause a change in an exit angle of the exit light, which may affect the reliability of the measurement result. Therefore, in order to minimize the adverse influences caused by the jitter, it is possible to fill a refractive index matcher between the photosensitive surface and the measurement region to improve the stability and efficiency of the photosensitive surface receiving the exit light.

In an example, a description is given by taking a jitter caused by a pulse beat as an example. The pulse beat may be reflected by a state of a blood vessel. FIG. 42 schematically shows a schematic diagram of the photosensitive surface receiving the exit light in a case of no refractive index matcher being filled according to embodiments of the present disclosure. As shown in FIG. 42, a blood vessel state 1 represents a vasoconstriction state, a blood vessel state 2 represents a vasodilation state, a skin state 1 represents a skin state corresponding to the blood vessel state 1, and a skin state 2 represents a skin state corresponding to the blood vessel state 2. As shown in FIG. 42, the jitter may cause an unstable skin surface at the measurement region, and then cause a change in the exit angle of the exit light.

FIG. 43 schematically shows a schematic diagram of the photosensitive surface receiving the exit light in a case of a refractive index matcher being filled according to embodiments of the present disclosure.

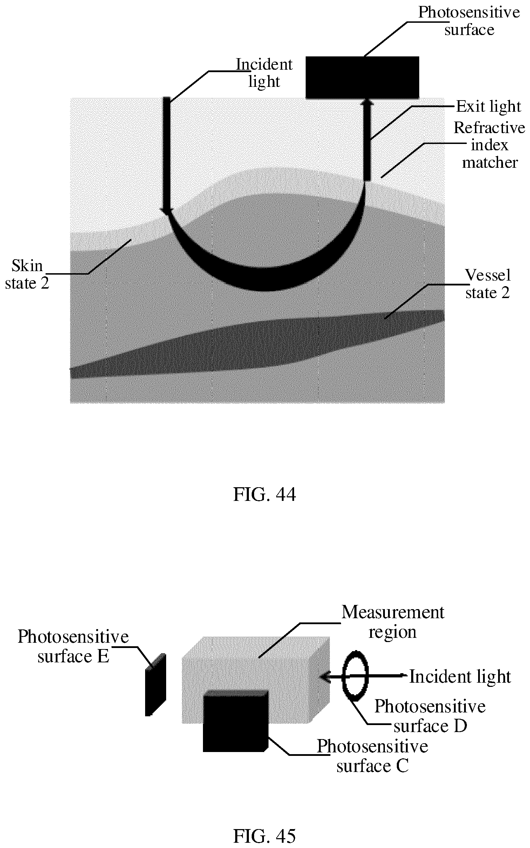

FIG. 44 schematically shows another schematic diagram of the photosensitive surface receiving the exit light in a case of a refractive index matcher being filled according to embodiments of the present disclosure.

As shown in FIG. 43 and FIG. 44, the stability and efficiency of the photosensitive surface receiving the exit light may be improve by filling the refractive index matcher between the photosensitive surface and the measurement region.

According to embodiments of the present disclosure, an angle between each part of the photosensitive surface and a direction of the corresponding incident light is greater than or equal to 0° and less than or equal to 360°.

According to embodiments of the present disclosure, the angle between each part of the photosensitive surface and a direction of the corresponding incident light is greater than or equal to 0° and less than or equal to 360°, so as to achieve a diffusion measurement. According to embodiments of the present disclosure, an appropriate arrangement position of the photosensitive surface may be determined according to a wavelength feature and/or a measurement region feature. The wavelength feature may include a penetration depth of the wavelength, and the measurement region feature may include a thickness of the measurement region. Alternatively, the angle between each part of the photosensitive surface and the direction of the corresponding incident light may generally be set to be a predetermined angle.

In an example, in a case of a large penetration depth of the wavelength and/or a small thickness of the measurement region, the position of the photosensitive surface and the incident position of the corresponding incident light may be arranged on different sides of the measurement region. In a case of a small penetration depth of the wavelength and/or a large thickness of the measurement region, the position of the photosensitive surface and the incident position of the corresponding incident light may be arranged on the same side of the measurement region.

In an example, FIG. 45 schematically shows a schematic diagram of a diffusion measurement according to embodiments of the present disclosure. As shown in FIG. 45, an angle between the photosensitive surface C and the incident light is 90°, the position of the photosensitive surface D and the position of the incident light are arranged on the same side of the measurement region, and the position of the photosensitive surface E and the position of the incident light are arranged on different sides of the measurement region.

Any number of the modules and units according to embodiments of the present disclosure, or at least part of functions of any number of them may be implemented in one module. Any one or more of the modules and units according to embodiments of the present disclosure may be split into a plurality of modules for implementation. Any one or more of the modules and units according to embodiments of the present disclosure may be implemented at least partially as a hardware circuit, such as a field programmable gate array (FPGA), a programmable logic array (PLA), a system on a chip, a system on a substrate, a system on a package, an application specific integrated circuit (ASIC), or may be implemented by hardware or firmware in any other rational manner of integrating or encapsulating the circuit, or may be implemented by any one of three implementation modes of software, hardware and firmware or an appropriate combination thereof. Alternatively, one or more of the modules and units according to embodiments of the present disclosure may be at least partially implemented as a computer program module that may performs the corresponding functions when executed.

For example, any number of the acquisition module and the processing module may be combined into one module/unit for implementation, or any one of the modules/units may be split into a plurality of modules/units. Alternatively, at least part of the functions of one or more of these modules/units may be combined with at least part of the functions of other modules/units and implemented in one module/unit. According to embodiments of the present disclosure, at least one of the acquisition module and the processing module may be implemented at least partially as a hardware circuit, such as a field programmable gate array (FPGA), a programmable logic array (PLA), a system on a chip, a system on a substrate, a system on a package, an application specific integrated circuit (ASIC), or may be implemented by hardware or firmware in any other rational manner of integrating or encapsulating the circuit, or may be implemented by any one of the three implementation modes of software, hardware and firmware or an appropriate combination thereof. Alternatively, at least one of the acquisition module and the processing module may be at least partially implemented as a computer program module that may perform the corresponding functions when executed.

It should be noted that the device of measuring the tissue element in embodiments of the present disclosure corresponds to the description of the method of measuring the tissue element in embodiments of the present disclosure. For the description of the device of measuring the tissue element, reference may be specifically made to the description of the method of measuring the tissue element, which will not be repeated here.

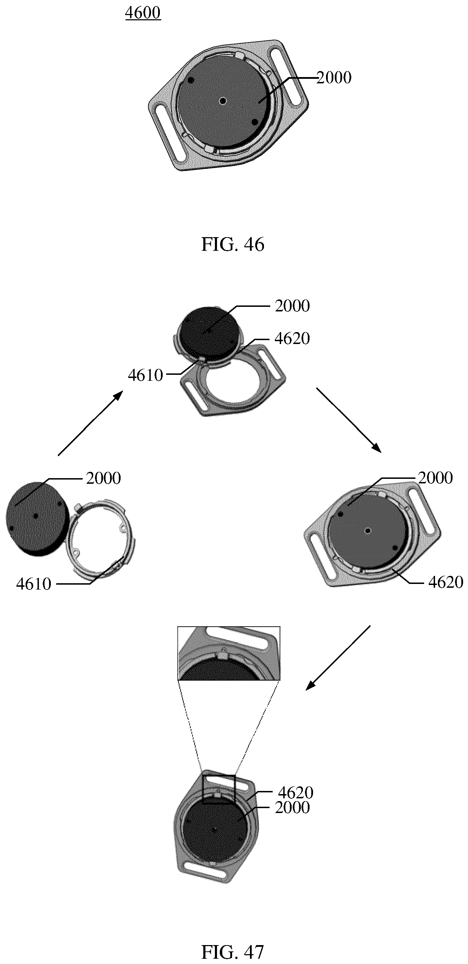

FIG. 46 schematically shows a schematic diagram of a wearable apparatus according to embodiments of the present disclosure. A wearable apparatus 4600 shown in FIG. 46 is just an example, and should not impose any limitation on a function and a scope of use of the present disclosure.

As shown in FIG. 46, the wearable apparatus 4600 includes the device 2000 of measuring the tissue element.

According to the technical solutions of embodiments of the present disclosure, a real signal of the detected tissue element may be directly obtained by performing the tissue element measurement using the single predetermined wavelength combined with the photosensitive surface with the above-mentioned characteristics. When the single predetermined wavelength is used for the tissue element measurement, the volume and structural complexity of the light source module may be reduced, so that the volume and structural complexity of the device and the apparatus may be reduced to facilitate a portable measurement. The capacity requirement for the power module may also be reduced, so that the production cost may be reduced. In addition, the amount of data processing may also be reduced.

As shown in FIG. 47, according to embodiments of the present disclosure, the wearable apparatus 4600 further includes a buckle portion 4610 and a body 4620. The buckle portion 4610 and the body 4620 are used to cooperate to fix the device 2000 of measuring the tissue element.

According to embodiments of the present disclosure, FIG. 47 schematically shows a schematic diagram of an assembly process of the wearable apparatus according to embodiments of the present disclosure.

According to embodiments of the present disclosure, a mass of the wearable apparatus 4600 is less than or equal to a mass threshold, so that a movement mode of the wearable apparatus 4600 is identical to a skin jitter mode at the measurement region.

According to embodiments of the present disclosure, in order to improve the reliability of the measurement result, the wearable apparatus 4600 may have a small mass, so that the wearable apparatus 4600 may follow the skin jitter at the measurement region when the wearable apparatus 4600 is arranged at a position corresponding to the measurement region, that is, the movement mode of the wearable apparatus 4600 may be identical to the skin jitter mode at the measurement region, and then the average optical path of the exit light received by the measurement probe 2070 may be maintained within the predetermined optical path range during the skin jitter process. The average optical path of the exit light received by the measurement probe 2070 may be maintained within the predetermined optical path range during the skin jitter process at the measurement region because a relative position of the measurement probe 2070 at the measurement region may be kept unchanged or substantially unchanged if the wearable apparatus 4600 may follow the skin jitter at the measurement region, and then the measurement probe 2070 may receive the exit light exited from a fixed exit position, where the fixed exit position means an exit position that remains unchanged or substantially unchanged relative to the measurement region. Furthermore, during the skin jitter process at the measurement region, the incident position of the incident light may remain unchanged or substantially unchanged relative to the measurement region. In a case that the incident position of the incident light and the exit position of the exit light are determined, it is possible to ensure as much as possible that the average optical path of the exit light remains unchanged.

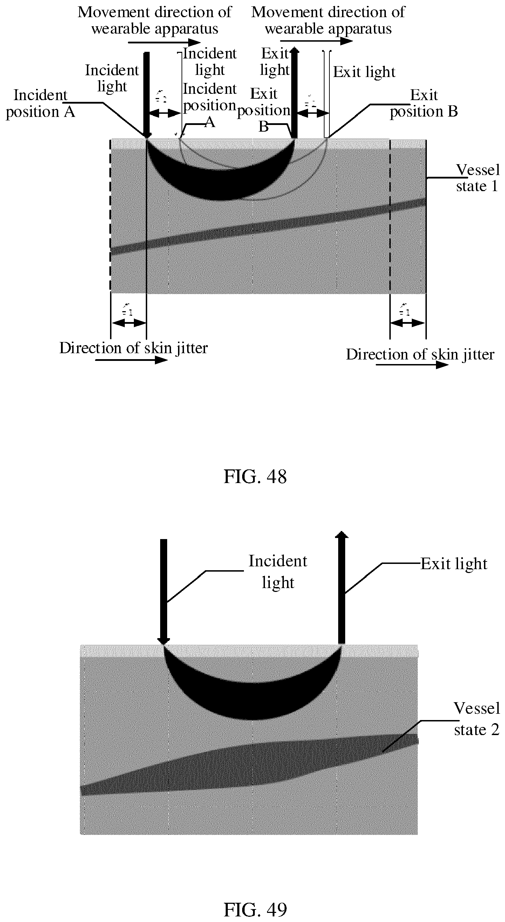

In an example, FIG. 48 schematically shows a schematic diagram of maintaining the average optical path of the exit light received by the measurement probe within a predetermined optical path range during a skin jitter process in a case that a movement mode of the wearable apparatus is identical to a skin jitter mode according to embodiments of the present disclosure. During the skin jitter process, the measurement probe 2070 (not shown in FIG. 48) may stably receive the exit light that is exited from the exit position B of the measurement region after the incident light is incident at the incident position A of the measurement region. A movement amplitude of the skin is represented by $\zeta_1$, and a movement amplitude of the measurement probe 2070 is represented by $\zeta_2$, $\zeta_1 = \zeta_2$.

According to embodiments of the present disclosure, the wearable apparatus 4600 may be used to cause the movement amplitude of the skin at the measurement region to be less than or equal to a movement amplitude threshold.

According to embodiments of the present disclosure, in order to improve the reliability of the measurement result, the wearable apparatus 4600 may have a large mass, so that when the wearable apparatus 4600 is arranged at a position corresponding to the measurement region, it may suppress the skin jitter at the measurement region, that is, the movement amplitude of the skin at the measurement region is less than or equal to a movement amplitude threshold, and then the average optical path of the exit light received by the measurement probe 2070 may be maintained within a predetermined optical path range during the skin jitter process. The average optical path of the exit light received by the measurement probe 2070 may be maintained within the predetermined optical path range during the skin jitter process at the measurement region because a relative position of the measurement probe 2070 at the measurement region may be kept unchanged or substantially unchanged if the wearable apparatus 4600 may suppress the skin jitter at the measurement region, and then the measurement probe 2070 may receive the exit light exited from a fixed exit position. Furthermore, during the skin jitter process at the measurement region, the incident position of the incident light may remain unchanged or substantially unchanged relative to the measurement region. In a case that the incident position of the incident light and the exit position of the exit light are determined, it is possible to ensure as much as possible that the average optical path of the exit light remains unchanged.

In an example, FIG. 49 schematically shows a schematic diagram of maintaining the average optical path of the exit light received by the measurement probe within a predetermined optical path range during a skin jitter process in a case that the wearable apparatus causes a movement amplitude of a skin at the measurement region to be less than or equal to a movement amplitude threshold according to embodiments of the present disclosure. As shown in FIG. 49, the movement amplitude of the skin at the measurement region is close to zero.

According to embodiments of the present disclosure, for the specific description of the device of measuring the tissue element, reference may be made to the corresponding part above, and details will not be repeated here. In addition, the device of measuring the tissue element includes a processor which may perform various appropriate actions and processes according to a program stored in a read only memory (ROM) or a program loaded from a storage portion into a random access memory (RAM). The processor may include, for example, a general-purpose microprocessor (for example, CPU), an instruction set processor and/or a related chipset and/or a special-purpose microprocessor (for example, an application specific integrated circuit (ASIC)), and the like. The processor may further include an on-board memory for caching purposes. The processor may include a single processing unit or a plurality of processing units for performing different actions of the method flow according to embodiments of the present disclosure.

Various programs and data required for the operation of the tissue element measurement device are stored in the RAM. The processor, the ROM and the RAM are connected to each other through a bus. The processor performs various operations of the method flow according to embodiments of the present disclosure by executing the programs in the ROM and/or the RAM. It should be noted that the program may also be stored in one or more memories other than the ROM and the RAM. The processor may also perform various operations of the method flow according to embodiments of the present disclosure by executing the programs stored in the one or more memories.

According to embodiments of the present disclosure, the wearable apparatus may further include an input/output (I/O) interface which is also connected to the bus. The wearable apparatus may further include one or more of the following components connected to the I/O interface: an input part including a keyboard, a mouse, etc.; an output part including a cathode ray tube (CRT), a liquid crystal display (LCD), etc. and a speaker, etc.; a storage part including a hard disk, etc.; and a communication part including a network interface card such as a LAN card, a modem, and the like. The communication part performs communication processing via a network such as the Internet. A drive is also connected to the I/O interface as required. A removable medium, such as a magnetic disk, an optical disk, a magneto-optical disk, a semiconductor memory, and the like, is installed on the drive as required, so that the computer program read therefrom is installed into the storage part.

The present disclosure further provides a computer-readable storage medium, which may be included in the apparatus/device/system described in the above embodiments; or exist alone without being assembled into the apparatus/device/system. The above-mentioned computer-readable storage medium carries one or more programs that perform the methods according to embodiments of the present disclosure when being executed.

According to embodiments of the present disclosure, the computer-readable storage medium may be a non-transitory computer-readable storage medium, for example, may include but not limited to: a portable computer disk, a hard disk, a random access memory (RAM), a read-only memory (ROM), an erasable programmable read-only memory (EPROM) or flash memory, a portable compact disk read-only memory (CD-ROM), an optical storage device, a magnetic storage device, or any suitable combination of the above. In the present disclosure, the computer-readable storage medium may be any tangible medium that contains or stores programs that may be used by or in combination with an instruction execution system, apparatus or device.

For example, according to embodiments of the present disclosure, the computer-readable storage medium may include the above-mentioned ROM and/or RAM and/or one or more memories other than the ROM and RAM.

Embodiments of the present disclosure further include a computer program product, which contains a computer program. The computer program contains program code for performing the method provided by embodiments of the present disclosure.

When the computer program is executed by the processor, the above-mentioned functions defined in the system/device of embodiments of the present disclosure are performed. According to embodiments of the present disclosure, the above-described systems, devices, modules, units, etc. may be implemented by computer program modules.

In an embodiment, the computer program may rely on a tangible storage medium such as an optical storage device and a magnetic storage device. In another embodiment, the computer program may also be transmitted and distributed in the form of signals on a network medium, downloaded and installed through the communication part, and/or installed from the removable medium. The program code contained in the computer program may be transmitted by any suitable medium, including but not limited to a wireless one, a wired one, or any suitable combination of the above.

According to embodiments of the present disclosure, the program code for executing the computer programs provided by embodiments of the present disclosure may be written in any combination of one or more programming languages. In particular, these computing programs may be implemented using high-level procedures and/or object-oriented programming languages, and/or assembly/machine languages. Programming languages include, but are not limited to, Java, C++, Python, "C" language or similar programming languages. The program code may be completely executed on the user computing apparatus, partially executed on the user device, partially executed on the remote computing apparatus, or completely executed on the remote computing apparatus or server. In a case of involving a remote computing apparatus, the remote computing apparatus may be connected to a user computing apparatus through any kind of network, including a local area network (LAN) or a wide area networks (WAN), or may be connected to an external computing apparatus (e.g., through the Internet using an Internet service provider).

A specific example will be illustrated for better understanding of the technical solutions provided in embodiments of the present disclosure. In the example, the detected tissue element is blood glucose, the measurement region is a forearm extensor side of a left arm, and the predetermined wavelength is 1550 nm. That is, through in vivo experiments, it is verified that a real signal of a blood glucose that changes synchronously with a concentration of the blood glucose may be directly obtained by performing a tissue element measurement using the device of measuring the tissue element that uses a single predetermined wavelength combined with a large-area photosensitive surface.

I. Preparation before the experiment

1. Parameters of the measurement probe

The measurement probe is provided with M=4 annular photosensitive surfaces, each annular photosensitive surface has an annular width of 0.2 mm, and the four annular photosensitive surfaces have a same geometric center. The four annular photosensitive surfaces from inside to outside in a radial direction respectively have a first inner diameter, a second inner diameter, a third inner diameter, and a fourth inner diameter, where the first inner diameter is 0.8 mm, the second inner diameter is 3.2 mm, the third inner diameter is 3.8 mm, and the fourth inner diameter is 4.4 mm. The first inner diameter, the second inner diameter, the third inner diameter and the fourth inner diameter all represent diameters.

2. An arrangement of the region positioning feature, the first posture positioning feature and the second posture positioning feature

In this specific example, the first posture positioning feature and the second posture positioning feature are the same, hereinafter referred to as a posture positioning feature.

First, the posture positioning feature is arranged, that is, an arrangement position of the posture positioning feature is determined according to the forearm extensor side and a bone-muscle relationship between the forearm extensor side and a surrounding part. For the forearm extensor side, a change in a wrist state may greatly affect the skin state of the forearm extensor side. In order to improve the positioning accuracy of the measurement posture, it is possible to determine two positions for arranging positioning features on both sides of the wrist respectively. The two positions may be a first predetermined position on the forearm extensor side of the left arm close to an elbow joint and a second predetermined position on a back of a left hand close to the wrist.

In order to reduce the number of posture positioning features required for positioning the measurement posture, a supporting plane is used to support the arm and the palm, and it is required that the arm is tightly attached to the support plane to limit a rotation of the arm during the positioning process. Therefore, in order to accurately position the measurement posture, it is only needed to arrange two posture positioning features at the selected first predetermined position and second predetermined position respectively. The two posture positioning features may be selected from inherent features on the detected object, or may be manually provided. In this specific example, the two posture positioning features are manually provided, which are cross markers.

Second, the region positioning feature is arranged. In order to accurately position the measurement region, a cross marker is arranged on the measurement probe as the region positioning feature.

3. A reproducibility structure for the controllable measurement condition

The positioning of the measurement region and the positioning of the measurement posture are achieved by an optical method, where the positioning of the measurement region and the positioning of the measurement posture are achieved based on a positioning portion. The positioning portion includes a region positioning portion and a posture positioning portion. The posture positioning portion is used to achieve the functions of the first posture positioning portion and the second posture positioning portion, that is, the first posture positioning portion and the second posture positioning portion are the same posture positioning portion. The positioning portion is separated from the measurement probe. Both the region positioning portion and the posture positioning portion adopt a red laser that may project a cross light spot.

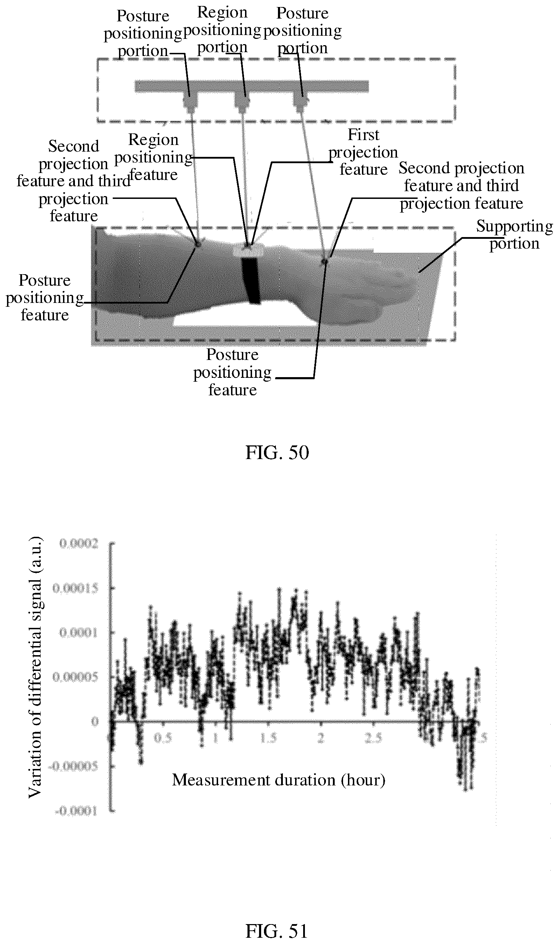

FIG. 50 schematically shows a schematic diagram of synchronously positioning the measurement region and the measurement posture based on an optical method according to embodiments of the present disclosure.

3. A method of adjusting a concentration of a blood glucose

A first method is to orally take a glucose solution. The concentration of the blood glucose is adjusted by an oral glucose tolerance test (OGTT). OGTT generally requires the detected object to orally take a glucose solution containing 75 g of glucose dissolved in 250 ml of water. This method is adopted for a healthy volunteer.

A second method is to take a meal. The concentration of the blood glucose is adjusted by a meal tolerance test (MTT). MTT generally requires the detected object to take a meal with carbohydrates as a main ingredient and a small amount of protein, and drink as few water or drink with high water content as possible, with a total water consumption of less than 50 ml. This method is adopted for a diabetic volunteer.

4. A method of obtaining a true value of the blood glucose

Venous blood was taken from a back of a right hand with a venous indwelling needle and measured by three portable blood glucose meters (GT-1820, Arkray, Japan). An average of the three values of the blood glucose was taken as the true value of the blood glucose.

II. Performance test of the experimental device

A measured signal-to-noise ratio is obtained by measuring a standard reflective plate and the forearm extensor side of the detected object. The measured signal-to-noise ratio is compared with a target signal-to-noise ratio to determine the performance of the experimental device. If the measured signal-to-noise ratio is less than the target signal-to-noise ratio, it may indicate that the experimental device is feasible. The standard reflective plate has a reflectance of 40%, and the light intensity values acquired by the four annular photosensitive surfaces when measuring the reflective plate are similar to those acquired when measuring the tissue element.

For the measurement of the standard reflective plate, a logarithmic differential operation is performed on the output light intensities acquired by the two annular photosensitive surfaces having the third inner diameter and the fourth inner diameter, so as to obtain a differential signal. 480 data are measured per minute, and the measurement is performed for 3.5 hours. A variation of the differential signal during the reflective plate measurement process is calculated based on a differential signal at the beginning of the measurement, and a result is shown in FIG. 51. FIG. 51 schematically shows a schematic diagram of a change in the variation of the differential signal obtained by measuring the standard reflective plate over the measurement duration according to embodiments of the present disclosure.

As shown in FIG. 51, when measuring the reflective plate, the differential signal fluctuates within a range of about 0.0003 a.u., which is equivalent to a change in a concentration of the blood glucose of 4.5 mg/dL.

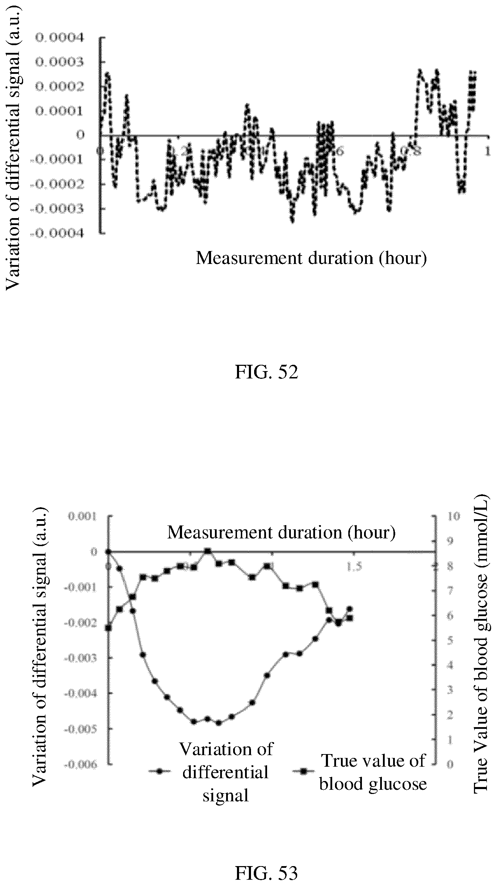

For the forearm extensor side of the detected object, the detected object is required to keep a fasting state during the measurement. The positioning of the measurement region and the positioning of the measurement posture are achieved by an optical method, and the measurement posture is the target measurement posture during the measurement process. A logarithmic differential operation is performed on the output light intensities acquired by the two annular photosensitive surfaces having the third inner diameter and the fourth inner diameter, so as to obtain a differential signal. The variation of the differential signal during the variation process of the differential signal corresponding to a stable concentration of the blood glucose is calculated based on a differential signal at an end of intaking, and a result is shown in FIG. 52. FIG. 52 schematically shows a schematic diagram of a change in the variation of the differential signal obtained by measuring the forearm extensor side of the detected object in a stable state of the concentration of the blood glucose over the measurement duration according to embodiments of the present disclosure.

As shown in FIG. 52, during the fasting measurement process, the differential signal fluctuates within a range of 0.0007 a.u., which is equivalent to a change in a concentration of a blood glucose of 10.5 mg/dL, and meets an accuracy requirement of a clinical blood glucose experiment.

III. Experimental process

1. Single glucose loading experiment

(1) Experimental arrangement

The detected objects include ten healthy volunteers and seven diabetic volunteers, including eleven males and six females, and twenty-seven tests were performed on the detected objects.

An age distribution of the detected objects is that five people are aged 20 to 30, four people are aged 30 to 50, and eight people are aged 50 to 72, where the eight people aged 50 to 72 include six diabetic patients.

Before the experiment, it is required to ensure that the detected objects have fasted for more than ten hours. The left arm of the detected object is placed on a workbench, and the arm may move slightly when no measurement is performed. After the device is warmed up, a continuous optical measurement is performed, and the true value of the blood glucose is also acquired at intervals of five minutes during the optical measurement until the concentration of the blood glucose of the detected object returns to a low level. During the signal acquisition process, the positioning of the measurement posture is performed using an optical method.

(2) Data acquisition

The experiment is divided into a warm-up stage, an intaking stage, and a glucose loading experiment stage. The warm-up stage is the first 0 to 1 hour of the experiment. In this stage, a heat exchange is conducted between the measurement probe and the skin until a thermal equilibrium is reached. It is needed to acquire a fasting blood glucose value in the warm-up stage.

The intaking stage follows the warm-up stage, and generally takes ten minutes. During the intaking stage, the detected object may move slightly. During a period from an end of intaking to an end of the measurement, the arm posture of the detected object may be adjusted to the target measurement posture by an optical method.

The glucose loading experiment stage lasts for 1 to 1.5 hours. In this stage, it is required to measure the true value of the blood glucose every 5 to 10 minutes and record the measurement signal at the same time.

(3) Analysis of results

Upon a data analysis, it is obtained that when the concentration of the blood glucose of the detected object changes by 1 mmol/L, the amplitude of the variation of the average differential signal is about 0.0012 a.u. Moreover, the variation of the differential signal is synchronized with the change in the concentration of the blood glucose, and a correlation coefficient between the two reaches 0.96 at most, with an average of 0.80. A corresponding root mean square error of the concentration of the blood glucose that may be directly resolved is 0.34 mmol/L at least, with an average of 0.82 mmol/L.

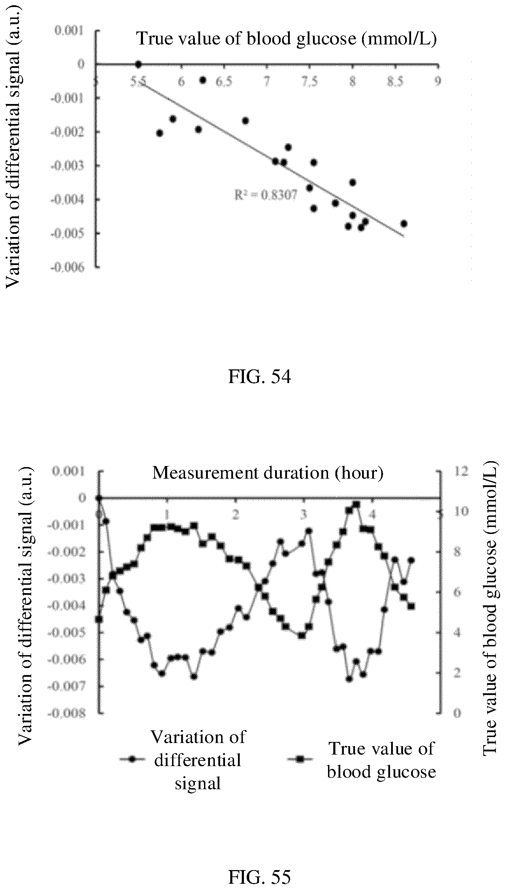

FIG. 53 schematically shows a schematic diagram of a result of the single glucose loading experiment using an OGTT method according to embodiments of the present disclosure. The result in FIG. 53 is for a particular detected object. FIG. 54 schematically shows a schematic diagram of a relationship between the variation of the differential signal and the true value of the blood glucose according to embodiments of the present disclosure.

2. Double glucose loading experiment

(1) Experimental arrangement

The detected object is a 29-year-old male healthy volunteer. A difference from the single glucose loading experiment is that this experiment requires two meals, that is, the experiment continues after the first glucose loading experiment is completed. After the blood glucose has dropped to a low level, it requires a second intaking so that the blood glucose rises. The overall experiment lasts about six hours, and the other arrangements are the same as the single glucose loading experiment.

(2) Analysis of results

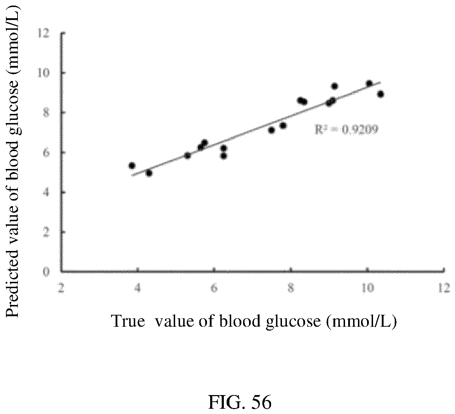

Upon a data analysis, it is proved that the signal of the blood glucose is obtained, and the variation of the differential signal has a good correlation with the concentration of the blood glucose. The result is shown in FIG. 55. FIG. 55 schematically shows a schematic diagram of a result of a double glucose loading experiment using an MTT method according to embodiments of the present disclosure. A blood glucose concentration prediction model established by using the data obtained from the first MTT may be used to directly predict the concentration of the blood glucose in the second MTT, and the root mean square error of the prediction reaches 0.68 mmol/L. Therefore, a direct mutual prediction may be achieved when the same measurement conditions are maintained in two MTTs. The result is shown in FIG. 56. FIG. 56 schematically shows a schematic diagram of a relationship between a predicted value of the blood glucose and a true value of the blood glucose according to embodiments of the present disclosure. The predicted value of the blood glucose in FIG. 56 represents a result of predicting the concentration of the blood glucose in the second MTT by the blood glucose concentration prediction model established using the data obtained in the first MTT.

In summary, according to the relationship between the variation of the differential signal and the concentration of the blood glucose in the single glucose loading experiment of 27 people and a comparison with the data of a control group, it is proved that the real signal of the blood glucose may be obtained by performing a tissue element measurement using the device of measuring the tissue element that uses a single predetermined wavelength combined with a large-area photosensitive surface. Furthermore, an importance and a validity of the three principles of the living tissue element measurement are also proved.

The flowcharts and block diagrams in the accompanying drawings illustrate the possible architecture, functions, and operations of the system, method, and computer program product according to various embodiments of the present disclosure. In this regard, each block in the flowcharts or block diagrams may represent a part of a module, a program segment, or a code, which part includes one or more executable instructions for implementing the specified logical function. It should be further noted that, in some alternative implementations, the functions noted in the blocks may also occur in a different order from that noted in the accompanying drawings. For example, two blocks shown in succession may actually be executed substantially in parallel, or they may sometimes be executed in a reverse order, depending on the functions involved. It should be further noted that each block in the block diagrams or flowcharts, and the combination of blocks in the block diagrams or flowcharts, may be implemented by a dedicated hardware-based system that performs the specified functions or operations, or may be implemented by a combination of dedicated hardware and computer instructions. Those skilled in the art may understand that the various embodiments of the present disclosure and/or the features described in the claims may be combined in various ways, even if such combinations are not explicitly described in the present disclosure. In particular, the various embodiments of the present disclosure and/or the features described in the claims may be combined in various ways without departing from the spirit and teachings of the present disclosure. All these combinations fall within the scope of the present disclosure.

Embodiments of the present disclosure have been described above. However, these embodiments are for illustrative purposes only, and are not intended to limit the scope of the present disclosure. Although the various embodiments have been described separately above, this does not mean that measures in the respective embodiments may not be used in combination advantageously. The scope of the present disclosure is defined by the appended claims and their equivalents. Those skilled in the art may make various substitutions and modifications without departing from the scope of the present disclosure, and these substitutions and modifications should all fall within the scope of the present disclosure.

**Claims**

1. A method of measuring a tissue element, comprising:

irradiating a measurement region with incident light having a single predetermined wavelength, wherein each beam of the incident light passes through the measurement region to form at least one beam of exit light exited from at least one exit position, and a number of incident positions of the incident light is at least one;
obtaining a light intensity value corresponding to each beam of the exit light acquired by M photosensitive surfaces, so as to obtain T output light intensities, wherein each of the T output light intensities is obtained by processing the light intensity value of the exit light acquired by one or more of the M photosensitive surfaces, and each of the M photosensitive surfaces is configured to acquire the light intensity value of the exit light exited from the exit position within a predetermined anti-jitter range corresponding to the photosensitive surface, wherein $1 \leq T \leq M$; and
determining a concentration of a detected tissue element according to at least one output light intensity corresponding to the predetermined wavelength.

**2.** The method according to claim 1, wherein a ratio of an average optical path of the exit light received by each photosensitive surface in a target tissue layer to a total optical path is greater than or equal to a ratio threshold, and the total optical path is a total distance that the exit light travels in the measurement region.

**3.** The method according to claim 1 or 2, further comprising:
determining a total area of a homogeneous photosensitive surface according to a tissue structure feature in the measurement region, wherein the homogeneous photosensitive surface comprises one or more photosensitive surfaces, and the homogeneous photosensitive surface is configured to output one output light intensity.

**4.** The method according to claim 1 or 2, wherein a ratio of an area of each photosensitive surface to a circumference of the photosensitive surface is greater than or equal to a ratio threshold.

**5.** The method according to claim 4, wherein the ratio threshold is greater than or equal to 0.04 mm.

**6.** The method according to claim 1 or 2, wherein the photosensitive surface is in contact or non-contact with a surface of the measurement region.

**7.** The method according to claim 6, wherein a distance between the photosensitive surface and the surface of the measurement region is less than or equal to a first distance threshold, and an efficiency of the photosensitive surface receiving the exit light is greater than or equal to an efficiency threshold.

**8.** The method according to claim 1, further comprising: before irradiating the measurement region with the incident light having the single predetermined wavelength,

determining a positioning feature;
determining the measurement region according to the positioning feature, wherein the measurement region meets a reproducibility of a controllable measurement condition; and
arranging a measurement probe at a position corresponding to the measurement region, wherein the measurement probe comprises the M photosensitive surfaces.

**9.** The method according to claim 6, wherein the positioning feature comprises a first posture positioning feature and a region positioning feature, and
wherein the determining the measurement region according to the positioning feature comprises:

adjusting a current measurement posture of a detected object to a target measurement posture according to the first posture positioning feature, wherein the target measurement posture meets a reproducibility of the controllable measurement condition; and
determining the measurement region according to the region positioning feature, in response to a determination that the current measurement posture is the target measurement posture.

**10.** The method according to claim 9, wherein the arranging a measurement probe at a position corresponding to the measurement region comprises:

arranging the measurement probe at the position corresponding to the measurement region by a fixing portion, wherein the fixing portion is integrated with, partially separated from or completely separated from the measurement probe.

**11.** The method according to claim 10, wherein the fixing portion comprises a fixing seat and a first fitting part, and
wherein the arranging the measurement probe at the position corresponding to the measurement region by the fixing portion comprises:

arranging the fixing seat at the position corresponding to the measurement region by the first fitting part; and
arranging the measurement probe on the fixing seat.

**12.** The method according to claim 11, wherein a skin state of a skin at the measurement region meets a first predetermined condition during a process of arranging the fixing seat at the position corresponding to the measurement region by the first fitting part.

**13.** The method according to claim 11, wherein a skin state of a skin at the measurement region meets a second predetermined condition during a process of arranging the measurement probe on the fixing seat.

**14.** The method according to claim 11, wherein the measurement probe is not movable on the fixing seat.

**15.** The method according to claim 10, wherein the fixing portion comprises a second fitting part, and
wherein the arranging the measurement probe at the position corresponding to the measurement region by the fixing portion comprises:
arranging the measurement probe at the position corresponding to the measurement region by the second fitting part.

**16.** The method according to claim 15, wherein a skin state of a skin at the measurement region meets a third predetermined condition during a process of arranging the measurement probe at the position corresponding to the measurement region by the second fitting part.

**17.** The method according to claim 10, wherein the determining the measurement region according to the region positioning feature comprises:

obtaining a first projection feature;
adjusting, in response to a determination that the region positioning feature is not matched with the first projection feature, a position of the measurement probe and/or the fixing portion until the region positioning feature is matched with the first projection feature; and
determining a region corresponding to the measurement probe and/or the fixing portion as the measurement region in response to a determination that the region positioning feature is matched with the first projection feature.

**18.** The method according to claim 10, wherein the determining the measurement region according to the region positioning feature comprises:

obtaining a first target image;
obtaining a first template image, wherein the first template image comprises the region positioning feature;
adjusting, in response to a determination that the first target image is not matched with the first template image, a position of the measurement probe and/or the fixing portion to obtain a new first target image until the new first target image is matched with the first template image; and
determining a region corresponding to the measurement probe and/or the fixing portion as the measurement region in response to a determination that the first target image is matched with the first template image.

**19.** The method according to claim 10, wherein the determining the measurement region according to the region positioning feature comprises:

obtaining a second target image, wherein the second target image comprises the region positioning feature;
adjusting, in response to a determination that a position of the region positioning feature in the second target image is not a first predetermined position, a position of the measurement probe and/or the fixing portion to obtain a new second target image until the position of the region positioning feature in the new second target image is the first predetermined position; and
determining a region corresponding to the measurement probe and/or the fixing portion as the measurement region in response to a determination that the position of the region positioning feature in the new second target image is the first predetermined position.

**20.** The method according to claim 10, wherein the adjusting a current measurement posture of a detected object to a target measurement posture according to the first posture positioning feature comprises:

obtaining a second projection feature;
adjusting, in response to a determination that the first posture positioning feature is not matched with the second projection feature, the current measurement posture until the first posture positioning feature is matched with the second projection feature; and
determining that the current measurement posture is the target measurement posture, in response to a determination that the first posture positioning feature is matched with the second projection feature.

21. The method according to claim 10, wherein the adjusting a current measurement posture of a detected object to a target measurement posture according to the first posture positioning feature comprises:

obtaining a third target image;
obtaining a second template image, wherein the second template image comprises the first posture positioning feature;
adjusting, in response to a determination that the third target image is not matched with the second template image, the current measurement posture to obtain a new third target image until the new third target image is matched with the second template image; and
determining that the current measurement posture is the target measurement posture, in response to a determination that the new third target image is matched with the second template image.

22. The method according to claim 10, wherein the adjusting a current measurement posture of a detected object to a target measurement posture according to the first posture positioning feature comprises:

obtaining a fourth target image, wherein the fourth target image comprises the first posture positioning feature;
adjusting, in response to a determination that a position of the first posture positioning feature in the fourth target image is not a second predetermined position, the current measurement posture to obtain a new fourth target image until the position of the first posture positioning feature in the new fourth target image is the second predetermined position; and
determining that the current measurement posture is the target measurement posture, in response to a determination that the position of the first posture positioning feature in the new fourth target image is the second predetermined position.

23. The method according to claim 10, further comprising:

determining a second posture positioning feature in response to a determination that the current measurement posture is not the target measurement posture, if the measurement probe is arranged at the position corresponding to the measurement region; and
adjusting the current measurement posture to the target measurement posture according to the second posture positioning feature.

24. The method according to claim 23, wherein the adjusting the current measurement posture to the target measurement posture according to the second posture positioning feature comprises:

obtaining a third projection feature;
adjusting, in response to a determination that the second posture positioning feature is not matched with the third projection feature, the current measurement posture until the second posture positioning feature is matched with the third projection feature; and
determining that the current measurement posture is the target measurement posture, in response to a determination that the second posture positioning feature is matched with the third projection feature.

25. The method according to claim 23, wherein the adjusting the current measurement posture to the target measurement posture according to the second posture positioning feature comprises:

obtaining a fifth target image;
obtaining a third template image, wherein the third template image comprises the second posture positioning feature;
adjusting, in response to a determination that the fifth target image is not matched with the third template image, the current measurement posture to obtain a new fifth target image until the new fifth target image is matched with the third template image; and
determining that the current measurement posture is the target measurement posture, in response to a determination that the new fifth target image is matched with the third template image.

26. The method according to claim 23, wherein the adjusting the current measurement posture to the target measurement posture according to the second posture positioning feature comprises:

obtaining a sixth target image, wherein the sixth target image comprises the second posture positioning feature;

adjusting, in response to a determination that a position of the second posture positioning feature in the sixth target image is not a third predetermined position, the current measurement posture to obtain a new sixth target image until the position of the second posture positioning feature in the new sixth target image is the third predetermined position; and

determining that the current measurement posture is the target measurement posture, in response to a determination that the position of the second posture positioning feature in the new sixth target image is the third predetermined position.

27. The method according to claim 23, further comprising:
generating a prompt information, wherein the prompt information is configured to prompt a completion of a positioning of the measurement posture and/or a positioning of the measurement region, and a form of the prompt information comprises at least one of an image, a speech, or a vibration.

28. The method according to claim 11, further comprising:

arranging the measurement probe on the fixing seat in response to a determination that the fixing seat is arranged at the position corresponding to the measurement region and the measurement probe is not arranged on the fixing portion; or

arranging the fixing seat at the position corresponding to the measurement region by the first fitting part in response to a determination that the fixing seat is not arranged at the position corresponding to the measurement region, and arranging the measurement probe on the fixing seat.

29. The method according to claim 15, further comprising:
arranging the measurement probe at the position corresponding to the measurement region by the second fitting part, in response to a determination that the measurement probe is not arranged at the position corresponding to the measurement region.

30. The method according to claim 1, wherein the determining a concentration of a detected tissue element according to at least one output light intensity corresponding to the predetermined wavelength comprises:

determining a first output light intensity and a second output light intensity from at least two output light intensities corresponding to the predetermined wavelength; and
determining the concentration of the detected tissue element according to the first output light intensity and the second output light intensity corresponding to the predetermined wavelength.

31. The method according to claim 30, wherein the determining the concentration of the detected tissue element according to the first output light intensity and the second output light intensity corresponding to the predetermined wavelength comprises:

performing a differential processing on the first output light intensity and the second output light intensity corresponding to the predetermined wavelength, so as to obtain a differential signal; and
determining the concentration of the detected tissue element according to the differential signal corresponding to the predetermined wavelength.

32. The method according to claim 31, wherein the performing a differential processing on the first output light intensity and the second output light intensity corresponding to the predetermined wavelength to obtain a differential signal comprises:
processing the first output light intensity and the second output light intensity corresponding to the predetermined wavelength by using a differential circuit, so as to obtain the differential signal.

33. The method according to claim 31, wherein the performing a differential processing on the first output light intensity and the second output light intensity corresponding to the predetermined wavelength to obtain a differential signal comprises:
processing the first output light intensity and the second output light intensity corresponding to the predetermined wavelength by using a differential algorithm, so as to obtain the differential signal.

34. The method according to claim 33, wherein the processing the first output light intensity and the second output light intensity corresponding to the predetermined wavelength by using a differential algorithm to obtain the differential signal comprises:

performing a direct differential operation on the first output light intensity and the second output light intensity corresponding to the predetermined wavelength, so as to obtain the differential signal.

35. The method according to claim 33, wherein the processing the first output light intensity and the second output light intensity corresponding to the predetermined wavelength by using a differential algorithm to obtain the differential signal comprises:

performing a logarithmic processing on the first output light intensity and the second output light intensity corresponding to the predetermined wavelength to obtain a first logarithmic light intensity and a second logarithmic light intensity; and

performing a direct differential operation on the first logarithmic light intensity and the second logarithmic light intensity corresponding to the predetermined wavelength, so as to obtain the differential signal.

36. The method according to claim 31, wherein the determining the concentration of the detected tissue element according to the differential signal corresponding to the predetermined wavelength comprises:
inputting the differential signal corresponding to the predetermined wavelength into a first concentration prediction model for the tissue element to output the concentration of the detected tissue element.

37. The method according to claim 36, further comprising:

obtaining a set of first training samples, wherein the set of first training samples comprises a plurality of first training samples, and each of the plurality of first training samples comprises a first true concentration of the detected tissue element and a differential signal corresponding to the first true concentration; and
establishing the first concentration prediction model for the tissue element according to the set of first training samples.

38. The method according to claim 37, wherein the establishing the first concentration prediction model for the tissue element according to the set of first training samples comprises:

pre-processing the set of first training samples to obtain a processed set of first training samples; and
establishing the first concentration prediction model for the tissue element according to the processed set of first training samples.

39. The method according to claim 36, further comprising:

in response to a fourth predetermined condition being met,
correcting the first concentration prediction model for the tissue element to obtain a corrected first concentration prediction model for the tissue element, so as to process a new differential signal by using the corrected first concentration prediction model for the tissue element to obtain a new concentration of the detected tissue element.

40. The method according to claim 39, wherein the correcting the first concentration prediction model for the tissue element comprises:

obtaining a first target concentration of the detected tissue element;
obtaining a differential signal corresponding to the first target concentration; and
correcting the first concentration prediction model for the tissue element according to the first target concentration and the differential signal corresponding to the first target concentration.

41. The method according to claim 36, further comprising:

in response to a fifth predetermined condition being met,
processing a new differential signal by using a new first concentration prediction model for the tissue element to obtain a new concentration of the detected tissue element.

42. The method according to claim 31, wherein the determining the concentration of the detected tissue element according to the differential signal corresponding to the predetermined wavelength comprises:

obtaining a current interference parameter value of each interference parameter of a plurality of interference parameters; and

inputting a plurality of current interference parameter values and the differential signal corresponding to the predetermined wavelength into a second concentration prediction model for the tissue element to output the concentration of the detected tissue element.

43. The method according to claim 42, further comprising:

obtaining a set of second training samples, wherein the set of second training samples comprises a plurality of second training samples, and each of the plurality of second training samples comprises a second true concentration of the detected tissue element and a differential signal corresponding to the second true concentration;
obtaining a set of third training samples, wherein the set of third training samples comprises a plurality of third training samples, and each of the plurality of third training samples comprises a training interference parameter value of each interference parameter of a plurality of interference parameters and a differential signal corresponding to each training interference parameter value;
establishing a tissue element concentration prediction model to be corrected, according to the set of second training samples;
establishing a correction parameter model according to the set of third training samples; and
obtaining the second concentration prediction model for the tissue element according to the tissue element concentration prediction model to be corrected and the correction parameter model.

44. The method according to claim 42, further comprising:

in response to a fourth predetermined condition being met,
correcting the second concentration prediction model for the tissue element to obtain a corrected second concentration prediction model for the tissue element, so as to process a new differential signal and a plurality of new current interference parameter values by using the corrected second concentration prediction model for the tissue element to obtain a new concentration of the detected tissue element.

45. The method according to claim 44, wherein the correcting the second concentration prediction model for the tissue element comprises:

obtaining a second target concentration of the detected tissue element;
obtaining a differential signal corresponding to the second target concentration;
obtaining a current interference parameter value of each interference parameter of a plurality of interference parameters; and
correcting the second concentration prediction model for the tissue element according to the second target concentration, a plurality of interference parameter values, and the differential signal corresponding to the second target concentration.

46. The method according to claim 42, further comprising:

in response to a fifth predetermined condition being met,
processing a new differential signal and a plurality of new current interference parameter values by using a new second concentration prediction model for the tissue element to obtain a new concentration of the detected tissue element.

47. The method according to claim 31, wherein the first output light intensity and the second output light intensity are acquired at different time instants by a same homogeneous photosensitive surface or different homogeneous photosensitive surfaces, the first output light intensity is a light intensity for a contraction period, the second output light intensity is a light intensity for a relaxation period, the homogeneous photosensitive surface comprises one or more photosensitive surfaces, and the homogeneous photosensitive surface is configured to output one output light intensity.

48. The method according to claim 31, wherein the first output light intensity corresponding to the predetermined wavelength is acquired by a first homogeneous photosensitive surface corresponding to the predetermined wavelength, the second output light intensity corresponding to the predetermined wavelength is acquired by a second homogeneous photosensitive surface corresponding to the predetermined wavelength, the first homogeneous photosensitive surface comprises one or more photosensitive surfaces, and the second homogeneous photosensitive surface comprises one or more photosensitive surfaces.

49. The method according to claim 48, wherein the first homogeneous photosensitive surface and the second homogeneous photosensitive surface are a same homogeneous photosensitive surface, and the exit light received by the first homogeneous photosensitive surface and the exit light received by the second homogeneous photosensitive surface are obtained by a transmission of the incident light incident at different incident positions.

50. The method according to claim 48, wherein the first homogeneous photosensitive surface and the second homogeneous photosensitive surface are different homogeneous photosensitive surfaces.

51. The method according to claim 48, wherein an average optical path of the exit light received at different photosensitive positions of each photosensitive surface in the first homogeneous photosensitive surface is within a first average optical path range, the first average optical path range is determined according to a first optical path average value, and the first optical path average value is an average value calculated according to the average optical paths of the exit light received at the photosensitive positions of the first homogeneous photosensitive surface; and
wherein an average optical path of the exit light received at different photosensitive positions of each photosensitive surface in the second homogeneous photosensitive surface is within a second average optical path range, the second average optical path range is determined according to a second optical path average value, and the second optical path average value is an average value calculated according to the average optical paths of the exit light received at the photosensitive positions of the second homogeneous photosensitive surface.

52. The method according to claim 51, wherein an absolute value of a difference between the first optical path average value and the second optical path average value is within a first optical path difference range.

53. The method according to claim 52, wherein the first average optical path range is less than or equal to the first optical path difference range, and the second average optical path range is less than or equal to the first optical path difference range.

54. The method according to claim 52, wherein the first optical path difference range is determined according to an optimal differential optical path corresponding to the predetermined wavelength.

55. The method according to claim 48, wherein a source-detection distance of each photosensitive surface in the first homogeneous photosensitive surface corresponding to the predetermined wavelength from a center of the incident light is within a predetermined source-detection distance range corresponds to the predetermined wavelength, and the predetermined source-detection distance range is determined according to a source-detection distance of a floating reference position corresponding to the predetermined wavelength from the center of the incident light.

56. The method according to claim 1, wherein the determining a concentration of a detected tissue element according to at least one output light intensity corresponding to the predetermined wavelength comprises:

determining a third output light intensity from the at least one output light intensity corresponding to the predetermined wavelength; and
determining the concentration of the detected tissue element according to the third output light intensity corresponding to the predetermined wavelength.

57. The method according to claim 56, wherein the third output light intensity corresponding to the predetermined wavelength is acquired by the homogeneous photosensitive surface corresponding to the predetermined wavelength, and a difference between the average optical path of the exit light received at different photosensitive positions of each photosensitive surface in the homogeneous photosensitive surface and an optimal optical path corresponding to the predetermined wavelength is within a second optical path difference range.

58. The method according to claim 56, wherein the determining the concentration of the detected tissue element according to the third output light intensity corresponding to the predetermined wavelength comprises:
inputting the third output light intensity corresponding to the predetermined wavelength into a third concentration prediction model for the tissue element to output the concentration of the detected tissue element.

59. The method according to claim 1 or 2, wherein each photosensitive surface comprises an annular photosensitive surface or a non-annular photosensitive surface, and different photosensitive surfaces have a same shape or different shapes.

60. The method according to claim 59, wherein the non-annular photosensitive surface comprises a sector-annular photosensitive surface, a circular photosensitive surface, a sector photosensitive surface, an elliptical photosensitive surface, or a polygonal photosensitive surface.

61. The method according to claim 60, wherein the polygonal photosensitive surface comprises a square photosensitive surface, a rectangular photosensitive surface, or a triangular photosensitive surface.

62. The method according to claim 59, wherein the homogeneous photosensitive surface comprises the annular photosensitive surface or the non-annular photosensitive surface, the homogeneous photosensitive surface comprises one or more photosensitive surfaces, and the homogeneous photosensitive surface is configured to output one output light intensity.

63. The method according to claim 62, wherein the homogeneous photosensitive surface being the annular photosensitive surface comprises that:

the homogeneous photosensitive surface is an independent annular photosensitive surface in response to the homogeneous photosensitive surface comprising one photosensitive surface; or
the homogeneous photosensitive surface is an annular photosensitive surface formed by combining a plurality of photosensitive surfaces in response to the homogeneous photosensitive surface comprising the plurality of photosensitive surfaces, and
wherein the homogeneous photosensitive surface being the non-annular photosensitive surface comprises that:

the homogeneous photosensitive surface is an independent non-annular photosensitive surface in response to the homogeneous photosensitive surface comprising one photosensitive surface; or
the homogeneous photosensitive surface is a non-annular photosensitive surface formed by combining a plurality of photosensitive surfaces in response to the homogeneous photosensitive surface comprising the plurality of photosensitive surfaces.

64. The method according to claim 63, wherein the homogeneous photosensitive surface comprises the annular photosensitive surface, a sector-annular photosensitive surface, a sector photosensitive surface, a circular photosensitive surface or a square photosensitive surface, in response to a determination that a distance between the homogeneous photosensitive surface and a target site is greater than or equal to a second distance threshold.

65. The method according to claim 63, wherein a shape of the homogeneous photosensitive surface is determined according to a jitter distribution of the exit light, in response to a determination that a distance between the homogeneous photosensitive surface and a target site is less than or equal to a third distance threshold.

66. The method according to claim 65, wherein the jitter distribution of the exit light is decomposed into a jitter distribution in a first direction and a jitter distribution in a second direction perpendicular to the first direction, a ratio of a length of the homogeneous photosensitive surface in the first direction to a length of the homogeneous photosensitive surface in the second direction is determined according to a ratio of a jitter amplitude of the exit light in the first direction to a jitter amplitude of the exit light in the second direction, and the exit light has a maximum jitter amplitude in the first direction.

67. The method according to claim 66, wherein the homogeneous photosensitive surface comprises a rectangular photosensitive surface or an elliptical photosensitive surface, a ratio of a length of the rectangular photosensitive surface to a width of the rectangular photosensitive surface is determined according to the ratio of the jitter amplitude of the exit light in the first direction to the jitter amplitude of the exit light in the second direction, and a ratio of a major axis of the elliptical photosensitive surface to a minor axis of the elliptical photosensitive surface is determined according to the ratio of the jitter amplitude of the exit light in the first direction to the jitter amplitude of the exit light in the second direction.

68. The method according to claim 1 or 2, wherein each of the output light intensities being obtained by processing the light intensity value of the exit light acquired by one or more of the photosensitive surfaces comprises:

outputting one output light intensity by using the one or more photosensitive surfaces in combination; or calculating the light intensity value of the exit light acquired by the one or more photosensitive surfaces to obtain one output light intensity, by using each of the one or more photosensitive surfaces separately.

69. The method according to claim 1 or 2, wherein the determining a concentration of a detected tissue element according to at least one output light intensity corresponding to the predetermined wavelength comprises:

determining at least one added light intensity corresponding to the predetermined wavelength, wherein the added light intensity is obtained by adding a plurality of output light intensities corresponding to the predetermined wavelength; and
determining the concentration of the detected tissue element according to at least one added light intensity corresponding to the predetermined wavelength.

70. The method according to claim 1 or 2, wherein the predetermined wavelength is a wavelength sensitive to the detected tissue element.

71. The method according to claim 1 or 2, wherein a temperature of the measurement region is maintained within a predetermined temperature range during a process of measuring the tissue element.

72. The method according to claim 1 or 2, wherein the photosensitive surface is obtained after providing a mask on an initial photosensitive surface, and a light transmittance of the mask is less than or equal to a light transmittance threshold.

73. The method according to claim 72, wherein a shape of the mask is determined according to a shape of a jitter distribution of the exit light.

74. The method according to claim 1 or 2, wherein a light spot irradiated to the measurement region by the incident light has a uniform intensity distribution.

75. The method according to claim 1 or 2, wherein an area of a light spot irradiated to the measurement region by the incident light is greater than or equal to a light spot area threshold.

76. A device of measuring a tissue element, comprising:

a light source module configured to irradiate a measurement region with incident light having a single predetermined wavelength, wherein each beam of the incident light passes through the measurement region to form at least one beam of exit light exited from at least one exit position, and a number of incident positions of the incident light is at least one;
an acquisition module comprising M photosensitive surfaces, wherein each of the M photosensitive surfaces is configured to acquire a light intensity value of the exit light exited from the exit position within a predetermined anti-jitter range corresponding to the photosensitive surface, the acquisition module is configured to obtain a light intensity value corresponding to each beam of the exit light acquired by the M photosensitive surfaces, so as to obtain T output light intensities, and each of the T output light intensities is obtained by processing the light intensity value of the exit light acquired by one or more of the M photosensitive surfaces, and wherein $1 < T < M$; and
a processing module configured to determine a concentration of a detected tissue element according to at least one output light intensity corresponding to the predetermined wavelength.

77. The device according to claim 76, wherein a ratio of an average optical path of the exit light received by each photosensitive surface in a target tissue layer to a total optical path is greater than or equal to a ratio threshold, and the total optical path is a total distance that the exit light travels in the measurement region.

78. The device according to claim 76 or 77, wherein a total area of a homogeneous photosensitive surface is determined according to a tissue structure feature in the measurement region, the homogeneous photosensitive surface comprises one or more photosensitive surfaces, and the homogeneous photosensitive surface is configured to output one output light intensity.

79. The device according to claim 76 or 77, wherein a ratio of an area of each photosensitive surface to a circumference of the photosensitive surface is greater than or equal to a ratio threshold.

80. The device according to claim 79, wherein the ratio threshold is greater than or equal to 0.04 mm.

81. The device according to claim 76 or 77, wherein the photosensitive surface is in contact or non-contact with a surface of the measurement region.

82. The device according to claim 81, wherein a distance between the photosensitive surface and the surface of the measurement region is less than or equal to a first distance threshold, and an efficiency of the photosensitive surface receiving the exit light is greater than or equal to an efficiency threshold.

83. The device according to claim 76, further comprising:

a first determination module configured to determine a positioning feature;
a second determination module configured to determine the measurement region according to the positioning feature, wherein the measurement region meets a reproducibility of a controllable measurement condition; and
an arrangement module configured to arrange a measurement probe at a position corresponding to the measurement region, wherein the measurement probe comprises the M photosensitive surfaces.

84. The device according to claim 83, wherein the positioning feature comprises a first posture positioning feature and a region positioning feature, and
wherein the second determination module comprises:

a first adjustment unit configured to adjust a current measurement posture of a detected object to a target measurement posture according to the first posture positioning feature, wherein the target measurement posture meets a reproducibility of the controllable measurement condition; and
a first determination unit configured to determine the measurement region according to the region positioning feature, in response to a determination that the current measurement posture is the target measurement posture.

85. The device according to claim 84, further comprising a fixing portion configured to arrange the measurement probe at the position corresponding to the measurement region, wherein the fixing portion is integrated with, partially separated from or completely separated from the measurement probe.

86. The device according to claim 85, wherein the fixing portion comprises a fixing seat and a first fitting part, and

wherein the first fitting part is configured to arrange the fixing seat at the position corresponding to the measurement region; and
the fixing seat is configured to fix the measurement probe.

87. The device according to claim 86, wherein a hardness of the first fitting part comprises a first hardness and a second hardness, the first hardness is less than the second hardness, the first hardness is a corresponding hardness in a process of fixing the fixing seat by the first fitting part, and the second hardness is a corresponding hardness after the fixing seat is fixed by the first fitting part.

88. The device according to claim 87, wherein the first fitting part comprises a first Velcro or a first elastic belt.

89. The device according to claim 87, wherein the hardness of the first fitting part is greater than or equal to a first hardness threshold and less than or equal to a second hardness threshold.

90. The device according to claim 86, further comprising a first magnetic portion, wherein the first fitting part is entirely or partially a metal hinge, and the first magnetic portion fits the first fitting part to fix the fixing seat.

91. The device according to claim 86, wherein a surface of the first fitting part is provided with a hole.

92. The device according to claim 86, wherein the measurement probe is fixed on the fixing seat by at least one of:

fixing the measurement probe on the fixing seat by an adhesive tape;
fixing the measurement probe on the fixing seat by a fastener;

fixing the measurement probe on the fixing seat by a magnetic force; or
setting a friction coefficient between the measurement probe and the fixing seat to be greater than or equal to a friction coefficient threshold.

93. The device according to claim 85, wherein the fixing portion comprises a second fitting part configured to arrange the measurement probe at the position corresponding to the measurement region.

94. The device according to claim 93, wherein a hardness of the second fitting part comprises a third hardness and a fourth hardness, the third hardness is less than the fourth hardness, the third hardness is a corresponding hardness in a process of fixing the measurement probe by the second fitting part, and the fourth hardness is a corresponding hardness after the measurement probe is fixed by the second fitting part.

95. The device according to claim 94, wherein the second fitting part comprises a second Velcro or a second elastic belt.

96. The device according to claim 94, wherein the hardness of the second fitting part is greater than or equal to a third hardness threshold and less than or equal to a fourth hardness threshold.

97. The device according to claim 93, further comprising a second magnetic portion, wherein the second fitting part is entirely or partially a metal hinge, and the second magnetic portion fits the second fitting part to fix the measurement probe.

98. The device according to claim 93, wherein a surface of the second fitting part is provided with a hole.

99. The device according to claim 85, wherein the first determination unit is configured to:

obtain a first projection feature;
adjust, in response to a determination that the region positioning feature is not matched with the first projection feature, a position of the measurement probe and/or the fixing portion until the region positioning feature is matched with the first projection feature; and
determine a region corresponding to the measurement probe and/or the fixing portion as the measurement region in response to a determination that the region positioning feature is matched with the first projection feature.

100. The device according to claim 99, further comprising a region positioning portion arranged on the detected object, the measurement probe, the fixing portion or other objects, wherein the region positioning portion is configured to project the first projection feature.

101. The device according to claim 100, wherein the region positioning feature is not arranged on the measurement probe in response to a determination that the region positioning portion is arranged on the measurement probe, and wherein the region positioning feature is not arranged on the fixing portion in response to a determination that the region positioning portion is arranged on the fixing portion.

102. The device according to claim 100, wherein the region positioning portion comprises a first laser.

103. The device according to claim 85, wherein the first determination unit is configured to:

obtain a first target image;
obtain a first template image, wherein the first template image comprises the region positioning feature;
adjust, in response to a determination that the first target image is not matched with the first template image, a position of the measurement probe and/or the fixing portion to obtain a new first target image until the new first target image is matched with the first template image; and
determine a region corresponding to the measurement probe and/or the fixing portion as the measurement region in response to a determination that the first target image is matched with the first template image.

104. The device according to claim 103, further comprising a first image acquisition portion arranged on the detected object, the measurement probe, the fixing portion or other objects, wherein the first image acquisition portion is configured to acquire the first target image.

105. The device according to claim 85, wherein the first determination unit is configured to:

obtain a second target image, wherein the second target image comprises the region positioning feature;

adjust, in response to a determination that a position of the region positioning feature in the second target image is not a first predetermined position, a position of the measurement probe and/or the fixing portion to obtain a new second target image until the position of the region positioning feature in the new second target image is the first predetermined position; and

determine a region corresponding to the measurement probe and/or the fixing portion as the measurement region in response to a determination that the position of the region positioning feature in the new second target image is the first predetermined position.

106. The device according to claim 105, further comprising a second image acquisition portion arranged on the detected object, the measurement probe, the fixing portion or other objects, wherein the second image acquisition portion is configured to acquire the second target image.

107. The device according to claim 106, wherein the region positioning feature is not arranged on the measurement probe in response to a determination that the second image acquisition portion is arranged on the measurement probe, and

wherein the region positioning feature is not arranged on the fixing portion in response to a determination that the second image acquisition portion is arranged on the fixing portion.

108. The device according to claim 85, wherein the first adjustment unit is configured to:

obtain a second projection feature;

adjust, in response to a determination that the first posture positioning feature is not matched with the second projection feature, the current measurement posture until the first posture positioning feature is matched with the second projection feature; and

determine that the current measurement posture is the target measurement posture, in response to a determination that the first posture positioning feature is matched with the second projection feature.

109. The device according to claim 109, further comprising a first posture positioning portion arranged on the detected object, the measurement probe, the fixing portion or other objects, wherein the first posture positioning portion is configured to project the second projection feature.

110. The device according to claim 109, wherein the first posture positioning feature is not arranged on the measurement probe in response to a determination that the first posture positioning portion is arranged on the measurement probe, and

wherein the first posture positioning feature is not arranged on the fixing portion in response to a determination that the first posture positioning portion is arranged on the fixing portion.

111. The device according to claim 109, wherein the first posture positioning portion comprises a second laser.

112. The device according to claim 85, wherein the first adjustment unit is configured to:

obtain a third target image;

obtain a second template image, wherein the second template image comprises the first posture positioning feature;

adjust, in response to a determination that the third target image is not matched with the second template image, the current measurement posture to obtain a new third target image until the new third target image is matched with the second template image; and

determine that the current measurement posture is the target measurement posture, in response to a determination that the new third target image is matched with the second template image.

113. The device according to claim 112, further comprising a third image acquisition portion arranged on the detected object, the measurement probe, the fixing portion or other objects, wherein the third image acquisition portion is configured to acquire the third target image.

114. The device according to claim 85, wherein the first adjustment unit is configured to:

obtain a fourth target image, wherein the fourth target image comprises the first posture positioning feature;

adjust, in response to a determination that a position of the first posture positioning feature in the fourth target image is not a second predetermined position, the current measurement posture to obtain a new fourth target image until the position of the first posture positioning feature in the new fourth target image is the second predetermined position; and

determine that the current measurement posture is the target measurement posture, in response to a determination that the position of the first posture positioning feature in the new fourth target image is the second predetermined position.

115. The device according to claim 114, further comprising a fourth image acquisition portion arranged on the detected object, the measurement probe, the fixing portion or other objects, wherein the fourth image acquisition portion is configured to acquire the fourth target image.

116. The device according to claim 115, wherein the first posture positioning feature is not arranged on the measurement probe in response to a determination that the fourth image acquisition portion is arranged on the measurement probe, and

wherein the first posture positioning feature is not arranged on the fixing portion in response to a determination that the fourth image acquisition portion is arranged on the fixing portion.

117. The device according to claim 85, further comprising:

a third determination module configured to determine a second posture positioning feature in response to a determination that the current measurement posture is not the target measurement posture, if the measurement probe is arranged at the position corresponding to the measurement region; and

an adjustment module configured to adjust the current measurement posture to the target measurement posture according to the second posture positioning feature.

118. The device according to claim 117, wherein the adjustment module comprises:

a first obtaining unit configured to obtain a third projection feature;

a second adjustment unit configured to adjust, in response to a determination that the second posture positioning feature is not matched with the third projection feature, the current measurement posture until the second posture positioning feature is matched with the third projection feature; and

a second determination unit configured to determine that the current measurement posture is the target measurement posture, in response to a determination that the second posture positioning feature is matched with the third projection feature.

119. The device according to claim 118, further comprising a second posture positioning portion arranged on the detected object, the measurement probe, the fixing portion or other objects, wherein the second posture positioning portion is configured to project the third projection feature.

120. The device according to claim 119, wherein the second posture positioning feature is not arranged on the measurement probe and the fixing portion in response to a determination that the second posture positioning portion is arranged on the measurement probe, and

wherein the second posture positioning feature is not arranged on the measurement probe and the fixing portion in response to a determination that the second posture positioning portion is arranged on the fixing portion.

121. The device according to claim 120, wherein the second posture positioning portion comprises a third laser.

122. The device according to claim 117, wherein the adjustment module comprises:

a second obtaining unit configured to obtain a fifth target image;

a third obtaining unit configured to obtain a third template image, wherein the third template image comprises the second posture positioning feature;

a third adjustment unit configured to adjust, in response to a determination that the fifth target image is not matched with the third template image, the current measurement posture to obtain a new fifth target image until the new fifth target image is matched with the third template image; and

a third determination unit configured to determine that the current measurement posture is the target measurement posture, in response to a determination that the new fifth target image is matched with the third template image.

123. The device according to claim 122, further comprising a fifth image acquisition portion arranged on the detected object, the measurement probe, the fixing portion or other objects, wherein the fifth image acquisition portion is configured to acquire the fifth target image.

124. The device according to claim 117, wherein the adjustment module comprises:

a fourth obtaining unit configured to obtain a sixth target image, wherein the sixth target image comprises the second posture positioning feature;

a fourth adjustment unit configured to adjust, in response to a determination that a position of the second posture positioning feature in the sixth target image is not a third predetermined position, the current measurement posture to obtain a new sixth target image until the position of the second posture positioning feature in the new sixth target image is the third predetermined position; and

a fourth determination unit configured to determine that the current measurement posture is the target measurement posture, in response to a determination that the position of the second posture positioning feature in the new sixth target image is the third predetermined position.

125. The device according to claim 124, further comprising a sixth image acquisition portion arranged on the detected object, the measurement probe, the fixing portion or other objects, wherein the sixth image acquisition portion is configured to acquire the sixth target image.

126. The device according to claim 125, wherein the second posture positioning feature is not arranged on the measurement probe and the fixing portion in response to a determination that the sixth image acquisition portion is arranged on the measurement probe, and

wherein the second posture positioning feature is not arranged on the measurement probe and the fixing portion in response to a determination that the sixth image acquisition portion is arranged on the fixing portion.

127. The device according to claim 117, further comprising:

a prompt module configured to generate a prompt information, wherein the prompt information is configured to prompt a completion of a positioning of the measurement posture and/or a positioning of the measurement region, and a form of the prompt information comprises at least one of an image, a speech, or a vibration.

128. The device according to claim 76, wherein the M photosensitive surfaces comprise one or more homogeneous photosensitive surfaces corresponding to the predetermined wavelength, the homogeneous photosensitive surface is configured to acquire a first output light intensity and/or a second output light intensity corresponding to the predetermined wavelength at different time instants, the first output light intensity is a light intensity for a contraction period, the second output light intensity is a light intensity for a relaxation period, and the homogeneous photosensitive surface comprises one or more photosensitive surfaces, and

wherein the processing module is configured to determine the concentration of the detected tissue element according to the first output light intensity and the second output light intensity corresponding to the predetermined wavelength.

129. The device according to claim 76, wherein the M photosensitive surfaces comprise a first homogeneous photosensitive surface and a second homogeneous photosensitive surface corresponding to the predetermined wavelength, the first homogeneous photosensitive surface is configured to acquire a first output light intensity corresponding to the predetermined wavelength, the second homogeneous photosensitive surface is configured to acquire a second output light intensity corresponding to the predetermined wavelength, the first homogeneous photosensitive surface comprises one or more photosensitive surfaces, and the second homogeneous photosensitive surface comprises one or more photosensitive surfaces, and

wherein the processing module is configured to determine the concentration of the detected tissue element according to the first output light intensity and the second output light intensity corresponding to the predetermined wavelength.

130. The device according to claim 129, wherein the first homogeneous photosensitive surface and the second homogeneous photosensitive surface are a same homogeneous photosensitive surface, and the exit light received by the first homogeneous photosensitive surface and the exit light received by the second homogeneous photosensitive surface are obtained by a transmission of the incident light incident at different incident positions.

131. The device according to claim 129, wherein the first homogeneous photosensitive surface and the second homogeneous photosensitive surface are different homogeneous photosensitive surfaces.

132. The device according to claim 129, wherein an average optical path of the exit light received at different photosensitive positions of each photosensitive surface in the first homogeneous photosensitive surface is within a first average optical path range, the first average optical path range is determined according to a first optical path average value, and the first optical path average value is an average value calculated according to the average optical paths of the exit light received at the photosensitive positions of the first homogeneous photosensitive surface, and
wherein an average optical path of the exit light received at different photosensitive positions of each photosensitive surface in the second homogeneous photosensitive surface is within a second average optical path range, the second average optical path range is determined according to a second optical path average value, and the second optical path average value is an average value calculated according to the average optical paths of the exit light received at the photosensitive positions of the second homogeneous photosensitive surface.

133. The device according to claim 132, wherein an absolute value of a difference between the first optical path average value and the second optical path average value is within a first optical path difference range.

134. The device according to claim 133, wherein the first average optical path range is less than or equal to the first optical path difference range, and the second average optical path range is less than or equal to the first optical path difference range.

135. The device according to claim 133, wherein the first optical path difference range is determined according to an optimal differential optical path corresponding to the predetermined wavelength.

136. The device according to claim 129, wherein a source-detection distance of each photosensitive surface in the first homogeneous photosensitive surface corresponding to the predetermined wavelength from a center of the incident light is within a predetermined source-detection distance range corresponds to the predetermined wavelength, and the predetermined source-detection distance range is determined according to a source-detection distance of a floating reference position corresponding to the predetermined wavelength from the center of the incident light.

137. The device according to claim 76, wherein the M photosensitive surfaces comprise a homogeneous photosensitive surface corresponding to the predetermined wavelength, the homogeneous photosensitive surface is configured to acquire a third output light intensity corresponding to the predetermined wavelength, and the homogeneous photosensitive surface comprises one or more photosensitive surfaces, and
wherein the processing module is configured to determine the concentration of the detected tissue element according to the third output light intensity corresponding to the predetermined wavelength.

138. The device according to claim 137, wherein a difference between the average optical path of the exit light received at different photosensitive positions of each photosensitive surface in the homogeneous photosensitive surface and an optimal optical path corresponding to the predetermined wavelength is within a second optical path difference range.

139. The device according to claim 76 or 77, wherein each photosensitive surface comprises an annular photosensitive surface or a non-annular photosensitive surface, and different photosensitive surfaces have a same shape or different shapes.

140. The device according to claim 139, wherein the non-annular photosensitive surface comprises a sector-annular photosensitive surface, a circular photosensitive surface, a sector photosensitive surface, an elliptical photosensitive surface, or a polygonal photosensitive surface.

141. The device according to claim 140, wherein the polygonal photosensitive surface comprises a square photosensitive surface, a rectangular photosensitive surface, or a triangular photosensitive surface.

142. The device according to claim 139, wherein the homogeneous photosensitive surface comprises the annular photosensitive surface or the non-annular photosensitive surface, the homogeneous photosensitive surface comprises one or more photosensitive surfaces, and the homogeneous photosensitive surface is configured to output one output light intensity.

143. The device according to claim 142, wherein the homogeneous photosensitive surface being the annular photosensitive surface comprises that:

the homogeneous photosensitive surface is an independent annular photosensitive surface in response to the homogeneous photosensitive surface comprising one photosensitive surface; or
the homogeneous photosensitive surface is an annular photosensitive surface formed by combining a plurality of photosensitive surfaces in response to the homogeneous photosensitive surface comprising the plurality of photosensitive surfaces, and
wherein the homogeneous photosensitive surface being the non-annular photosensitive surface comprises that:

the homogeneous photosensitive surface is an independent non-annular photosensitive surface in response to the homogeneous photosensitive surface comprising one photosensitive surface; or
the homogeneous photosensitive surface is a non-annular photosensitive surface formed by combining a plurality of photosensitive surfaces in response to the homogeneous photosensitive surface comprising the plurality of photosensitive surfaces.

144. The device according to claim 143, wherein the homogeneous photosensitive surface comprises the annular photosensitive surface, a sector-annular photosensitive surface, a sector photosensitive surface, a circular photosensitive surface or a square photosensitive surface, in response to a determination that a distance between the homogeneous photosensitive surface and a target site is greater than or equal to a second distance threshold.

145. The device according to claim 143, wherein a shape of the homogeneous photosensitive surface is determined according to a jitter distribution of the exit light, in response to a determination that a distance between the homogeneous photosensitive surface and a target site is less than or equal to a third distance threshold.

146. The device according to claim 145, wherein the jitter distribution of the exit light is decomposed into a jitter distribution in a first direction and a jitter distribution in a second direction perpendicular to the first direction, a ratio of a length of the homogeneous photosensitive surface in the first direction to a length of the homogeneous photosensitive surface in the second direction is determined according to a ratio of a jitter amplitude of the exit light in the first direction to a jitter amplitude of the exit light in the second direction, and the exit light has a maximum jitter amplitude in the first direction.

147. The device according to claim 146, wherein the homogeneous photosensitive surface comprises a rectangular photosensitive surface or an elliptical photosensitive surface, a ratio of a length of the rectangular photosensitive surface to a width of the rectangular photosensitive surface is determined according to the ratio of the jitter amplitude of the exit light in the first direction to the jitter amplitude of the exit light in the second direction, and a ratio of a major axis of the elliptical photosensitive surface to a minor axis of the elliptical photosensitive surface is determined according to the ratio of the jitter amplitude of the exit light in the first direction to the jitter amplitude of the exit light in the second direction.

148. The device according to claim 76 or 77, wherein anodes of different photosensitive surfaces among the M photosensitive surfaces are not electrically connected to each other, anodes of at least two photosensitive surfaces among the M photosensitive surfaces are electrically connected to each other, or anodes of the M photosensitive surfaces are electrically connected to each other.

149. The device according to claim 76 or 77, wherein different parts of a same photosensitive surface are on a same plane or on different planes.

150. The device according to claim 76 or 77, wherein a set of photosensitive surfaces is on a same plane or on different planes, and the set of photosensitive surfaces comprises a plurality of photosensitive surfaces.

151. The device according to claim 76 or 77, wherein the predetermined wavelength is a wavelength sensitive to the detected tissue element.

152. The device according to claim 76 or 77, further comprising a temperature control module configured to control a temperature of the measurement region to be maintained within a predetermined temperature range during a process of measuring the tissue element.

153. The device according to claim 76 or 77, further comprising a mask on an initial photosensitive surface, wherein a light transmittance of the mask is less than or equal to a light transmittance threshold, and
wherein the mask is configured to be provided on the initial photosensitive surface to obtain the photosensitive surface.

154. The device according to claim 153, wherein a shape of the mask is determined according to a shape of the jitter distribution of the exit light.

155. The device according to claim 83, wherein the measurement probe is provided with a first sleeve, and
wherein a first end surface of the first sleeve exceeds a target surface of the measurement probe, the first end surface represents an end surface facing the measurement region, and the target surface of the measurement probe represents a surface facing the measurement region.

156. The device according to claim 155, wherein a second end surface and/or an inner region of the first sleeve are/is provided with a scatterer, the first end surface and the second end surface are opposite end surfaces, and the inner region comprises a partial inner region or an entire inner region.

157. The device according to claim 155 or 156, further comprising a second sleeve outside a target region of the first sleeve, wherein the target region represents a partial region or an entire region of the first sleeve exceeding the target surface of the measurement probe.

158. The device according to claim 157, wherein the second sleeve is provided with the scatterer.

159. The device according to claim 155, wherein an inner diameter of the first sleeve is greater than or equal to an inner diameter threshold.

160. The device according to claim 155, wherein an opening of the first end surface of the first sleeve is greater than or equal to an opening of the second end surface of the first sleeve.

161. The device according to claim 76 or 77, wherein a refractive index matcher is filled between the photosensitive surface and the measurement region.

162. The device according to claim 76 or 77, further comprising a protective portion arranged on a target surface of the photosensitive surface to protect the photosensitive surface, wherein the target surface of the photosensitive surface represents a surface facing the measurement region.

163. A wearable apparatus, comprising the device of measuring the tissue element according to any one of claims 76 to 162.

164. The wearable apparatus according to claim 163, wherein a mass of the wearable apparatus is less than or equal to a mass threshold, so that a movement mode of the wearable apparatus is identical to a skin jitter mode at the measurement region.

165. The wearable apparatus according to claim 163, wherein the wearable apparatus is configured to cause a movement amplitude of a skin at the measurement region to be less than or equal to a movement amplitude threshold.

FIG. 1

FIG. 2

```
                                                              ┌─── S310
┌─────────────────────────────────────────┐
│ A measurement region is irradiated by incident │
│ light having a single predetermined wavelength │
└─────────────────────────────────────────┘
                    │
                    ▼                                         ┌─── S320
┌─────────────────────────────────────────┐
│   A light intensity value corresponding to each │
│      beam of the exit light acquired by M        │
│    photosensitive surfaces is obtained, so as to │
│    obtain T output light intensities, where each │
│   output light intensity is obtained by processing │
│  the light intensity value of the exit light acquired │
│   by one or more photosensitive surfaces, and each │
│   photosensitive surface is used to acquire the light │
│     intensity value of the exit light exited from the │
│    exit position within a predetermined anti-jitter │
│      range corresponding to the photosensitive │
│              surface, 1≤T ≤M.                    │
└─────────────────────────────────────────┘
                    │
                    ▼                                         ┌─── S330
┌─────────────────────────────────────────┐
│ A concentration of the detected tissue element is │
│ determined according to at least one output light │
│   intensity corresponding to the predetermined │
│                 wavelength                       │
└─────────────────────────────────────────┘
```

FIG. 3

FIG. 4

FIG. 5

FIG. 6

FIG. 7

Not match

First target   First template
image        image

Measurement
probe

Region
positioning
feature

Match

New first target   First template
image        image

Measurement
probe

Region
positioning
feature

FIG. 8

Region
positioning
feature
First
predetermined
position

Second target image

Fixing
seat

Measurement
probe
Region
positioning
feature

Region
positioning
feature being
located at first
predetermined
position

New second target image

Fixing
seat

Measurement
probe

Region
positioning
feature

FIG. 9

Region positioning feature

First predetermined position

Second target image

Region positioning feature being located at first predetermined position

New second target image

Fixing seat

Measurement probe

Region positioning feature

Fixing seat

Measurement probe

Region positioning feature

FIG. 10

First posture positioning feature

First posture positioning feature

Second projection feature

Second projection feature

First posture positioning feature being matched with second projection feature

First posture positioning feature being matched with second projection feature

FIG. 11

Not match                                    Match

Third target        Second          New third target        Second
image        ↑ template image         image        ↑ template image

First posture                                    First posture
positioning                                    positioning
feature                                    feature
First posture                                    First posture
positioning                                    positioning
feature                                    feature

→

FIG. 12

First posture      Second                      First posture
positioning    predetermined                    positioning
feature        position                    feature being
located at
Second                      second
predetermined First                    predetermined
position  posture                    position
positioning
First posture                    feature being
positioning                    located at
feature   Fourth target image         second   New fourth target image
predetermined
↑           position           ↑

First posture                                    First posture
positioning                                    positioning
feature                                    feature

First posture                                    First posture
positioning                    →                positioning
feature                                    feature

FIG. 13

84

Sector-annular
photosensitive
surface 4

Incident light

Sector-annular
photosensitive
surface 1

Sector-annular
photosensitive
surface 3

Sector-annular
photosensitive
surface 2

FIG. 14

FIG. 15

FIG. 16

FIG. 17

FIG. 18

FIG. 19

FIG. 20

FIG. 21

FIG. 22

FIG. 23

2082

2081

2070

2082

Paste

Paste

Hook surface

FIG. 24

2082

2081

2070

2082

Attaching

Attaching

2090

2090

FIG. 25

2100

Region
positioning
feature

First projection feature

Detected
object

Positioning of measurement region is
not completed

Positioning of measurement region is completed

FIG. 26

2080

2070

2100

Region positioning feature

FIG. 27

2080

2070

2110

Region positioning feature

FIG. 28

First posture
positioning feature

FIG. 29

First posture positioning feature

2080

2070

2120

FIG. 30

First posture positioning feature

2080

2070

2130

FIG. 31

FIG. 32

FIG. 33

FIG. 34

FIG. 35

Photosensitive surface

Incident light

FIG. 36

Photosensitive surface

Incident light

FIG. 37

Photosensitive surface

Incident light

FIG. 38

Incident light

Photosensitive
surface

$h_1$

$h_2$

FIG. 39

First end surface

Target
surface

2140

FIG. 40

2150

First end surface

Target surface

2140

## FIG. 41

Photosensitive
surface

Incident
light

Incident
light

Exit light
Exit light

Skin
state 2

Skin
state 1

Vessel
state 1

Vessel
state 2

## FIG. 42

FIG. 43

FIG. 44

FIG. 45

4600

FIG. 46

FIG. 47

FIG. 48

FIG. 49

Posture
positioning
portion

Region
positioning
portion

Posture
positioning
portion

Second
projection
feature and third
projection
feature

Region
positioning
feature

First
projection
feature

Second projection
feature and third
projection feature

Supporting
portion

Posture
positioning
feature

Posture
positioning
feature

FIG. 50

Measurement duration (hour)

FIG. 51

FIG. 52

FIG. 53

FIG. 54

FIG. 55

FIG. 56

**EP 4 292 532 A1**

## INTERNATIONAL SEARCH REPORT

| International application No. |
|---|
| **PCT/CN2021/143795** |

**A.  CLASSIFICATION OF SUBJECT MATTER**

A61B 5/1455(2006.01)i;  A61B 5/00(2006.01)i

According to International Patent Classification (IPC) or to both national classification and IPC

**B.  FIELDS SEARCHED**

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

CNABS, CNTXT, WPI, EPODOC, CNKI: 先阳科技有限公司, 徐可欣, 入射, 出射, 光, 波长, 采集, 感光, 检测, 面, 手指, 抖, incidence, exit, light, wavelength, collection, detect, measure, plane, surface, finger, shake

**C.  DOCUMENTS CONSIDERED TO BE RELEVANT**

| Category* | Citation of document, with indication, where appropriate, of the relevant passages | Relevant to claim No. |
|---|---|---|
| X | WO 2020119825 A1 (TIANJIN SUNRISE TECHNOLOGIES DEVELOPMENT CO., LTD.) 18 June 2020 (2020-06-18) description, pages 9-33, and figures 1-19 | 1-165 |
| A | US 2016242682 A1 (GULATI, Sandeep et al.) 25 August 2016 (2016-08-25) entire document | 1-165 |
| A | US 2009312615 A1 (CADUFF, Andreas et al.) 17 December 2009 (2009-12-17) entire document | 1-165 |
| A | US 2016249836 A1 (GULATI, Sandeep et al.) 01 September 2016 (2016-09-01) entire document | 1-165 |
| A | GB 2357844 A (OPTEL INSTRUMENTS LIMITED) 04 July 2001 (2001-07-04) entire document | 1-165 |

☐ Further documents are listed in the continuation of Box C.  ☑ See patent family annex.

\*    Special categories of cited documents:
"A"   document defining the general state of the art which is not considered to be of particular relevance
"E"   earlier application or patent but published on or after the international filing date
"L"   document which may throw doubts on priority claim(s) or which is cited to establish the publication date of another citation or other special reason (as specified)
"O"   document referring to an oral disclosure, use, exhibition or other means
"P"   document published prior to the international filing date but later than the priority date claimed

"T"   later document published after the international filing date or priority date and not in conflict with the application but cited to understand the principle or theory underlying the invention
"X"   document of particular relevance; the claimed invention cannot be considered novel or cannot be considered to involve an inventive step when the document is taken alone
"Y"   document of particular relevance; the claimed invention cannot be considered to involve an inventive step when the document is combined with one or more other such documents, such combination being obvious to a person skilled in the art
"&"   document member of the same patent family

| Date of the actual completion of the international search | Date of mailing of the international search report |
|---|---|
| **11 March 2022** | **29 March 2022** |

| Name and mailing address of the ISA/CN | Authorized officer |
|---|---|
| **China National Intellectual Property Administration (ISA/CN)** No. 6, Xitucheng Road, Jimenqiao, Haidian District, Beijing 100088, China | |
| Facsimile No. **(86-10)62019451** | Telephone No. |

Form PCT/ISA/210 (second sheet) (January 2015)

| INTERNATIONAL SEARCH REPORT<br>Information on patent family members | | | | International application No.<br>**PCT/CN2021/143795** | | |
|---|---|---|---|---|---|---|

| Patent document<br>cited in search report | | | Publication date<br>(day/month/year) | Patent family member(s) | | Publication date<br>(day/month/year) |
|---|---|---|---|---|---|---|
| WO | 2020119825 | A1 | 18 June 2020 | CN 113164109 A | | 23 July 2021 |
| | | | | AU 2019399791 A1 | | 22 July 2021 |
| | | | | CA 3122875 A1 | | 18 June 2020 |
| | | | | US 2022054051 A1 | | 24 February 2022 |
| | | | | SG 11202106281 Q A | | 29 July 2021 |
| | | | | JP 2022514226 A | | 10 February 2022 |
| | | | | CN 111317442 A | | 23 June 2020 |
| | | | | KR 20210104081 A | | 24 August 2021 |
| | | | | CN 111317443 A | | 23 June 2020 |
| | | | | EP 3895615 A1 | | 20 October 2021 |
| US | 2016242682 | A1 | 25 August 2016 | None | | |
| US | 2009312615 | A1 | 17 December 2009 | US 9713447 B2 | | 25 July 2017 |
| | | | | EP 1954175 A1 | | 13 August 2008 |
| | | | | EP 1954175 B1 | | 13 July 2016 |
| | | | | EP 2329764 A2 | | 08 June 2011 |
| | | | | JP 2009514619 A | | 09 April 2009 |
| | | | | JP 4947440 B2 | | 06 June 2012 |
| | | | | WO 2007053963 A1 | | 18 May 2007 |
| US | 2016249836 | A1 | 01 September 2016 | None | | |
| GB | 2357844 | A | 04 July 2001 | GB 0108162 D0 | | 23 May 2001 |
| | | | | GB 0108164 D0 | | 23 May 2001 |
| | | | | GB 2357846 A | | 04 July 2001 |
| | | | | GB 2357846 B | | 17 October 2001 |
| | | | | IN 242KOL2004 A | | 04 August 2006 |
| | | | | IN 243KOL2004 A | | 28 July 2006 |
| | | | | GB 0108163 D0 | | 23 May 2001 |
| | | | | GB 2357845 A | | 04 July 2001 |
| | | | | GB 2357845 B | | 19 September 2001 |
| | | | | IN 244KOL2004 A | | 25 August 2006 |
| | | | | GB 9804828 D0 | | 29 April 1998 |
| | | | | GB 2322941 A | | 09 September 1998 |
| | | | | GB 2322941 B | | 25 July 2001 |

Form PCT/ISA/210 (patent family annex) (January 2015)